# EUROPEAN PATENT SPECIFICATION

(11) **EP 2 663 553 B1**
(45) Date of publication and mention of the grant of the patent: **26.08.2015**
(21) Application number: 11755509.4
(22) Date of filing: 31.08.2011
(51) Int. Cl.: C07D 401/12, C07D 401/14, C07D 405/14, C07D 413/12, C07D 417/12, A61K 31/4709, A61K 31/4725, A61P 35/00

(54) **QUINOLINE AND ISOQUINOLINE DERIVATIVES FOR USE AS JAK MODULATORS**
CHINOLIN- UND ISOCHINOLINVERBINDUNGEN ZUR VERWENDUNG ALS JAK-MODULATOREN
COMPOSÉS QUINOLÉINE ET ISOQUINOLÉINE EN TANT QUE MODULATEURS DE JAK

(30) Priority: 01.09.2010 US 379324 P
(43) Date of publication of application: 20.11.2013
(73) Proprietor: Ambit Biosciences Corporation, 11080 Roselle Street San Diego, CA 92121 (US)
(72) Inventor: FARAONI, Raffaella, San Diego CA 92121 (US); HADD, Michael, J., San Diego CA 92121 (US); HOLLADAY, Mark, W., San Diego CA 92121 (US); ROWBOTTOM, Martin, San Diego CA 92121 (US); SETTI, Eduardo, San Mateo CA 94401 (US)
(74) Representative: Savic, Bojan
(86) International application number: PCT/US2011/049945
(87) International publication number: WO 2012/030944

(56) References cited:
- WO-A1-2010/099379
- US-A- 6 080 747
- US-A1- 2005 038 023

## Description

Provided herein are compounds that are modulators of JAK kinases, compositions comprising the compounds and the compounds for use in methods of treating. The compounds provided are useful in the treatment, prevention, or amelioration of a disease or disorder related to JAK, including JAK2, JAK3 or TYK2 kinases, or one or more symptoms associated with such diseases or disorders. Further provided are the compounds for use in methods for treatment of cancer, including blood borne and solid tumors.

The JAK kinase family is a cytoplasmic protein kinase family comprising the members JAK1, JAK2, JAK3 and TYK2. Growth factor or cytokine receptors that recruit JAK kinases include the interferon receptors, interleukin receptors (receptors for the cytokines IL-2 to IL-7, IL-9 to IL-13, I-15, IL-23), various hormone receptors (erythropoietin (Epo) receptor, the thrombopoietin (Tpo) receptor, the leptin receptor, the insulin receptor, the prolactin (PRL) receptor, the Granulocyte Colony-Stimulating Factor (G-CSF) receptor and the growth hormone receptor, receptor protein tyrosine kinases (such as EGFR and PDGFR), and receptors for other growth factors such as leukemia inhibitory factor (LIF), Oncostatin M (OSM), IFNα/β/γ, Granulocyte-macrophage colony-stimulating factor (GM-CSF), Ciliary neurotrophic factor (CNTF), cardiotrophin-1 (CT-1) (Sec, Rane, S.G. and Reddy E.P., Oncogene 2000 19, 5662-5679).

Phosphorylated receptors serve as docking sites for other SH-2 domain containing signaling molecules that interact with JAKs such as the STAT family of transcription factors, Src family of kinases, MAP kinases, PI3 kinase and protein tyrosine phosphatases (Ranc S.G. and Reddy E.P., Oncogene 2000 19, 5662-5679). The family of latent cytoplasmic transcription factors, STATs, is the most well characterized downstream substrates for JAKs. The STAT proteins bind to phosphorylated cytokine receptors through their SH2 domains to become phosphorylated by JAKs, which leads to their dimerization and release and eventual translocation to the nucleus where they activate gene transcription. The various members of STAT which have been identified thus far, are STAT1, STAT2, STAT3, STAT4, STAT5 (including STAT5a and STAT5b) and STAT6.

Since the JAK kinases may play an important signaling role via such receptors, disorders of fat metabolism, growth disorders and disorders of the immune system are all potential therapeutic targets.

The JAK kinases and JAK2 mutations are implicated in myeloproliferative disorders, cancers, including blood borne and solid tumors. Exemplary disorders include chronic myeloid leukemia (CML), polycythemia vera (PV), essential thrombocythemia (ET), primary myelorbrosis (PMF), chronic eosinophilic leukemia (CEL), chronic myelomonocytic leukemia (CMML) and systemic mastocytosis (SM). Myeloproliferative disorders are believed to arise from either gain-of-function mutations to JAK itself or from activation by the oncoprotein BCR-ABL, which specifically activates the JAK2 pathway. Several literature reports describe role of JAK2 mutations in various disorders. *See*, Samanta et al. Cancer Res 2006, 66(13), 6468-6472, Sawyers et al. Cell, 1992, 70, 901-910, Tefferi N. Eng. J. Med. (2007) 356(5): 444-445) Baxter *metal.* Lancet (2005) 365:1054-1056, Levine et al. Blood (2006, Jones et al. Blood (2005) 106:2162-2168) 107:4139-4141, Campbell et al. Blood (2006) 107(5): 2098-2100, Scott et al. N Eng J Med 2007 356(5): 459-468, Mercher et al. Blood (2006) 108(8): 2770-2778, Lacronique et al. Science (1997) 278:1309-1312, Lacronique et al. Blood (2000) 95:2535-2540, Griesinger F. et al. Genes Chromosomes Cancer (2005) 44:329-333, Bouquet et al. Oncogene (2005) 24:7248-7252, Schwaller et al. Mol. Cell. 2000 6,693-704 and Zhao et al. EMBO 2002 21(9), 2159-2167.

Literature indicates that JAK may also serve as a target for prostate cancer, including androgcn-rcsistant prostate cancer. *See,* Barton et al. Mol. Canc. Ther. 2004 3(1), 11-20, Blume-Jensen et al. Nature (2001) 411(b835):355-356 and Bromberg J Clin Invest. (2002) 109(9):1139-1142 and Rane Oncogene (2000) 19(49):5662-5679. JAK as a prominent mediator of the cytokine signaling pathway, is considered to be a therapeutic target for inflammation and transplant rejections. *See*, Borie et al., Transplantation (2005) 79(7):791-801 and Milici et al., Arthritis Research (2008) 10(R14):1-9

Given the multitude of diseases attributed to the dysregulation of JAK signaling, many small molecule inhibitors of JAK are currently being developed. Examples of compounds in preclinical development include TG101209 (TargeGen). Examples of compounds being investigated in clinical studies include TNCB018424 (Incyte), XL019 (Exelixis) and TG101348 (TargeGen). *See*, Pardanani et al. Leukemia 2007, 21:1658-1668; and Pardanai, A. Leukemia 2008 22:23-20.

There is, however, an ever-existing need to provide novel classes of compounds that are useful as inhibitors of enzymes in the JAK signaling pathway.

Provided herein are compounds of formula (I) 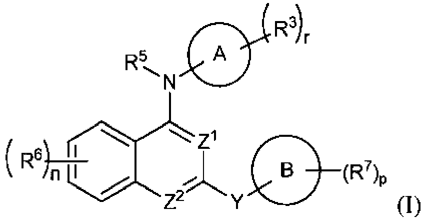 or pharmaceutically acceptable salts, solvates or hydrates thereof, wherein
Z¹ and Z² are each independently N or CR⁰, such that one of Z¹ or Z² is N and the other is CR⁰;
ring A is azolyl;
ring B is aryl or heteroaryl;
Y is a linker selected from -C(R¹)(R²)-, -S(O)- and -S(O)₂-;
R⁰ is hydrogen, cyano or alkyl;
R¹ and R² are selected from (i), (ii), (iii), (iv) and (v) as follows:
   (i) R¹ and R² together form =O, =S, =NR⁹ or =CR¹⁰R¹¹;
   (ii) R¹ and R² are both -OR⁸, or R¹ and R², together with the carbon atom to which they are attached, form cycloalkyl or heterocyclyl wherein the cycloalkyl is substituted with one or more, in one embodiment, one to four, in one embodiment, one to three, in one embodiment, one or two, substituents selected from halo, deutero, alkyl, cycloalkyl, heterocyclyl, aryl, heteroaryl, cyano, =O, =N-OR²¹, -R^{x}OR²¹, -R^{x}N(R²²)₂, -R^{x}S(O)_{q}R²³, -C(O)R²¹, -C(O)OR²¹ and -C(O)N(R²²)₂, and wherein the heterocyclyl contains one to two heteroatoms each independently selected from O, NR²⁴, S, S(O) and S(O)₂;
   (iii) R¹ is hydrogen or halo; and R² is halo;
   (iv) R¹ is alkyl, alkenyl, alkynyl, cycloalkyl or aryl, wherein the alkyl, alkenyl, alkynyl, cycloalkyl and aryl are each optionally substituted with one or more, in one embodiment, one to four, in one embodiment, one to three, in one embodiment, one, two or three, substitutcnts selected from halo, cyano, alkyl, -R^{x}OR^{w}, -R^{x}S(O)_{q}R^{v}, -R^{x}NR^{y}R^{z} and -C(O)OR^{w}; and R² is hydrogen, halo or -OR⁸; and
   (v) R¹ is halo, deutero, -OR¹², -NR¹³R¹⁴, or -S(O)_{q}R¹⁵; and R² is hydrogen, deutero, alkyl, alkenyl, alkynyl, cycloalkyl or aryl, wherein the alkyl, alkenyl, alkynyl, cycloalkyl and aryl are each optionally substituted with one or more, in one embodiment, one to four, in one embodiment, one to three, in one embodiment, one, two or three, substitutents selected from halo, cyano, alkyl, -R^{x}OR^{w}, -R^{x}S(O)_{q}R^{v} and -R^{x}NR^{y}R^{z};
R³ is hydrogen, deutero, halo, alkyl, cyano, haloalkyl, deuteroalkyl, cycloalkyl, cycloalkylalkyl, hydroxy or alkoxy;
R⁵ is hydrogen or alkyl;
each R⁶ is independently selected from deutero, halo, nitro, cyano, alkyl, alkenyl, alkynyl, haloalkyl, cycloalkyl, aryl, arylalkyl, heteroaryl, heteroarylalkyl, heterocyclyl, heterocyclylalkyl, -R^{x}OR¹⁸, -R^{x}NR¹⁹R²⁰, -R^{x}C(O)NR^{y}R^{z},-R^{x}S(O)_{q}R^{v}, -R^{x}NR¹⁹C(O)R¹⁸, -R^{x}C(O)OR¹⁸ and -R^{x}NR¹⁹S(O)_{q}R^{v}; where the alkyl, alkenyl, alkynyl, haloalkyl, cycloalkyl, aryl, heteroaryl and heterocyclyl groups are optionally substituted with one, two or three halo, oxo, hydroxy, alkoxy, alkyl, alkenyl, alkynyl, haloalkyl, or cycloalkyl groups;
each R⁷ is independently halo, alkyl, haloalkyl or -R^{x}OR^{w};
R⁸ is alkyl, alkenyl or alkynyl;
R⁹ is hydrogen, alkyl, haloalkyl, hydroxy, alkoxy or amino;
R¹⁰ is hydrogen or alkyl;
R¹¹ is hydrogen, alkyl, haloalkyl or -C(O)OR⁸;
R¹² is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, -C(O)R^{v}, -C(O)OR^{w} and -C(O)NR^{y}R^{z}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more, in one embodiment, one to four, in one embodiment, one to three, in one embodiment, one, two or three, substituents independently selected from halo, oxo, alkyl, hydroxy, alkoxy, amino and alkylthio;
R¹³ and R¹⁴ are selected as follows:
   (i) R¹³ is hydrogen or alkyl; and R¹⁴ is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, alkoxy, -C(O)R^{v}, -C(O)OR^{w}, -C(O)NR^{y}R^{z} and -S(O)_{q}R^{v}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more, in one embodiment, one to four, in one embodiment, one to three, in one embodiment, one, two or three, substituents independently selected from halo, oxo, alkyl, hydroxy, alkoxy, amino and alkylthio; or
   (ii) R¹³ and R¹⁴, together with the nitrogen atom to which they are attached, form heterocyclyl or heteroaryl wherein the heterocyclyl and heteroaryl are substituted with one or more, in one embodiment, one to four, in one embodiment, one to three, in one embodiment, one, two or three, substituents independently selected from halo, alkyl, hydroxy, alkoxy, amino and alkylthio and wherein the heterocyclyl is optionally substituted with oxo;
R¹⁵ is alkyl, alkenyl, alkynyl, cycloalkyl, cycloalkylalkyl, heterocyclyl, heterocyclylalkyl, aryl, aralkyl, heteroaryl, heteroaralkyl, -C(O)NR^{y}R^{z} or -NR^{y}R^{z}, wherein the alkyl, alkenyl, alkynyl, cycloalkyl, cycloalkylalkyl, heterocyclyl, heterocyclylalkyl, aryl, aralkyl, heteroaryl and heteroaralkyl arc each optionally substituted with one or more, in one embodiment, one to four, in one embodiment, one to three, in one embodiment, one, two or three, substituents independently selected from halo, oxo, alkyl, hydroxy, alkoxy, amino and alkylthio;
R¹⁸ is hydrogen, alkyl, haloalkyl, hydroxyalkyl, alkenyl, alkynyl, cycloalkyl, cycloalkylalkyl, heterocyclyl, heterocyclylalkyl, aryl, aralkyl, heteroaryl or heteroarylalkyl; wherein R¹⁸ is optionally substituted with 1 to 3 groups Q¹, each Q¹ independently selected from alkyl, hydroxy, halo, oxo, haloalkyl, alkoxy, aryloxy, alkoxyalkyl, alkoxycarbonyl, alkoxysulfonyl, carboxyl, cycloalkyl, heterocyclyl, aryl, heteroaryl, haloaryl and amino;
R¹⁹ and R²⁰ are selected as follows:
   (i) R¹⁹ and R²⁰ are each independently hydrogen or alkyl; or
   (ii) R¹⁹ and R¹⁰, together with the nitrogen atom to which they are attached, form a heterocyclyl or heteroaryl which are each optionally substituted with 1 to 2 groups each independently selected from halo, oxo, alkyl, haloalkyl, hydroxyl and alkoxy;
R²¹ is hydrogen, alkyl, alkenyl, alkynyl, haloalkyl or cycloalkyl;
each R²² is independently hydrogen, alkyl, alkenyl, alkynyl, haloalkyl or cycloalkyl; or both R²², together with the nitrogen atom to which they are attached, form a heterocyclyl optionally substituted with oxo;
R²³ is alkyl, alkenyl, alkynyl or haloalkyl;
R²⁴ is hydrogen or alkyl;
each R^{x} is independently alkylene or a direct bond;
R^{v} is hydrogen, alkyl, alkenyl or alkynyl;
R^{w} is independently hydrogen, alkyl, alkenyl, alkynyl or haloalkyl;
R^{y} and R^{z} arc selected as follows:
   (i) R^{y} and R^{z} are each independently hydrogen, alkyl, alkenyl, alkynyl, cycloalkyl, haloalkyl or heterocyclyl;
   (ii) R^{y} and R^{z}, together with the nitrogen atom to which they arc attached, form a heterocyclyl or heteroaryl which are optionally substituted with 1 to 2 groups each independently selected from halo, alkyl, haloalkyl, hydroxyl and alkoxy;
n is 0-4;
p is 0-5;
each q is independently 0, 1 or 2; and
r is 1-3.

In certain embodiments, the compounds have activity as JAK kinase, including JAK2 kinase, modulators. The compounds are useful in medical treatments, pharmaceutical compositions and methods for modulating the activity of JAK kinase, including wildtype and/or mutated forms of JAK kinase. In certain embodiments, the compounds provided herein have activity as JAK2 kinase modulators. In certain embodiments, the compounds are inhibitors of JAK kinase, including JAK2 kinase.

In one embodiment, the compounds for use in the compositions and methods provided herein are compounds of formula (I).

In one embodiment, the compound provided herein is a compound of formula (I). In one embodiment, the compound provided herein is a pharmaceutically acceptable salt of the compound of formula (I). In one embodiment, the compound provided herein is a solvate of the compound of formula (I). In one embodiment, the compound provided herein is a hydrate of compound of formula (I).

Also provided are pharmaceutical compositions formulated for administration by an appropriate route and means containing effective concentrations of one or more of the compounds provided herein, or pharmaceutically acceptable salts, solvates and hydrates thereof, and optionally comprising at least one pharmaceutical carrier.

Such pharmaceutical compositions deliver amounts effective for the treatment, prevention, or amelioration of diseases or disorders that include without limitation, myeloproliferative disorders such as polycythemia vera (PCV), essential thrombocythemia (ET), primary myelofibrosis (PMF), chronic eosinophilic leukemia (CEL), chronic myelomonocytic leukemia (CMML), systemic mastocytosis (SM) and idiopathic myelofibrosis (IMF); leukemia such as myeloid leukemia including chronic myeloid leukemia (CML), imatinib-resistant forms of CML, acute myeloid leukemia (AML), and a subtype of AML, acute megakaryoblastic leukemia (AMKL); lymphoproliferative diseases such as myeloma; cancer such as cancer of the head and neck, prostate cancer, breast cancer, ovarian cancer, melanoma, lung cancers, brain tumors, pancreatic cancer and renal cancer; and inflammatory diseases or disorders related to immune dysfunction, immunodeficiency, immunomodulation, autoimmune diseases, tissue transplant rejection, graft-versus-host disease, wound healing, kidney disease, diabetic neuropathy, multiple sclerosis, thyroiditis, type 1 diabetes, sarcoidosis, psoriasis, allergic rhinitis, inflammatory bowel disease including Crohn's disease and ulcerative colitis (UC), systemic lupus erythematosis (SLE), arthritis, osteoarthritis, rheumatoid arthritis, osteoporosis, asthma chronic obstructive pulmonary disease (COPD) and dry eye syndrome (or keratoconjunctivitis sicca (KCS)). In one embodiment, such diseases or disorders are modulated or otherwise affected by the JAK kinases, including JAK2, JAK3 or TYK2.

Also provided herein are combination therapies using one or more compounds or compositions provided herein, or pharmaceutically acceptable salts, solvates or hydrates thereof, in combination with other pharmaceutically active agents for the treatment of the diseases and disorders described herein.

In one embodiment, such additional pharmaceutical agents include one or more chemotherapeutic agents, anti-proliferative agents, anti-inflammatory agents, immunomodulatory agents or immunosuppressive agents.

The compounds or compositions provided herein, or pharmaceutically acceptable salts, solvates or hydrates thereof, may be administered simultaneously with, prior to, or after administration of one or more of the above agents. Pharmaceutical compositions containing a compound provided herein and one or more of the above agents are also provided.

In certain embodiments, provided herein are the compounds provided herein for use in methods of treating, preventing or ameliorating a disease or disorder that is modulated or otherwise affected by JAK kinases, including JAK2 kinase such as wild type and/or mutant JAK2 kinase, or one or more symptoms or causes thereof. In another embodiment, provided herein are the compounds provided herein for use in methods of treating, preventing or ameliorating a disease or disorder by modulating the JAK2 kinase selectively over JAK3 kinase. In yet another embodiment, provided herein are the compounds provided herein for use in methods of treating, preventing or ameliorating a disease or disorder by modulating the JAK3 kinase selectively over JAK2 kinase. In another embodiment, provided herein are the compounds provided herein for use in methods of treating, preventing or amerliorating a disease or disorder by modulating both JAK2 and JAK3. In one embodiment, provided are the compounds provided herein for use in methods for treatment of cancer, including blood borne and solid tumors.

In practicing the methods, effective amounts of the compounds or compositions containing therapeutically effective concentrations of the compounds, which are formulated for systemic delivery, including parenteral, oral, or intravenous delivery, or for local or topical application are administered to an individual exhibiting the symptoms of the disease or disorder to be treated. The amounts arc effective to ameliorate or eliminate one or more symptoms of the disease or disorder.

These and other aspects of the subject matter described herein will become evident upon reference to the following detailed description.

Provided herein are compounds of formula (I) that have activity as JAK kinase, including JAK2 kinase, modulators. Further provided are the compounds provided herein for use in methods of treating, preventing or ameliorating diseases that are modulated by JAK kinases, including JAK2 kinase, and pharmaceutical compositions and dosage forms useful for such methods. The methods and compositions are described in detail in the sections below.

In certain embodiments, the compounds provided herein are JAK2 selective, *i.e.,* the compounds bind or interact with JAK2 at substantially lower concentrations than they bind or interact with other JAK receptors, including JAK3 receptor, at that same concentration. In certain embodiments, the compounds bind to JAK3 receptor at a binding constant at least about 3-fold higher, about 5-fold higher, about 10-fold higher, about 20-fold higher, about 25-fold higher, about 50-fold higher, about 75-fold higher, about 100-fold higher, about 200-fold higher, about 225-fold higher, about 250 fold higher, or about 300 fold higher than they bind JAK2 receptor.

In certain embodiments, the compounds provided herein are JAK3 selective, *i.e.*, the compounds bind or interact with JAK3 at substantially lower concentrations than they bind or interact with other JAK receptors, including JAK2 receptor, at that same concentration. In certain embodiments, the compounds bind to JAK2 receptor at a binding constant at least about 3-fold higher, about 5-fold higher, about10-fold higher, about 20-fold higher, about 25-fold higher, about 50-fold higher, about 75-fold higher, about 100-fold higher, about 200-fold higher, about 225-fold higher, about 250 fold higher, or about 300 fold higher than they bind with JAK3 receptor.

In certain embodiments, the compounds provided herein have Kd of greater than about 10 nM, 20 nM, 25 nM, 40 nM, 50 nM, or 70 nM against Aurora B kinase. Methods for determining binding constant against Aurora B kinase arc known to one of skill in the art. Exemplary methods are described in US provisional application no. 61/294,413, International Publication No. WO 2011/088045 and Fabian et al., Nature Biotechnology 2005, 23, 329-336.

### A. DEFINITIONS

Unless defined otherwise, all technical and scientific terms used herein have the same meaning as is commonly understood by one of ordinary skill in the art. In the event that there are a plurality of definitions for a term herein, those in this section prevail unless stated otherwise.

"Alkyl" refers to a straight or branched hydrocarbon chain group consisting solely of carbon and hydrogen atoms, containing no unsaturation, having from one to ten, one to eight, one to six or one to four carbon atoms, and which is attached to the rest of the molecule by a single bond, *e.g.,* methyl, ethyl, *n*-propyl, 1-methylethyl (*iso*-propyl), *n*-butyl, *n*-pentyl, 1,1-dimethylethyl (*t*-butyl), and the like.

"Alkenyl" refers to a straight or branched hydrocarbon chain group consisting solely of carbon and hydrogen atoms, containing at least one double bond, in certain embodiment, having from 2 to 10 carbon atoms, from 2 to 8 carbon atoms, or from 2 to 6 carbon atoms, and which is attached to the rest of the molecule by a single bond or a double bond, *e.g.,* ethenyl, prop-1-enyl, but-1-enyl, pent-1-enyl, penta-1,4-dienyl, and the like.

"Alkynyl" refers to a straight or branched hydrocarbon chain group consisting solely of carbon and hydrogen atoms, containing at least one triple bond, having from two to ten carbon atoms, and which is attached to the rest of the molecule by a single bond or a triple bond, *e.g.*, ethynyl, prop-1-ynyl, but-1-ynyl, pent-1-ynyl, pent-3-ynyl and the like.

"Alkylene" and "alkylene chain" refer to a straight or branched divalent hydrocarbon chain consisting solely of carbon and hydrogen, containing no unsaturation and having from one to eight carbon atoms, *e.g.,* methylene, ethylene, propylene, *n*-butylene and the like. The alkylene chain may be attached to the rest of the molecule through any two carbons within the chain.

"Alkoxy" refers to the group having the formula -OR wherein R is alkyl or haloalkyl, where the alkyl may be optionally substituted by one or more substituents, in one embodiment, one, two or three substitutents independently selected from the group consisting of nitro, halo, hydroxyl, alkoxy, oxo, thioxo, amino, carbony, carboxy, azido, cyano, cycloalkyl, heteroaryl, and heterocyclyl.

"Alkoxyalkyl" refers to a group having the formula -R_{b}OR wherein Rₕ is a straight or branched alkylene chain and OR is alkoxy as defined above.

"Alkylthio" refers to a group having the formula -SR wherein R is alkyl or haloalkyl.

"aryloxy" refers to the group -OR, in which R is aryl, including lower aryl, such as phenyl.

"Amine" or "amino" refers to a group having the formula -NR'R" wherein R' and R" are each independently hydrogen, alkyl, haloalkyl, hydroxyalkyl or alkoxyalkyl or wherein R' and R", together with the nitrogen atom to which they are attached form a heterocyclyl optionally substituted with halo, oxo, hydroxy or alkoxy.

"Aminoalkyl" refers to a group having the formula -RₕNR'R" wherein Rₕ is a straight or branched alkylene chain and wherein NR'R" is amino as defined above.

"Aminocarbonyl" refers to a group having the formula -C(O)NR'R" wherein -NR'R" is amino as defined above.

"Aryl" refers to a group of carbocylic ring system, including monocyclic, bicyclic, tricyclic, tetracyclic C₆-C₁₈ ring systems, wherein at least one of the rings is aromatic. The aryl may be fully aromatic, examples of which are phenyl, naphthyl, anthracenyl, acenaphthylenyl, azulenyl, fluorenyl, indenyl and pyrenyl. The aryl may also contain an aromatic ring in combination with a non-aromatic ring, examples of which are acenaphene, indene, and fluorene. The term includes both substituted and unsubstitutcd moieties. The aryl group can be substituted with any described moiety, including, but not limited to, one or more moieties selected from the group consisting of halo (fluoro, chloro, bromo or iodo), alkyl, hydroxyl, amino, alkoxy, aryloxy, nitro and cyano.

"Cycloalkyl" refers to a stable monovalent monocyclic or bicyclic hydrocarbon group consisting solely of carbon and hydrogen atoms, having from three to ten carbon atoms, and which is saturated and attached to the rest of the molecule by a single bond, *e.g*., cyclopropyl, cyclobutyl, cyclopentyl, cyclohexyl, decalinyl, norbornane, norbornene, adamantyl, bicyclo[2.2.2]octane and the like.

"Cycloalkylalkyl" refers to a group of the formula -RₐR_{d} where Rₐ is an alkyl group as defined above and R_{d} is a cycloalkyl group as defined above. The alkyl group and the cylcoalkyl group may be optionally substituted as defined herein.

"Deutero" or "deuterium" refers to the hydrogen isotope deuterium having the chemical symbol D.

"Deuteroalkyl" refers to an isotopically enriched alkyl group in which one or more of the hydrogen atoms are replaced by deuterium.

"Halo", "halogen" or "halide" refers to F, Cl, Br for I.

"Haloalkyl" refers to an alkyl group, in certain embodiments, C₁₋₆alkyl group in which one or more of the hydrogen atoms are replaced by halogen. Such groups include, but are not limited to, chloromethyl, trifluoromethyl, 1-chloro-2-fluoroethyl, 2,2-difluoroethyl, 2-fluoropropyl, 2-fluoropropan-2-yl, 2,2,2-trifluoroethyl, 1,1-difluoroethyl, 1,3-difluoro-2-methylpropyl, 2,2-difluorocyclopropyl, (trifluoromethyl)cyclopropyl, 4,4-difluorocyclohexyl and 2,2,2-trifluoro-1,1-dimethyl-ethyl.

"Heterocyclyl" refers to a stable 3- to 15-membered ring group which consists of carbon atoms and from one to five heteroatoms selected from a group consisting of nitrogen, oxygen and sulfur. In one embodiment, the heterocyclic ring system group may be a monocyclic, bicyclic or tricyclic ring or tetracyclic ring system, which may include fused or bridged ring systems; and the nitrogen or sulfur atoms in the heterocyclic ring system group may be optionally oxidized; the nitrogen atom may be optionally quaternized; and the heterocyclyl group may be partially or fully saturated or aromatic. The heterocyclic ring system may be attached to the main structure at any heteroatom or carbon atom which results in the creation of a stable compound. Exemplary heterocylic radicals include, azetidinyl, benzopyranonyl, benzopyranyl, benzotetrahydrofuranyl, benzotetrahydrothienyl, chromanyl, chromonyl, coumarinyl, decahydroisoquinolinyl, dibenzofuranyl, dihydrobenzisothiazinyl, dihydrobenzisoxazinyl, dihydrofuryl, dihydropyranyl, dioxolanyl, dihydropyrazinyl, dihydropyridinyl, dihydropyrazolyl, dihydropyrimidinyl, dihydropyrrolyl, dioxolanyl, 1,4 dithianyl, isobenzotetrahydrofuranyl, isobenzotetrahydrothienyl, isochromanyl, isocoumarinyl, benzo[1,3]dioxol-5-yl, benzodioxolyl, 1,3-dioxolan-2-yl, dioxolanyl, morpholinyl, octahydroindolyl, octahydroisoindolyl, tetrahydrofuran, oxazolidin-2-onyl, oxazolidinonyl, piperidinyl, piperazinyl, pyranyl, tetrahydrofuryl, tetrahydrofuranyl, tetrahydroisoquinolinyl, tetrahydropyranyl, tetrahydrothienyl, pyrrolidinonyl, oxathiolanyl, and pyrrolidinyl.

"Heteroaryl" refers to a heterocyclyl group as defined above which is aromatic. The heteroaryl group may be attached to the main structure at any heteroatom or carbon atom which results in the creation of a stable compound. Examples of such heteroaryl groups include, but are not limited to: acridinyl, benzimidazolyl, benzindolyl, benzisoxazinyl, benzo[4,6]imidazo[1,2-*a*]pyridinyl, benzofuranyl, benzonaphthofuranyl, benzothiadiazolyl, benzothiazolyl, benzothiophenyl, benzotriazolyl, benzothiopyranyl, benzoxazinyl, benzoxazolyl, benzothiazolyl, β-carbolinyl, carbazolyl, cinnolinyl, dibenzofuranyl, furanyl, imidazolyl, imidazopyridinyl, imidazothiazolyl, indazolyl, indolizinyl, indolyl, isobenzothienyl, isoindolinyl, isoquinolinyl, isothiazolidinyl, isothiazolyl, naphthyridinyl, octahydroindolyl, octahydroisoindolyl, oxazolidinonyl, oxazolidinyl, oxazolopyridinyl, oxazolyl, isoxazolyl, oxiranyl, perimidinyl, phenanthridinyl, phenathrolinyl, phenarsazinyl, phenazinyl, phenothiazinyl, phenoxazinyl, phthalazinyl, pteridinyl, purinyl, pyrazinyl, pyrazolyl, pyridazinyl, pyridinyl, pyridopyridinyl, pyrimidinyl, pyrrolyl, quinazolinyl, quinolinyl, quinoxalinyl, tetrazolyl, thiadiazolyl, thiazolyl, thienyl, triazinyl and triazolyl.

"Azolyl" refers to a 5-membered heterocyclic or heteroaryl ring system containing at least one nitrogen atom. Exemplary azolyl rings include pyrazolyl, imidazolyl, thiazolyl, isothiazolyl, oxazolyl, isoxazolyl, oxadiazolyl, thiadiazolyl, diazolyl, and triazolyl.

"Aralkyl" refers to a group of the formula -RₐR_{b} where Rₐ is an alkyl group as defined above, substituted by R_{b}, an aryl group, as defined above, *e.g.*, benzyl. Both the alkyl and aryl groups may be optionally substituted as defined herein.

"Heteroaralkyl" refers to a group of the formula -RₐR_{f} where Rₐ is an alkyl group as defined above and R_{f} is a heteroaryl group as defined herein. The alkyl group and the heteroaryl group may be optionally substituted as defined herein.

"Heterocyclylalkyl" refers to a group of the formula -RₐRₑ wherein Rₐ is an alkyl group as defined above and Rₑ is a heterocyclyl group as defined herein, where the alkyl group R_{d} may attach at either the carbon atom or the heteroatom of the heterocyclyl group Rₑ. The alkyl group and the heterocyclyl group may be optionally substituted as defined herein.

"Alkoxycarbonyl" refers to a group having the formula -C(O)OR in which R is alkyl, including lower alkyl.

The term "dioxacycloalkyl" as used herein means a heterocyclic group containing two oxygen ring atoms and two or more carbon ring atoms.

"Oxo" refers to the group =O attached to a carbon atom.

"Thioalkyl" refers to a group having the formula -RₕSRᵢ where the Rₕ is a straight or branched alkylene chain and Rᵢ is alkyl or haloalkyl.

"Thioxo" refers to the group =S attached to a carbon atom.

"IC₅₀" refers to an amount, concentration or dosage of a particular test compound that achieves a 50% inhibition of a maximal response, such as cell growth or proliferation measured via any the *in vitro* or cell based assay described herein.

Unless stated otherwise specifically described in the specification, it is understood that the substitution can occur on any atom of the alkyl, alkenyl, alkynyl, cycloalkyl, heterocyclyl, aryl or heteroaryl group.

Pharmaceutically acceptable salts include, but are not limited to, amine salts, such as but not limited to N,N'-dibenzylethylenediamine, chloroprocaine, choline, ammonia, diethanolamine and other hydroxyalkylamines, ethylenediamine, N-methylglucamine, procaine, N-benzylphenethylamine, 1-para-chlorobenzyl-2-pyrrolidin-1'-ylmethylbenzimidazole, diethylamine and other alkylamines, piperazine and tris(hydroxymethyl)aminomethane; alkali metal salts, such as but not limited to lithium, potassium and sodium; alkali earth metal salts, such as but not limited to barium, calcium and magnesium; transition metal salts, such as but not limited to zinc; and inorganic salts, such as but not limited to, sodium hydrogen phosphate and disodium phosphate; and also including, but not limited to, salts of mineral acids, such as but not limited to hydrochlorides, hydrobromides, phosphates and sulfates; and salts of organic acids, such as but not limited to acetates, lactates, malates, tartrates, citrates, ascorbates, succinates, butyrates, valerates, mesylates, esylates, tosylates, besylates, trifluoroacetates, benzoates, fumarates, maleates, and oxalates.

As used herein and unless otherwise indicated, the term "hydrate" means a compound provided herein or a salt thereof, that further includes a stoichiometric or non-stoichiometeric amount of water bound by non-covalent intermolecular forces.

As used herein and unless otherwise indicated, the term "solvate" means a solvate formed from the association of one or more solvent molecules to a compound provided herein. The term "solvate" includes hydrates (e.g., monohydrate, dihydrate, trihydrate, tetrahydrate and the like).

As used herein, "substantially pure" means sufficiently homogeneous to appear free of readily detectable impurities as determined by standard methods of analysis, such as thin layer chromatography (TLC), gel electrophoresis, high performance liquid chromatography (HPLC) and mass spectrometry (MS), used by those of skill in the art to assess such purity, or sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound may, however, be a mixture of stereoisomers. In such instances, further purification might increase the specific activity of the compound.

Unless specifically stated otherwise, where a compound may assume alternative tautomeric, regioisomeric and/or stereoisomeric forms, all alternative isomers arc intended to be encompassed within the scope of the claimed subject matter. For example, where a compound is described as having one of two tautomeric forms, it is intended that the both tautomers be encompassed herein. Thus, the compounds provided herein may be enantiomerically pure, or be stereoisomeric or diastereomeric mixtures.

It is to be understood that the compounds provided herein may contain chiral centers. Such chiral centers may be of either the (*R*) or (*S*) configuration, or may be a mixture thereof.

Optically active (+) and (-), (R)- and (*S*)-, or (D)- and (L)-isomers may be prepared using chiral synthons or chiral reagents, or resolved using conventional techniques, such as reverse phase HPLC or by crystallization.

As used herein, the term "enantiomerically pure" or "pure enantiomer" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of the desired enantiomer.

Where the number of any given substituent is not specified (*e.g.,* haloalkyl), there may be one or more substituents present. For example, "haloalkyl" may include one or more of the same or different halogens.

In the description herein, if there is any discrepancy between a chemical name and chemical structure, the structure preferably controls.

As used herein, "isotopic composition" refers to the amount of each isotope present for a given atom, and "natural isotopic composition" refers to the naturally occurring isotopic composition or abundance for a given atom. Atoms containing their natural isotopic composition may also be referred to herein as "non-enriched" atoms. Unless otherwise designated, the atoms of the compounds recited herein are meant to represent any stable isotope of that atom. For example, unless otherwise stated, when a position is designated specifically as "H" or "hydrogen", the position is understood to have hydrogen at its natural isotopic composition.

As used herein, "isotopically enriched" refers to an atom having an isotopic composition other than the natural isotopic composition of that atom. "Isotopically enriched" may also refer to a compound containing at least one atom having an isotopic composition other than the natural isotopic composition of that atom.

As used herein, "isotopic enrichment" refers to the percentage of incorporation of an amount of a specific isotope at a given atom in a molecule in the place of that atom's natural isotopic abundance. For example, deuterium enrichment of 1% at a given position means that 1% of the molecules in a given sample contain deuterium at the specified position. Because the naturally occurring distribution of deuterium is about 0.0156%, deuterium enrichment at any position in a compound synthesized using non-enriched starting materials is about 0.0156%. The isotopic enrichment of the compounds provided herein can be determined using conventional analytical methods known to one of ordinary skill in the art, including mass spectrometry and nuclear magnetic resonance spectroscopy. In certain embodiments, compounds herein having one or more deutero substituents have an isotopic enrichment factor for each designated deuterium atom of from about 50% to about 99.5%, 60% to about 99.5%, 70% to about 99.5% deuterium incorporation.

In certain embodiments, compounds herein having one or more deutero substituents have an isotopic enrichment factor for each designated deuterium atom of at least about 3500 (about 52.5% deuterium incorporation), at least about 4000 (about 60% deuterium incorporation), at least about 4500 (about 67.5% deuterium incorporation), at least about 5000 (about 75% deuterium incorporation), at least about 5500 (82.5% deuterium incorporation), at least about 6000 (about 90% deuterium incorporation), at least about 6466.7 (about 97% deuterium incorporation), at least about 6600 (about 99% deuterium incorporation), or at least about 6633.3 (99.5% deuterium incorporation).

In certain embodiments, compounds herein having one or more deutero substituents have an isotopic enrichment factor for each designated deuterium atom of about 3500 (about 52.5% deuterium incorporation), about 4000 (about 60% deuterium incorporation), about 4500 (about 67.5% deuterium incorporation), about 5000 (about 75% deuterium incorporation), about 5500 (82.5% deuterium incorporation), about 6000 (about 90% deuterium incorporation), about 6466.7 (about 97% deuterium incorporation), about 6600 (about 99% deuterium incorporation), or about 6633.3 (99.5% deuterium incorporation).

"Anti-cancer agents" refers to anti-metabolites (e.g., 5-fluoro-uracil, methotrexate, fludarabine), antimicrotubule agents (e.g., vinca alkaloids such as vincristine, vinblastine; taxanes such as paclitaxel, docctaxcl), alkylating agents (e.g., cyclophosphamide, melphalan, carmustine, nitrosoureas such as bischloroethylnitrosurea and hydroxyurea), platinum agents (e.g. cisplatin, carboplatin, oxaliplatin, JM-216 or satraplatin, CI-973), anthracyclines (e.g., doxrubicin, daunorubicin), antitumor antibiotics (e.g., mitomycin, idarubicin, adriamycin, daunomycin), topoisomerase inhibitors (e.g., etoposide, camptothecins), anti-angiogenesis agents (*e.g.* Sutent® and Bevacizumab) or any other cytotoxic agents, (estramustine phosphate, prednimustine), hormones or hormone agonists, antagonists, partial agonists or partial antagonists, kinase inhibitors, and radiation treatment.

"Anti-inflammatory agents" refers to methotrexate, matrix metalloproteinase inhibitors, inhibitors of pro-inflammatory cytokines (e.g., anti-TNF molecules, TNF soluble receptors, and IL1) non-steroidal anti-inflammatory drugs (NSAIDs) such as prostaglandin synthase inhibitors (e.g., choline magnesium salicylate, salicylsalicyclic acid), COX-1 or COX-2 inhibitors), or glucocorticoid receptor agonists such as corticosteroids, methylprednisone, prednisone, or cortisone.

As used herein, the abbreviations for any protective groups, amino acids and other compounds, are, unless indicated otherwise, in accord with their common usage or recognized abbreviations including abbreviations found in J. Org. Chem. 2007 72(1): 23A-24A or abbreviations established by the IUPAC-IUB Commission on Biochemical Nomenclature (see, Biochem. 1972, 11:942-944).

### B. COMPOUNDS

Provided herein are compounds of formula (I) 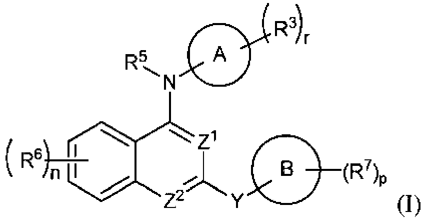 or pharmaceutically acceptable salts, solvates or hydrates thereof, wherein
Z¹ and Z² are each independently N or CR⁰, such that one of Z¹ or Z² is N and the other is CR⁰;
ring A is azolyl;
ring B is aryl or heteroaryl;
Y is a linker selected from -C(R¹)(R²)-, -S(O)- and -S(O)₂-;
R⁰ is hydrogen, cyano or alkyl;
R¹ and R² are selected from (i), (ii), (iii), (iv) and (v) as follows:
   (i) R¹ and R² together form =O, =S, =NR⁹ or =CR¹⁰R¹¹;
   (ii) R¹ and R² are both -OR⁸, or R¹ and R², together with the carbon atom to which they are attached, form cycloalkyl or heterocyclyl wherein the cycloalkyl is substituted with one or more, in one embodiment, one to four, in one embodiment, one to three, in one embodiment, one or two, substituents selected from halo, deutero, alkyl, cycloalkyl, heterocyclyl, aryl, heteroaryl, cyano, =O, =N-OR²¹, -R^{x}OR²¹, -R^{x}N(R²²)₂, -R^{x}S(O)_{q}R²³, -C(O)R²¹, -C(O)OR²¹ and -C(O)N(R²²)₂, and wherein the heterocyclyl contains one to two heteroatoms each independently selected from O, NR²⁴, S, S(O) and S(O)₂;
   (iii) R¹ is hydrogen or halo; and R² is halo;
   (iv) R¹ is alkyl, alkenyl, alkynyl, cycloalkyl or aryl, wherein the alkyl, alkenyl, alkynyl, cycloalkyl and aryl are each optionally substituted with one or more, in one embodiment, one to four, in one embodiment, one to three, in one embodiment, one, two or three, substitutcnts selected from halo, cyano, alkyl, -R^{x}OR^{w}, -R^{x}S(O)_{q}R^{v}, -R^{x}NR^{y}R^{z} and -C(O)OR^{w}; and R² is hydrogen, halo or -OR⁸; and
   (v) R¹ is halo, deutero, -OR¹², -NR¹³R¹⁴, or -S(O)_{q}R¹⁵; and R² is hydrogen, deutero, alkyl, alkenyl, alkynyl, cycloalkyl or aryl, wherein the alkyl, alkenyl, alkynyl, cycloalkyl and aryl are each optionally substituted with one or more, in one embodiment, one to four, in one embodiment, one to three, in one embodiment, one, two or three, substitutents selected from halo, cyano, alkyl, -R^{x}OR^{w}, -R^{x}S(O)_{q}R^{v} and -R^{x}NR^{y}R^{z};
R³ is hydrogen, dcutcro, halo, alkyl, cyano, haloalkyl, cycloalkyl, cycloalkylalkyl, hydroxy or alkoxy;
R⁵ is hydrogen or alkyl;
each R⁶ is independently selected from deutero, halo, nitro, cyano, alkyl, alkenyl, alkynyl, haloalkyl, cycloalkyl, aryl, arylalkyl, heteroaryl, heteroarylalkyl, heterocyclyl, heterocyclylalkyl, -R^{x}OR¹⁸, -R^{x}NR¹⁹R²⁰, -R^{x}C(O)NR^{y}R^{z} and -R^{x}S(O)_{q}R^{v}; where the alkyl, alkenyl, alkynyl, haloalkyl, cycloalkyl, aryl, heteroaryl and hoterocyclyl groups are optionally substituted with one, two or three halo, oxo, hydroxy, alkoxy, alkyl, alkenyl, alkynyl, haloalkyl, or cycloalkyl groups;
each R⁷ is independently halo, alkyl, haloalkyl or -R^{x}OR^{w};
R⁸ is alkyl, alkenyl or alkynyl;
R⁹ is hydrogen, alkyl, haloalkyl, hydroxy, alkoxy or amino;
R¹⁰ is hydrogen or alkyl;
R¹¹ is hydrogen, alkyl, haloalkyl or -C(O)OR⁸;
R¹² is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, -C(O)R^{v}, -C(O)OR^{w} and -C(O)NR^{y}R^{z}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more, in one embodiment, one to four, in one embodiment, one to three, in one embodiment, one, two or three, substituents independently selected from halo, oxo, alkyl, hydroxy, alkoxy, amino and alkylthio;
R¹³ and R¹⁴ are selected as follows:
   (i) R¹³ is hydrogen or alkyl; and R¹⁴ is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, alkoxy, -C(O)R^{v}, -C(O)OR^{w}, -C(O)NR^{y}R^{z} and -S(O)_{q}R^{v}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more, in one embodiment, one to four, in one embodiment, one to three, in one embodiment, one, two or three, substituents independently selected from halo, oxo, alkyl, hydroxy, alkoxy, amino and alkylthio; or
   (ii) R¹³ and R¹⁴, together with the nitrogen atom to which they are attached, form heterocyclyl or heteroaryl wherein the heterocyclyl and heteroaryl are substituted with one or more, in one embodiment, one to four, in one embodiment, one to three, in one embodiment, one, two or three, substituents independently selected from halo, alkyl, hydroxy, alkoxy, amino and alkylthio and wherein the heterocyclyl is optionally substituted with oxo;
R¹⁵ is alkyl, alkenyl, alkynyl, cycloalkyl, cycloalkylalkyl, heterocyclyl, heterocyclylalkyl, aryl, aralkyl, heteroaryl, heteroaralkyl, -C(O)NR^{y}R^{z} or -NR^{y}R^{z}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more, in one embodiment, one to four, in one embodiment, one to three, in one embodiment, one, two or three, substituents independently selected from halo, oxo, alkyl, hydroxy, alkoxy, amino and alkylthio;
R¹⁸ is hydrogen, alkyl, haloalkyl, hydroxyalkyl, alkenyl, alkynyl, cycloalkyl, cycloalkylalkyl, heterocyclyl, heterocyclylalkyl, aryl, aralkyl, heteroaryl or heteroarylalkyl; wherein R¹⁸ is optionally substituted with 1 to 3 groups Q¹, each Q¹ independently selected from alkyl, hydroxy, halo, oxo, haloalkyl, alkoxy, aryloxy, alkoxyalkyl, alkoxycarbonyl, alkoxysulfonyl, carboxyl, cycloalkyl, heterocyclyl, aryl, heteroaryl, haloaryl and amino;
R¹⁹ and R²⁰ are selected as follows:
   (i) R¹⁹ and R²⁰ are each independently hydrogen or alkyl; or
   (ii) R¹⁹ and R²⁰, together with the nitrogen atom to which they are attached, form a heterocyclyl or heteroaryl which are each optionally substituted with 1 to 2 groups each independently selected from halo, oxo, alkyl, haloalkyl, hydroxyl and alkoxy;
R²¹ is hydrogen, alkyl, alkenyl, alkynyl, haloalkyl or cycloalkyl;
each R²² is independently hydrogen, alkyl, alkenyl, alkynyl, haloalkyl or cycloalkyl; or both R²2, together with the nitrogen atom to which they are attached, form a heterocyclyl optionally substituted with oxo;
R²³ is alkyl, alkenyl, alkynyl or haloalkyl;
R²⁴ is hydrogen or alkyl;
each R^{x} is independently alkylene or a direct bond;
R^{v} is hydrogen, alkyl, alkenyl or alkynyl;
R^{w} is independently hydrogen, alkyl, alkenyl, alkynyl or haloalkyl;
R^{y} and R^{z} are selected as follows:
   (i) R^{y} and R^{z} are each independently hydrogen, alkyl, alkenyl, alkynyl, cycloalkyl or haloalkyl;
   (ii) R^{y} and R^{z}, together with the nitrogen atom to which they are attached, form a heterocyclyl or heteroaryl which arc optionally substituted with 1 to 2 groups each independently selected from halo, alkyl, haloalkyl, hydroxyl and alkoxy;
n is 0-4;
p is 0-5;
each q is independently 0, 1 or 2; and
r is 1-3.

In certain embodiments, provided herein are compounds of formula (I), wherein p is 1-5, and other variables are as described above. In certain embodiments, provided herein are compounds of formula (I), wherein p is 1 or 2, and other variables are as described above.

In certain embodiments, provided herein are compounds of formula (II) 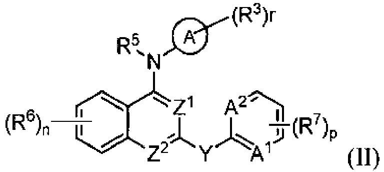 or pharmaceutically acceptable salts, solvates or hydrates thereof, wherein A¹ and A² arc each independently selected from N and CR^{7a}, R^{7a} is hydrogen or alkyl; and the other variables are as described elsewhere herein. In certain embodiments, A¹ and A² are CR^{7a}, R^{7a} is hydrogen; and the other variables are as described elsewhere herein. In certain embodiments, one of A¹ and A² is N and the other of A¹ and A² is CR^{7a} where R^{7a} is hydrogen or alkyl. In certain embodiments, provided herein are compounds of formula (II), wherein Z¹ and Z² are each independently N or CR⁰, such that one of Z¹ or Z² is N and the other is CR⁰;
ring A is azolyl;
A¹ and A² are each independently selected from N and CR^{7a},;
R^{7a} is hydrogen or alkyl;
Y is a linker selected from -C(R¹)(R²)-, -S(O)- and -S(O)₂-;
R⁰ is hydrogen, cyano or alkyl;
R¹ and R² are selected from (i), (ii), (iii), (iv) and (v) as follows:
   (i) R¹ and R² together form =O, =S, =NR⁹ or =CR¹⁰R¹¹;
   (ii) R¹ and R² are both -OR⁸, or R¹ and R², together with the carbon atom to which they are attached, form cycloalkyl or heterocyclyl wherein the cycloalkyl is substituted with one or more, in one embodiment, one to four, in one embodiment, one to three, in one embodiment, one or two, substituents selected from halo, deutero, alkyl, cycloalkyle, heterocyclyl, aryl, heteroaryl, cyano, =O, =N-OR²¹, -R^{x}OR²¹, -R^{x}N(R²²)₂, -R^{x}S(O)_{q}R²³, -C(O)R²¹, -C(O)OR²¹ and -C(O)N(R²²)₃, and wherein the heterocyclyl contains one to two heteroatoms each independently selected from O, NR²⁴, S, S(O) and S(O)₂;
   (iii) R¹ is hydrogen or halo; and R² is halo;
   (iv) R¹ is alkyl, alkenyl, alkynyl, cycloalkyl or aryl, wherein the alkyl, alkenyl, alkynyl, cycloalkyl and aryl are each optionally substituted with one or more, in one embodiment, one to four, in one embodiment, one to three, in one embodiment, one, two or three, substitutents selected from halo, cyano, alkyl, -R^{x}OR^{w}, -R^{x}S(O)_{q}R^{v}, -R^{x}NR^{y}R^{z} and -C(O)OR^{w}; and R² is hydrogen, halo or -OR⁸; and
   (v) R¹ is halo, deutero, -OR¹², -NR¹³R¹⁴, or -S(O)_{q}R¹⁵; and R² is hydrogen, deutero, alkyl, alkenyl, alkynyl, cycloalkyl or aryl, wherein the alkyl, alkenyl, alkynyl, cycloalkyl and aryl are each optionally substituted with one or more, in one embodiment, one to four, in one embodiment, one to three, in one embodiment, one, two or three, substitutents selected from halo, cyano, alky, -R^{x}OR^{w}, -R^{x}S(O)_{q}R^{v} and -R^{x}NR^{y}R^{z};
R³ is hydrogen, deutero, halo, alkyl, cyano, haloalkyl, deuteroalkyl, cycloalkyl, cycloalkylalkyl, hydroxy or alkoxy;
R⁵ is hydrogen or alkyl;
each R⁶ is independently selected from deutero, halo, nitro, cyano, alkyl, alkenyl, alkynyl, haloalkyl, cycloalkyl, aryl, arylalkyl, heteroaryl, heteroarylalkyl, heterocyclyl, heterocyclylalkyl, -R^{x}OR¹⁸, -R^{x}NR¹⁹R²⁰, -R^{x}C(O)NR^{y}R^{z},-R^{x}S(O)_{q}R^{v}, -R^{x}NR¹⁹C(O)R¹⁸, -R^{x}C(O)OR¹⁸ and -R^{x} NR¹⁹S(O)_{q}R^{v}; where the alkyl, alkenyl, alkynyl, haloalkyl, cycloalkyl, aryl, heteroaryl and heterocyclyl groups arc optionally substituted with one, two or three halo, oxo, hydroxy, alkoxy, alkyl, alkenyl, alkynyl, haloalkyl, or cycloalkyl groups:
each R⁷ is independently halo, alkyl, haloalkyl or -R^{x}OR^{w};
R⁸ is alkyl, alkenyl or alkynyl;
R⁹ is hydrogen, alkyl, haloalkyl, hydroxy, alkoxy or amino;
R¹⁰ is hydrogen or alkyl;
R¹¹ is hydrogen, alkyl, haloalkyl or -C(O)OR⁸;
R¹² is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, -C(O)R^{v}, -C(O)OR^{w} and -C(O)NR^{y}R^{z}, wherein the alkyl, alkenyl, alkynyl, cycloalkyl, cycloalkytatkyl, heterocyclyl, heterocyclylalkyl, aryl, aralkyl, heteroaryl and heteroaralkyl are each optionally substituted with one or more, in one embodiment, one to four, in one embodiment, one to three, in one embodiment, one, two or three, substituents independently selected from halo, oxo, alkyl, hydroxy, alkoxy, amino and alkylthio;
R¹³ and R¹⁴ arc selected as follows:
   (i) R¹³ is hydrogen or alkyl; and R¹⁴ is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, alkoxy, -C(O)R^{v}, -C(O)OR^{w}, -C(O)NR^{y}R^{z} and -S(O)_{q}R^{v}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more, in one embodiment, one to four, in one embodiment, one to three, in one embodiment, one, two or three, substituents independently selected from halo, oxo, alkyl, hydroxy, alkoxy, amino and alkylthio; or
   (ii) R¹³ and R¹⁴, together with the nitrogen atom to which they are attached, form heterocyclyl or heteroaryl wherein the heterocyclyl and heteroaryl are substituted with one or more, in one embodiment, one to four, in one embodiment, one to three, in one embodiment, one, two or three, substituents independently selected from halo, alkyl, hydroxy, alkoxy, amino and alkylthio and wherein the heterocyclyl is optionally substituted with oxo;
R¹⁵ is alkyl, alkenyl, alkynyl, cycloalkyl, cycloalkylalkyl, heterocyclyl, heterocyclylalkyl, aryl, aralkyl, heteroaryl, heteroaralkyl, -C(O)NR^{y}R^{z} or -NR^{y}R^{z}, wherein the alkyl, alkenyl, alkynyl, cycloalkyl, cycloalkylalkyl, heterocyclyl, heterocyclylalkyl, aryl, aralkyl, heteroaryl, and heteroaralkyl are each optionally substituted with one or more, in one embodiment, one to four, in one embodiment, one to three, in one embodiment, one, two or three, substituents independently selected from halo, oxo, alkyl, hydroxy, alkoxy, amino and alkylthio;
R¹⁸ is hydrogen, alkyl, haloalkyl, hydroxyalkyl, alkenyl, alkynyl, cycloalkyl, cycloalkylalkyl, heterocyclyl, heterocyclylalkyl, aryl, aralkyl, heteroaryl or heteroarylalkyl; wherein R¹⁸ is optionally substituted with 1 to 3 groups Q¹, each Q¹ independently selected from alkyl, hydroxy, halo, oxo, haloalkyl, alkoxy, aryloxy, alkoxyalkyl, alkoxycarbonyl, alkoxysulfonyl, carboxyl, cycloalkyl, heterocyclyl, aryl, heteroaryl, haloaryl and amino;
R¹⁹ and R²⁰ are selected as follows:
   (i) R¹⁹ and R²⁰ are each independently hydrogen or alkyl; or
   (ii) R¹⁹ and R²⁰, together with the nitrogen atom to which they are attached, form a heterocyclyl or heteroaryl which are each optionally substituted with 1 to 2 groups each independently selected from halo, oxo, alkyl, haloalkyl, hydroxyl and alkoxy;
R²¹ is hydrogen, alkyl, alkenyl, alkynyl, haloalkyl or cycloalkyl;
each R²² is independently hydrogen, alkyl, alkenyl, alkynyl, haloalkyl or cycloalkyl; or both R²², together with the nitrogen atom to which they are attached, form a heterocyclyl optionally substituted with oxo;
R²³ is alkyl, alkenyl, alkynyl or haloalkyl;
R²⁴ is hydrogen or alkyl;
each R^{x} is independently alkylene or a direct bond;
R^{v} is hydrogen, alkyl, alkenyl or alkynyl;
R^{w} is independently hydrogen, alkyl, alkenyl, alkynyl or haloalkyl;
R^{y} and R^{z} are selected as follows:
   (i) R^{y} and R^{z} are each independently hydrogen, alkyl, alkenyl, alkynyl, cycloalkyl, haloalkyl or heterocyclyl;
   (ii) R^{y} and R^{z}, together with the nitrogen atom to which they arc attached, form a heterocyclyl or heteroaryl which are optionally substituted with 1 to 2 groups each independently selected from halo, alkyl, haloalkyl, hydroxyl and alkoxy;
n is 0-4;
p is 0-5;
each q is independently 0, 1 or 2; and
r is 1-3.

In certain embodiments, provided herein are compounds of formula (II), wherein p is 1-5, and other variables are as described above. In certain embodiments, provided herein are compounds of formula (II), wherein p is 1 or 2, and other variables are as described above.

In certain embodiments, provided herein are compounds of formula (IIIa) or (IIIb) 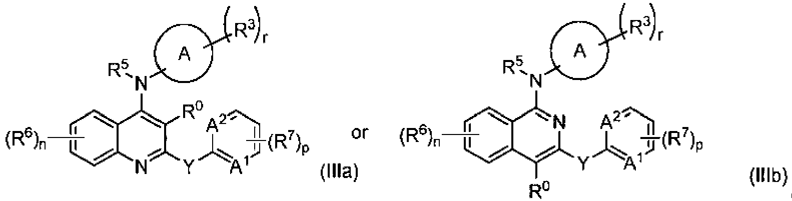 or pharmaceutically acceptable salts, solvates or hydrates thereof, wherein
A¹ and A² are each independently selected from N and CH;
Y is a linker selected from -C(R¹)(R²)-, -S(O)- and -S(O)₂-;
R⁰ is hydrogen, cyano or alkyl;
R¹ and R² are selected from (i), (ii), (iii), (iv) and (v) as follows:
   (i) R¹ and R² together form =O, =S, =NR⁹ or =CR¹⁰R¹¹;
   (ii) R¹ and R² are both -OR⁸, or R¹ and R², together with the carbon atom to which they are attached, form cycloalkyl or heterocyclyl wherein the cycloalkyl is substituted with one or more, in one embodiment, one to four, in one embodiment, one to three, in one embodiment, one or two, substitutents selected from halo, deutero, alkyl, cycloalkyl, heterocyclyl, aryl, heteroaryl, cyano, =O, =N-OR²¹, - R^{x}OR²¹, -R^{x}N(R²²)₂, -R^{x}S(O)_{q}R²³, -C(O)R²¹, -C(O)OR²¹ and -C(O)N(R²²)₂ and wherein the heterocyclyl contains one to two heteroatoms wherein each heteroatom is independently selected from O,NR, S, S(O) and S(O)₂;
   (iii) R¹ is hydrogen or halo; and R² is halo;
   (iv) R¹ is alkyl, alkenyl, alkynyl, cycloalkyl or aryl, wherein the alkyl, alkenyl, alkynyl, cycloalkyl and aryl are each optionally substituted with one or more, in one embodiment, one to four, in one embodiment, one to three, in one embodiment, one, two or three, substitutents selected from halo, cyano, alkyl, -R^{x}OR^{w}, -R^{x}S(O)_{q}R^{v}, -R^{x}NR^{y}R^{z} and -C(O)OR^{w}; and R² is hydrogen, halo or -OR⁸; and
   (v) R¹ is halo, deutero, -OR¹², -NR¹³R¹⁴, or -S(O)_{q}R¹⁵; and R² is hydrogen, deutero, alkyl, alkenyl, alkynyl, cycloalkyl or aryl, wherein the alkyl, alkenyl, alkynyl, cycloalkyl and aryl are each optionally substituted with one or more, in one embodiment, one to four, in one embodiment, one to three, in one embodiment, one, two or three, substitutents selected from halo, cyano, alkyl, -R^{x}OR^{w}, -R^{x}S(O)_{q}R^{v} and -R^{x}NR^{y}R^{z};
R³ is hydrogen, halo, alkyl, cyano, haloalkyl, cycloalkyl, cycloalkylalkyl, hydroxy or alkoxy;
R⁵ is hydrogen or alkyl;
each R⁶ is independently selected from deutero, halo, cyano, nitro, alkyl, alkenyl, alkynyl, haloalkyl, cycloalkyl, aryl, arylalkyl, heteroaryl, heteroarylalkyl, heterocyclyl, heterocyclylalkyl, -R^{x}OR¹⁸, -R^{x}NR¹⁹R²⁰, -R^{x}C(O)NR^{y}R^{z} and - R^{x}S(O)_{q}R^{v}; where the alkyl, alkenyl, alkynyl, haloalkyl, cycloalkyl, aryl, heteroaryl and heterocyclyl groups are optionally substituted with one, two or three halo, oxo, hydroxy, alkoxy, alkyl, alkenyl, alkynyl, haloalkyl, or cycloalkyl groups;
each R⁷ is independently halo, alkyl, haloalkyl or -R^{x}OR^{w};
R⁸ is alkyl, alkenyl or alkynyl;
R⁹ is hydrogen, alkyl, haloalkyl, hydroxy, alkoxy or amino;
R¹⁰ is hydrogen or alkyl;
R¹¹ is hydrogen, alkyl, haloalkyl or -C(O)OR⁸;
R¹² is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, -C(O)R^{v}, -C(O)OR^{w} and -C(O)NR^{y}R^{z}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more, in one embodiment, one to four, in one embodiment, one to three, in one embodiment, one, two or three, substitucnts independently selected from halo, oxo, alkyl, hydroxy, alkoxy, amino and alkylthio;
R¹³ and R¹⁴ are selected as follows:
   (i) R¹³ is hydrogen or alkyl; and R¹⁴ is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, alkoxy, -C(O)R^{v}, -C(O)OR^{w}, -C(O)NR^{y}R^{z} and -S(O)_{q}R^{v}, wherein the alkyl, alkenyl, alkynyl, cycloalkyl, cycloalkylalkyl, heterocyclyl, heterocyclylalkyl, aryl, aralkyl, heteroaryl and heteroaralkyl arc each optionally substituted with one or more, in one embodiment, one to four, in one embodiment, one to three, in one embodiment, one, two or three, substituents independently selected from halo, oxo, alkyl, hydroxy, alkoxy, amino and alkylthio; or
   (ii) R¹³ and R¹⁴, together with the nitrogen atom to which they are attached, form heterocyclyl or heteroaryl wherein the heterocyclyl and heteroaryl are substituted with one or more, in one embodiment, one to four, in one embodiment, one to three, in one embodiment, one, two or three, substituents independently selected from halo, alkyl, hydroxy, alkoxy, amino and alkylthio and wherein the heterocyclyl is optionally substituted with oxo;
R¹⁵ is alkyl, alkenyl, alkynyl, cycloalkyl, cycloalkylalkyl, heterocyclyl, heterocyclylalkyl, aryl, aralkyl, heteroaryl, heteroaralkyl, -C(O)NR^{y}R^{z} or -NR^{y}R^{z}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one or more, in one embodiment, one to four, in one embodiment, one to three, in one embodiment, one, two or three, substituents independently selected from halo, oxo, alkyl, hydroxy, alkoxy, amino and alkylthio;
R¹⁸ is hydrogen, alkyl, haloalkyl, hydroxyalkyl, alkenyl, alkynyl, cycloalkyl, cycloalkylalkyl, heterocyclyl, heterocyclylalkyl, aryl, aralkyl, heteroaryl or heteroarylalkyl; wherein R¹⁸ is optionally substituted with 1 to 3 groups Q¹, each Q¹ independently selected from alkyl, hydroxyl, halo, oxo, haloalkyl, alkoxy, aryloxy, alkoxyalkyl, alkoxycarbonyl, alkoxysulfonyl, carboxyl, cycloalkyl, heterocyclyl, aryl, heteroaryl, haloaryl and amino;
R¹⁹ and R²⁰ are selected as follows:
   (i) R¹⁹ and R²⁰ arc each independently hydrogen or alkyl; or
   (ii) R¹⁹ and R²⁰, together with the nitrogen atom to which they arc attached, form a heterocyclyl or heteroaryl which are each optionally substituted with 1 to 2 groups each independently selected from halo, oxo, alkyl, haloalkyl, hydroxyl and alkoxy;
R²¹ is hydrogen, alkyl, alkenyl, alkynyl, haloalkyl or cycloalkyl;
each R²² is independently hydrogen, alkyl, alkenyl, alkynyl, haloalkyl or cycloalkyl; or both R²², together with the nitrogen atom to which they are attached, form a heterocyclyl optionally substituted with oxo;
R²³ is alkyl, alkenyl, alkynyl or haloalkyl;
R²⁴ is hydrogen or alkyl;
each R^{x} is independently alkylene or a direct bond;
R^{v} is hydrogen, alkyl, alkenyl or alkynyl;
R^{w} is independently hydrogen, alkyl, alkenyl, alkynyl or haloalkyl;
R^{y} and R^{z} are selected as follows:
   (i) R^{y} and R^{z} are each independently hydrogen, alkyl, alkenyl, alkynyl, cycloalkyl or haloalkyl;
   (ii) R^{y} and R^{z}, together with the nitrogen atom to which they are attached, form a heterocyclyl or heteroaryl which are optionally substituted with 1 to 2 groups each independently selected from halo, alkyl, haloalkyl, hydroxyl and alkoxy;
n is 0-4;
r is 1-3;
p is 0-4; and
each q is independently 0, 1 or 2.

In certain embodiments, provided herein arc compounds of formula (IIIa) or (IIIB), wherein p is 1-4, and other variables are as described above. In certain embodiments, provided herein are compounds of formula (IIIa) or (IIIB), wherein p is 1 or 2, and other variables are as described above.

In certain embodiments, provided herein are compounds of formula (IIIa) or (IIIb) or pharmaceutically acceptable salts, solvates or hydrates thereof, wherein
A¹ and A² arc each independently selected from N and CH;
Y is a linker selected from -C(R¹)(R²)-, -S(O)- and -S(O)₂-;
R⁰ is hydrogen, cyano or alkyl;
R¹ and R² are selected as follows:
   (i) R¹ and R² together form =O;
   (ii) R¹ and R² arc both hydroxy or alkoxy, or R¹ and R², together with the carbon atom to which they arc attached, form cycloalkyl or heterocyclyl wherein the cycloalkyl is substituted with one or more, in one embodiment, one to four, in one embodiment, one to three, in one embodiment, one or two, substituents selected from halo, deutero, alkyl, haloalkyl, -OR²¹, ,-N(R²²)₂, and -S(O)_{q}R²³ and wherein the heterocyclyl contains one to two heteroatoms selected from O, NR²⁴, S, S(O) and S(O)₂;
   (iii) R¹ is hydrogen or halo; and R² is halo;
   (iv) R¹ is alkyl, alkenyl, alkynyl, cycloalkyl or aryl, and R² is hydrogen, halo, hydroxy or alkoxy; and
   (v) R¹ is halo, deutero, NR¹³R¹⁴, hydroxy or alkoxy; and R² is hydrogen, deutero, alkyl, alkenyl, alkynyl, cycloalkyl or aryl;
R³ is hydrogen, halo, alkyl, cyano, haloalkyl, deuteroalkyl, cycloalkyl, cycloalkylalkyl, hydroxy or alkoxy;
R⁵ is hydrogen or alkyl;
each R⁶ is independently selected from deutero, halo, alkyl, haloalkyl, cycloalkyl, aryl, heteroaryl, heterocyclyl and -R^{x}QR¹⁸; where the alkyl, haloalkyl, cycloalkyl, aryl, heteroaryl and heterocyclyl groups are optionally substituted with one, two or three halo, oxo, hydroxy, alkoxy, alkyl, alkenyl, alkynyl, haloalkyl, or cycloalkyl groups;
each R⁷ is independently halo or alkyl;
R⁸ is alkyl;
R¹³ is selected from hydrogen and alkyl;
R¹⁴ is selected from hydrogen, alkyl and cycloalkyl;
R¹⁸ is hydrogen or alkyl;
R²¹ is hydrogen, alkyl, alkenyl, alkynyl or haloalkyl;
each R²² is independently hydrogen, alkyl, alkenyl, alkynyl or haloalkyl;
R²³ is alkyl, alkenyl, alkynyl or haloalkyl;
R²⁴ is hydrogen or alkyl;
R^{x} is alkylene or a direct bond;
n is 0-4;
p is 0-4;
r is 1-3; and
each q is independently 0, 1 or 2.

In certain embodiments, provided herein are compounds of formula (IIIa) or (IIIb) or pharmaceutically acceptable salts, solvates or hydrates thereof, wherein
A¹ and A² are each independently selected from N and CH;
Y is a linker selected from -C(R¹)(R²)-, -S(O)- and -S(O)₂-;
R⁰ is hydrogen, cyano or alkyl;
R¹ and R² are selected as follows:
   (i) R¹ and R² together form =O;
   (ii) R¹ and R² are both hydroxy or alkoxy, or R¹ and R², together with the carbon atom to which they are attached, form cycloalkyl or heterocyclyl wherein the cycloalkyl is substituted with one or more, in one embodiment, one to four, in one embodiment, one to three, in one embodiment, one or two, substituents selected from halo, deutero, alkyl, haloalkyl, -OR²¹, -N(R²²)₂, and -S(O)_{q}R²³ and wherein the heterocyclyl contains one to two heteroatoms selected from O, NR²⁴, S, S(O) and S(O)₂;
   (iii) R¹ is hydrogen or halo; and R² is halo;
   (iv) R¹ is alkyl, alkenyl, alkynyl, cycloalkyl or aryl, and R² is hydrogen, halo, hydroxy or alkoxy; and
   (v) R¹ is halo, deutero, hydroxy or alkoxy; and R² is hydrogen, deutero, alkyl, alkenyl, alkynyl, cycloalkyl or aryl;
R³ is hydrogen, halo, alkyl, cyano, haloalkyl, cycloalkyl, cycloalkylalkyl, hydroxy or alkoxy;
R⁵ is hydrogen or alkyl;
each R⁶ is independently selected from deutero, halo, alkyl, haloalkyl, cycloalkyl, aryl, heteroaryl, heterocyclyl and -R^{x}OR¹⁸; where the alkyl, haloalkyl, cycloalkyl, aryl, heteroaryl and heterocyclyl groups arc optionally substituted with one, two or three halo, oxo, hydroxy, alkoxy, alkyl, alkenyl, alkynyl, haloalkyl, or cycloalkyl groups;
each R⁷ is independently halo or alkyl;
R⁸ is alkyl;
R¹⁸ is hydrogen or alkyl;
R²¹ is hydrogen, alkyl, alkenyl, alkynyl or haloalkyl;
each R²² is independently hydrogen, alkyl, alkenyl, alkynyl or haloalkyl;
R²³ is alkyl, alkenyl, alkynyl or haloalkyl;
R²⁴ is hydrogen or alkyl;
R^{x} is alkylene or a direct bond;
n is 0-4;
p is 0-4;
r is 1-3; and
each q is independently 0, 1 or 2.

In certain embodiments, provided herein are compounds of formula (IIIa) or (IIIB), wherein p is 1-4, and other variables are as described above. In certain embodiments, provided herein are compounds of formula (IIIa) or (IIIB), wherein p is 1 or 2, and other variables are as described above.

In certain embodiments, provided herein arc compounds of formula (IIIa) or (IIIb) or pharmaceutically acceptable salts, solvates or hydrates thereof, wherein
A¹ and A² are each independently selected from N and CH;
Y is a linker selected from -C(R¹)(R²)-, -S(O)- and -S(O)₂-;
R⁰ is hydrogen, cyano or alkyl;
   R¹ and R² are selected as follows:
      (i) R¹ and R² together form =O;
      (ii) R¹ is hydrogen or halo; and R² is halo;
      (iii) R¹ is alkyl, and R² is hydrogen, alkyl, halo, hydroxy or alkoxy; or
      (iv) R¹ is halo, hydroxy or alkoxy; and R² is hydrogen or alkyl;
R³ is hydrogen, halo, alkyl, hydroxy or alkoxy;
R⁵ is hydrogen or alkyl;
each R⁶ is independently deutero, halo or alkyl;
R⁷ halo;
n is 0 or 1;
p is 1 or 2; and
r is 1-3.

In certain embodiments, provided herein are compounds of formula (IVa) or (IVb) 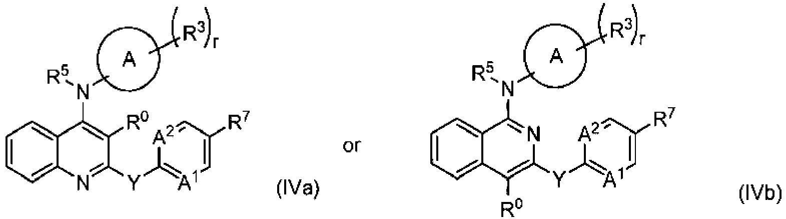 or pharmaceutically acceptable salts, solvates or hydrates thereof, wherein
A is pyrazolyl, imidazolyl, oxazolyl or thiazolyl;
A¹ and A² are each independently selected from N and CH;
Y is a linker selected from -C(R¹)(R²)-, -S(O)- and -S(O)₂-;
R⁰ is hydrogen, cyano or alkyl;
R¹ and R² are selected as follows:
   (i) R¹ and R² together form =O;
   (ii) R¹ is hydrogen or halo; and R² is halo;
   (iii) R¹ is alkyl, and R² is hydrogen, alkyl, halo, hydroxy or alkoxy; or
   (iv) R¹ is halo, NR¹³R¹⁴, hydroxy or alkoxy; and R² is hydrogen or alkyl;
R³ is hydrogen, halo, alkyl, hydroxy or alkoxy;
R⁵ is hydrogen or alkyl;
R¹³ is hydrogen or alkyl;
R¹⁴ is hydrogen, alkyl or cycloalkyl;
R⁷ halo; and
r is 1-3.

In certain embodiments, provided herein are compounds of formula (IVa) or (IVb)
or pharmaceutically acceptable salts, solvates or hydrates thereof, wherein
A is pyrazolyl, imidazolyl, oxazolyl or thiazolyl;
A¹ and A² are each independently selected from N and CH;
Y is a linker selected from -C(R¹)(R²)-, -S(O)- and -S(O)₂-;
R⁰ is hydrogen, cyano or alkyl;
R¹ and R² arc selected as follows:
   (i) R¹ and R² together form =O;
   (ii) R¹ is hydrogen or halo; and R² is halo;
   (iii) R¹ is alkyl, and R² is hydrogen, alkyl, halo, hydroxy or alkoxy; or
   (iv) R¹ is halo, hydroxy or alkoxy; and R² is hydrogen or alkyl;
R³ is hydrogen, halo, alkyl, deuteroalkyl, hydroxy or alkoxy;
R⁵ is hydrogen or alkyl;
R⁷ halo; and
r is 1-3.

In certain embodiments, provided herein are compounds of formula (Va) or (Vb) 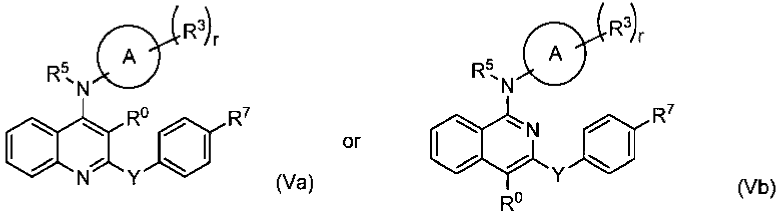 or pharmaceutically acceptable salts, solvates or hydrates thereof, wherein the variables are as described elsewhere herein.

In certain embodiments, provided herein are compounds of formula (Va) or (Vb), where
A is pyrazolyl, imidazolyl, oxazolyl or thiazolyl;
Y is a linker selected from -C(R¹)(R²)-, -S(O)- and -S(O)₂-;
R⁰ is hydrogen, cyano or alkyl;
   R¹ and R² are selected as follows:
      (i) R¹ and R² together form =O;
      (ii) R¹ is hydrogen or halo; and R² is halo;
      (iii) R¹ is alkyl, and R² is hydrogen, alkyl, halo, hydroxy or alkoxy; or
      (iv) R¹ is halo, NR¹³R¹⁴, hydroxy or alkoxy; and R² is hydrogen or alkyl;
R¹³ is hydrogen or alkyl;
R¹⁴ is hydrogen, alkyl or cycloalkyl;
R³ is hydrogen, halo, alkyl, hydroxy or alkoxy;
R⁵ is hydrogen or alkyl;
R⁷ halo; and
r is 1-3.

In certain embodiments, provided herein are compounds of formula (Va) or (Vb), where
A is pyrazolyl, imidazolyl, oxazolyl or thiazolyl;
Y is a linker selected from -C(R¹)(R²)-, -S(O)- and -S(O)₂-;
R⁰ is hydrogen, cyano or alkyl;
   R¹ and R³ are selected as follows:
      (i) R¹ and R² together form =O;
      (ii) R¹ is hydrogen or halo; and R² is halo;
      (iii) R¹ is alkyl, and R² is hydrogen, alkyl, halo, hydroxy or alkoxy; or
      (iv) R¹ is halo, hydroxy or alkoxy; and R² is hydrogen or alkyl;
R³ is hydrogen, halo, alkyl, hydroxy or alkoxy;
R⁵ is hydrogen or alkyl;
R⁷ halo; and
ris 1-3.

In certain embodiments, provided herein are compounds of formula (VIa) or (VIb) 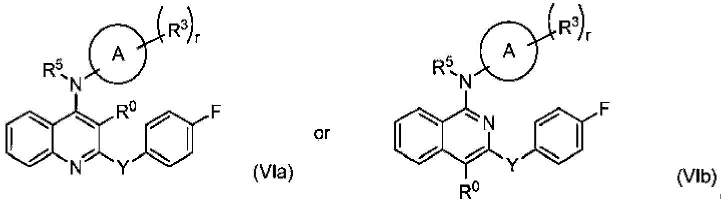 or pharmaceutically acceptable salts, solvates or hydrates thereof, wherein the variables are as described elsewhere herein.

In one embodiment, provided herein are compounds of formula formula (VIa) or (VIb), or pharmaceutically acceptable salts, solvates or hydrates thereof, wherein
A is pyrazolyl, imidazolyl, oxazolyl or thiazalyl;
Y is a linker selected from -C(R¹)(R²)-, -S(O)- and -S(O)₂-;
R⁰ is hydrogen, cyano or alkyl;
   R¹ and R² are selected as follows:
      (i) R¹ and R² together form =O;
      (ii) R¹ is hydrogen or halo; and R² is halo;
      (iii) R¹ is alkyl, and R² is hydrogen, alkyl, halo, hydroxy or alkoxy; or
      (iv) R¹ is halo, hydroxy, amino, alkylamino, cycloalkylamino or alkoxy; and R² is hydrogen or alkyl;
R³ is hydrogen, halo, alkyl, deuteroalkyl, hydroxy or alkoxy;
R⁵ is hydrogen; and
r is 1-3.

In one embodiment, provided herein arc compounds of formula formula (VIa) or (VIb), or pharmaceutically acceptable salts, solvates or hydrates thereof, wherein
A is pyrazolyl, imidazolyl, oxazolyl or thiazolyl;
Y is a linker selected from -C(R¹)(R²)-, -S(O)- and -S(O)₂-;
R⁰ is hydrogen, cyano or alkyl;
   R¹ and R² are selected as follows:
      (i) R¹ and R² together form =O;
      (ii) R¹ is hydrogen or halo; and R² is halo;
      (iii) R¹ is alkyl, and R² is hydrogen, alkyl, halo, hydroxy or alkoxy; or
      (iv) R¹ is halo, hydroxy or alkoxy; and R² is hydrogen or alkyl;
R³ is hydrogen, halo, alkyl, hydroxy or alkoxy;
R⁵ is hydrogen; and
r is 1-3.

In one embodiment, A is pyrazolyl, imidazolyl, oxazolyl, thiazolyl, thiadiazolyl, or triazolyl. In one embodiment, A is pyrazolyl. In one embodiment, A is imidazolyl.

In one embodiment, A is 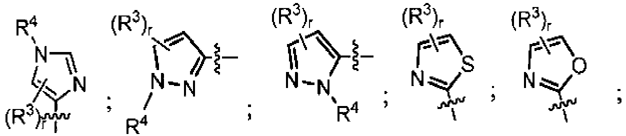 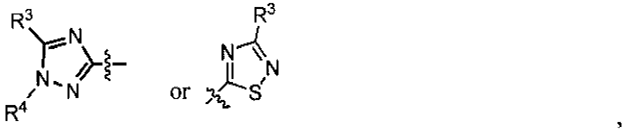 wherein each R³ is independently hydrogen, halo, alkyl, cyano, haloalkyl, cycloalkyl, cycloalkylalkyl, hydroxy or alkoxy; r is 1 or 2; and each R⁴ is independently hydrogen or alkyl.

In one embodiment, A is 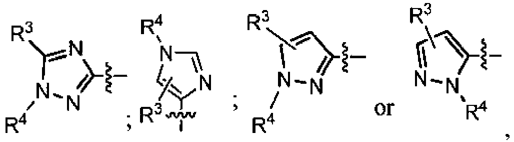 wherein each R³ is independently hydrogen, halo, alkyl, hydroxy or alkoxy; and each R⁴ is independently hydrogen, or alkyl.

In one embodiment, A is 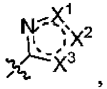 wherein X¹, X² and X³ are selected from (i) through (v) as follows
(i) X¹ is NR⁴, X² is CR³ and X³ is CH;
(ii) X¹ is CR³, X² is NR⁴ and X³ is CH;
(iii) X¹ is CR³, X² is NR⁴ and X³ is S;
(iv) X¹ is CR³, X² is S or O and X³ is CR³; and
(v) X¹ is CR³, X² is CR³ and X³ is S or O;
and the other variables are as described elsewhere herein.

In one embodiment, A is 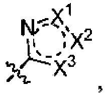 wherein X¹, X² and X³ are selected from (i) through (iv) as follows
(i) X¹ is NR⁴, X² is CR³ and X³ is CH;
(ii) X¹ is CR³, X² is NR⁴ and X³ is CH;
(iii) X¹ is CR³, X² is NR⁴ and X³ is S; and
(iv) X¹ is CR³, X² is CR³ and X³ is S;
and the other variables are as described elsewhere herein.

In one embodiment, A is 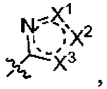 wherein X¹, X² and X³ are selected from (i) and (ii) as follows
(v) X¹ is NR⁴, X² is CR³ and X³ is CH; and
(vi) X¹ is CH, X² is CR³ and X³ is S,
and the other variables are as described elsewhere herein.

In one embodiment, A is 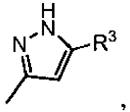 where R³ is hydrogen or alkyl. In one embodiment, R³ is hydrogen or methyl,

In one embodiment, provided herein is a compound of Formula (VIIa) or (VIIb): 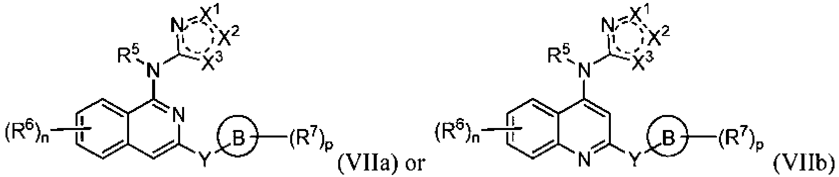 wherein X¹, X² and X³ are selected from (i) and (iii) as follows
(i) X¹ is NR⁴, X² is CR³ and X³ is CH;
(ii) X¹ is CH, X² is CR³ and X³ is S; and
(iii) X¹ is CH, X² is NR⁴ and X³ is CH;
R⁴ is hydrogen, or alkyl;
and the other variables are as described elsewhere herein.

In one embodiment, X¹ is NR⁴, X² is CR³ and X³ is CH.

In one embodiment, R¹ and R² together form =O.

In one embodiment, R¹ and R², together with the carbon atom to which they are attached, form cycloalkyl or heterocyclyl wherein the cycloalkyl is substituted with one or more, in one embodiment, one to four, in one embodiment, one to three, in one embodiment, one or two, substitutents selected from halo, deutero, alkyl, cycloalkyl, hetcrocyclyl, aryl, heteroaryl, cyano, =O, =N-OR²¹, -R^{x}OR²¹,-R^{x}N(R²²)₂, -R^{x}S(O)_{q}R²³, -C(O)R²¹, -C(O)OR²¹ and -C(O)N(R²²)₂ and wherein the heterocyclyl contains not more than two heteroatoms wherein the first heteroatom is selected from O, NR²⁴, S, S(O) and S(O)₂ and the second optional heteroatom is selected from NR²⁴, S, S(O) and S(O)₂.

In one embodiment, R¹ and R² are both halo. In one embodiment, R¹ and R² are both fluoro.

In one embodiment, R¹ is hydrogen or alkyl, and R² is hydroxy. In one embodiment, R¹ is hydrogen or methyl, and R² is hydroxy. In one embodiment, R¹ is halo, hydroxy, amino, alkylamino, cycloalkylamino or alkoxy; and R² is hydrogen or alkyl.

In one embodiment, Y is a linker selected from -C(R¹)(R²)-, -S(O)- and-S(O)₂-;
R¹ and R² are selected as follows:
   (i) R¹ and R² together form =O or dioxacycloalkyl;
   (ii) R¹ is hydrogen or halo; and R² is halo;
   (iii) R¹ is alkyl, and R² is hydrogen, alkyl, halo, hydroxy or alkoxy; or
   (iv) R¹ is halo, hydroxyl, alkoxy or amino; and R² is hydrogen or alkyl; and
R³ is hydrogen, halo, alkyl, hydroxy or alkoxy.

In one embodiment, R³ is hydrogen, halo, alkyl, deuteroalkyl, cycloalkyl, hydroxy or alkoxy. In one embodiment, R³ is hydrogen, halo, alkyl, cycloalkyl, hydroxy or alkoxy, In one embodiment, R³ is hydrogen, halo, alkyl, hydroxy or alkoxy. In one embodiment, R³ is hydrogen, halo, alkyl, deuteroalkyl, hydroxy or alkoxy. In another embodiment, R³ is hydrogen, methyl, hydroxy or methoxy. In another embodiment, R³ is hydrogen, methyl, -CD₃, hydroxy or methoxy.

In one embodiment, R⁰ is hydrogen. In one embodiment, R⁰ is alkyl. In one embodiment, R⁰ is cyano.

In one embodiment, R⁷ is halo. In one embodiment, R⁷ is fluoro.

In one embodiment, p is 1 or 2. In one embodiment, p is 1.

In one embodiment, provided herein is a compound selected from 3-((4-fluorophenyl)sulfinyl)-N-(5-methyl-1H-pyrazol-3-yl)isoquinolin-1-amine; (4-fluorophenyl)(1-((5-methyl-1H-pyrazol-3-yl)amino)isoquinolin-3-yl)methanone; (4-fluorophenyl)(1-((5-methyl-1H-pyrazol-3-yl)amino)isoquinolin-3-yl)methanol; N-(3-((4-fluorophenyl)sulfinyl)isoquinolin-1-yl)thiazol-2-amine; N-(3-((4-fluorophenyl)sulfinyl)isoquinolin-1-yl)-5-methylthiazol-2-amine; N-(3-((4-fluorophcnyl)sulfinyl)isoquinolin-1-yl)-4-methyloxazol-2-amine; 3-((4-fluorophenyl)sulfinyl)-N-(4-methoxypyridin-2-yl)isoquinolin-1-amine; (4-fluorophenyl)(4-(4-fluorophenyl)-1-((5-methyl-1H-pyrazol-3-yl)amino)isoquinolin-3-yl)methanone; (4-fluorophenyl)(4-(4-fluorophenyl)-1-((5-methyl-1H-pyrazol-3-yl)amino)isoquinolin-3-yl)methanol; (4-fluorophenyl)(4-(4-fluorophenyl)-1-((5-methyl-1H-pyrazol-3-yl)amino)isoquinolin-3-yl)methyl formate; (4-fluorophenyl)(4-(2-methoxyethoxy)-1-((5-methyl-1H-pyrazol-3-yl)amino)isoquinotin-3-yl)methanone; 3-((4-fluorophenyl)sulfinyl)-1-(1H-pyrazol-4-yl)isoquinoline; 3-((4-fluorophenyl)sulfonyl)-N-(5-methyl-1H-pyrazol-3-yl)isoquinolin-1-amine; 3-((4-fluorophenyl)sulfonyl)-N-(5-methyl-1H-pyrazol-3-yl)-4-nitroisoquinolin-1-amine; 3-((4-fluorophenyl)sulfonyl)-N-(1H-pyrazol-3-yl)isoquinolin-1-amine; 3-((4-fluorophenyl)sulfonyl)-N-(5-methoxy-1H-pyrazol-3-yl)isoquinolin-1-amine; N-(3-((4-fluorophenyl)sulfonyl)isoquinolin-1-yl)-5-methylthiazol-2-amine; N-(3-((4-fluorophenyl)sulfonyl)isoquinolin-1-yl)thiazol-2-amine; N-(3-((4-fluorophenyl)sulfonyl)isoquinolin-1-yl)oxazol-2-amine; 3-((3-((4-fluoropheny))sulfonyl)isoquinolin-1-yl)amino)-1H-pyrazol-5-ol; N-(3-((4-fluorophenyl)sulfonyl)isoquinolin-1-yl)-1,2,4-thiadiazol-5-amine; and 3-((4-fluorophenyl)sulfonyl)-N-(1-methyl-1H-imidazol-4-yl)isoquinolin-1-amine; or a pharmaceutically acceptable salt, solvate or hydrate thereof. In another embodiment, provided herein are compounds selected from: 3-((4-fluorophenyl)sulfonyl)-N-(1-methyl-*d₃*-1H-imidazol-4-yl)isoquinolin-1-amine; N-(1-ethyl-1H-imidazol-4-yl)-3-((4-fluorophenyl)sulfonyl)isoquinolin-1-amine; and 5-bromo-3-((4-fluorophenyl)sulfonyl)-N-(5-methyl-1H-pyrazol-3-yl)isoquinolin-1-amine, or a pharmaceutically acceptable salt, solvate or hydrate thereof.

Also provided herein are isotopically enriched analogs of the compounds provided herein. Isotopic enrichment (for example, deuteration) of pharmaceuticals to improve pharmacokinetics ("PK"), pharmacodynamics ("PD"), and toxicity profiles, has been demonstrated previously with some classes of drugs. See, for example, Lijinsky et. al., Food Cosmet. Toxicol., 20: 393 (1982); Lijinsky et. al., J. Nat. Cancer Inst., 69: 1127 (1982); Mangold et. al., Mutation Res. 308: 33 (1994); Gordon et. al., Drug Metab. Dispos., 15: 589 (1987); Zello et. al., Metabolism, 43: 487 (1994); Gatcly et. al., J. Nucl Med., 27: 388 (1986); Wade D, Chem. Biol. Interact. 117: 191 (1999).

Isotopic enrichment of a drug can be used, for example, to (1) reduce or eliminate unwanted metabolites, (2) increase the half-life of the parent drug, (3) decrease the number of doses needed to achieve a desired effect, (4) decrease the amount of a dose necessary to achieve a desired effect, (5) increase the formation of active metabolites, if any are formed, and/or (6) decrease the production of deleterious metabolites in specific tissues and/or create a more effective drug and/or a safer drug for combination therapy, whether the combination therapy is intentional or not.

Replacement of an atom for one of its isotopes often will result in a change in the reaction rate of a chemical reaction. This phenomenon is known as the Kinetic Isotope Effect ("KIE"). For example, if a C-H bond is broken during a rate-determining step in a chemical reaction (*i.e*. the step with the highest transition state energy), substitution of a deuterium for that hydrogen will cause a decrease in the reaction rate and the process will slow down. This phenomenon is known as the Deuterium Kinetic Isotope Effect ("DKIE"). (*See, e.g,* Foster et al., Adv. Drug Res., vol. 14, pp. 1-36 (1985); Kushner et al., Can. J. Physiol. Pharmacol., vol. 77, pp. 79-88 (1999)).

Tritium ("T") is a radioactive isotope of hydrogen, used in research, fusion reactors, neutron generators and radiopharmaceuticals. Tritium is a hydrogen atom that has 2 neutrons in the nucleus and has an atomic weight close to 3. It occurs naturally in the environment in very low concentrations, most commonly found as T₂O. Tritium decays slowly (half-life = 12.3 years) and emits a low energy beta particle that cannot penetrate the outer layer of human skin. Internal exposure is the main hazard associated with this isotope, yet it must be ingested in large amounts to pose a significant health risk. As compared with deuterium, a lesser amount of tritium must be consumed before it reaches a hazardous level. Substitution of tritium ("T") for hydrogen results in yet a stronger bond than deuterium and gives numerically larger isotope effects. Similarly, substitution of isotopes for other elements, including, but not limited to, ¹³C or ¹⁴C for carbon, ³³5, ³⁴S, or ³⁶S for sulfur, ¹⁵N for nitrogen, and ¹⁷O or ¹⁸O for oxygen, will provide a similar kinetic isotope effects.

### C. FORMULATION OF PHARMACEUTICAL COMPOSITIONS

Provided herein are pharmaceutical compositions comprising a compound provided herein, e.g., a compound of Formula 1, as an active ingredient, or a pharmaceutically acceptable salt, solvate or hydrate thereof; in combination with a pharmaceutically acceptable vehicle, carrier, diluent, or excipient, or a mixture thereof.

The compound provided herein may be administered alone, or in combination with one or more other compounds provided herein. The pharmaceutical compositions that comprise a compound provided herein, e.g., a compound of Formula I, can be formulated in various dosage forms for oral, parenteral, and topical administration. The pharmaceutical compositions can also be formulated as modified release dosage forms, including delayed-, extended-, prolonged-, sustained-, pulsatile-, controlled-, accelerated- and fast-, targeted-, programmed-release, and gastric retention dosage forms. These dosage forms can be prepared according to conventional methods and techniques known to those skilled in the art (*see, Remington: The Science and Practice of Pharmacy,* supra; Modified-Release Drug Deliver Technology, Rathbone et al., Eds., Drugs and the Pharmaceutical Science, Marcel Dekker, Inc.: New York, NY, 2003; Vol. 126).

In one embodiment, the pharmaceutical compositions are provided in a dosage form for oral administration, which comprise a compound provided herein, e.g., a compound of Formula I, or a pharmaceutically acceptable salt, solvate or hydrate thereof; and one or more pharmaceutically acceptable excipients or carriers.

In another embodiment, the pharmaceutical compositions are provided in a dosage form for parenteral administration, which comprise a compound provided herein, e.g., a compound of Formula I, or a pharmaceutically acceptable salt, solvate or hydrate thereof; and one or more pharmaceutically acceptable excipients or carriers.

In yet another embodiment, the pharmaceutical compositions are provided in a dosage form for topical administration, which comprise a compound provided herein, e.g., a compound of Formula I, or a pharmaceutically acceptable salt, solvateor hydrate thereof; and one or more pharmaceutically acceptable excipients or carriers.

The pharmaceutical compositions provided herein can be provided in a unit-dosage form or multiple-dosage form. A unit-dosage form, as used herein, refers to physically discrete a unit suitable for administration to a human and animal subject, and packaged individually as is known in the art. Each unit-dose contains a predetermined quantity of an active ingredient(s) sufficient to produce the desired therapeutic effect, in association with the required pharmaceutical carriers or excipients. Examples of a unit-dosage form include an ampoule, syringe, and individually packaged tablet and capsule. A unit-dosage form may be administered in fractions or multiples thereof. A multiple-dosage form is a plurality of identical unit-dosage forms packaged in a single container to be administered in segregated unit-dosage form. Examples of a multiple-dosage form include a vial, bottle of tablets or capsules, or bottle of pints or gallons.
The pharmaceutical compositions provided herein can be administered at once, or multiple times at intervals of time. It is understood that the precise dosage and duration of treatment may vary with the age, weight, and condition of the patient being treated, and may be determined empirically using known testing protocols or by extrapolation from *in vivo* or *in vitro* test or diagnostic data. It is further understood that for any particular individual, specific dosage regimens should be adjusted over time according to the individual need and the professional judgment of the person administering or supervising the administration of the formulations.

In one embodiment, the therapeutically effective dose is from about 0.1 mg to about 2,000 mg per day of a compound provided herein. The pharmaceutical compositions therefore should provide a dosage of from about 0.1 mg to about 2000 mg of the compound. In certain embodiments, pharmaceutical dosage unit forms are prepared to provide from about 1 mg to about 2000 mg, from about 10 mg to about 1000 mg, from about 20 mg to about 500 mg or from about 25 mg to about 250 mg of the essential active ingredient or a combination of essential ingredients per dosage unit form. In certain embodiments, the pharmaceutical dosage unit forms are prepared to provide about 10 mg, 20 mg, 25 mg, 50 mg, 100 mg, 250 mg, 500 mg, 1000 mg or 2000 mg of the essential active ingredient.

### Oral Administration

The pharmaceutical compositions provided herein can be provided in solid, semisolid, or liquid dosage forms for oral administration. As used herein, oral administration also includes buccal, lingual, and sublingual administration. Suitable oral dosage forms include, but are not limited to, tablets, fastmelts, chewable tablets, capsules, pills, troches, lozenges, pastilles, cachets, pellets, medicated chewing gum, bulk powders, effervescent or non-effervescent powders or granules, solutions, emulsions, suspensions, wafers, sprinkles, elixirs, and syrups. In addition to the active ingredient(s), the pharmaceutical compositions can contain one or more pharmaceutically acceptable carriers or excipients, including, but not limited to, binders, fillers, diluents, disintegrants, wetting agents, lubricants, glidants, coloring agents, dye-migration inhibitors, sweetening agents, and flavoring agents.

Binders or granulators impart cohesiveness to a tablet to ensure the tablet remaining intact after compression. Suitable binders or granulators include, but are not limited to, starches, such as corn starch, potato starch, and pre-gelatinized starch (e.g., STARCH 1500); gelatin; sugars, such as sucrose, glucose, dextrose, molasses, and lactose; natural and synthetic gums, such as acacia, alginic acid, alginates, extract of Irish moss, panwar gum, ghatti gum, mucilage of isabgol husks, carboxymethylcellulose, methylcellulose, polyvinylpyrrolidone (PVP), Veegum, larch arabogalactan, powdered tragacanth, and guar gum; celluloses, such as ethyl cellulose, cellulose acetate, carboxymethyl cellulose calcium, sodium carboxymethyl cellulose, methyl cellulose, hydroxyethylcellulose (HEC), hydroxypropylcellulose (HPC), hydroxypropyl methyl cellulose (HPMC); microcrystalline celluloses, such as AVICEL-PH-101, AVICEL-PH-103, AVICEL RC-581, AVICEL-PH-105 (FMC Corp., Marcus Hook, PA); and mixtures thereof. Suitable fillers include, but are not limited to, talc, calcium carbonate, microcrystalline cellulose, powdered cellulose, dextrates, kaolin, mannitol, silicic acid, sorbitol, starch, pre-gelatinized starch, and mixtures thereof. The binder or filler may be present from about 50 to about 99% by weight in the pharmaceutical compositions provided herein.

Suitable diluents include, but are not limited to, dicalcium phosphate, calcium sulfate, lactose, sorbitol, sucrose, inositol, cellulose, kaolin, mannitol, sodium chloride, dry starch, and powdered sugar. Certain diluents, such as mannitol, lactose, sorbitol, sucrose, and inositol, when present in sufficient quantity, can impart properties to some compressed tablets that permit disintegration in the mouth by chewing. Such compressed tablets can be used as chewable tablets.

Suitable disintegrants include, but are not limited to, agar; bentonite; celluloses, such as methylcellulose and carboxymethylcellulose; wood products; natural sponge; cation-exchange resins; alginic acid; gums, such as guar gum and Veegum HV; citrus pulp; cross-linked celluloses, such as croscarmellose; cross-linked polymers, such as crospovidone; cross-linked starches; calcium carbonate; microcrystalline cellulose, such as sodium starch glycolate; polacrilin potassium; starches, such as corn starch, potato starch, tapioca starch, and pre-gelatinized starch; clays; aligns; and mixtures thereof. The amount of a disintegrant in the pharmaceutical compositions provided herein varies upon the type of formulation, and is readily discernible to those of ordinary skill in the art. The pharmaceutical compositions provided herein may contain from about 0.5 to about 15% or from about 1 to about 5% by weight of a disintegrant.

Suitable lubricants include, but are not limited to, calcium stearate; magnesium stearate; mineral oil; light mineral oil; glycerin; sorbitol; mannitol; glycols, such as glycerol behenate and polyethylene glycol (PEG); stearic acid; sodium lauryl sulfate; talc; hydrogenated vegetable oil, including peanut oil, cottonseed oil, sunflower oil, sesame oil, olive oil, corn oil, and soybean oil; zinc stearate; ethyl oleate; ethyl laureate; agar; starch; lycopodium; silica or silica gels, such as AERoSIL^{®} 200 (W.R. Grace Co., Baltimore, MD) and CAB-O-SIL® (Cabot Co. of Boston, MA); and mixtures thereof. The pharmaceutical compositions provided herein may contain about 0.1 to about 5% by weight of a lubricant.

Suitable glidants include colloidal silicon dioxide, CAB-O-SIL^{®} (Cabot Co. of Boston, MA), and asbestos-free talc. Coloring agents include any of the approved, certified, water soluble FD&C dyes, and water insoluble FD&C dyes suspended on alumina hydrate, and color lakes and mixtures thereof. A color lake is the combination by adsorption of a water-soluble dye to a hydrous oxide of a heavy metal, resulting in an insoluble form of the dye. Flavoring agents include natural flavors extracted from plants, such as fruits, and synthetic blends of compounds which produce a pleasant taste sensation, such as peppermint and methyl salicylate. Sweetening agents include sucrose, lactose, mannitol, syrups, glycerin, and artificial sweeteners, such as saccharin and aspartame. Suitable emulsifying agents include gelatin, acacia, tragacanth, bentonite, and surfactants, such as polyoxyethylene sorbitan monooleate (TWEEN^{®} 20), polyoxyethylene sorbitan monooleate 80 (TWEEN^{®} 80), and triethanolamine oleate. Suspending and dispersing agents include sodium carboxymethylcellulose, pectin, tragacanth, Veegum, acacia, sodium carbomethylcellulose, hydroxypropyl methylcellulose, and polyvinylpyrrolidone. Preservatives include glycerin, methyl and propylparaben, benzoic add, sodium benzoate and alcohol. Wetting agents include propylene glycol monostearate, sorbitan monooleate, diethylene glycol monolaurate, and polyoxyethylene lauryl ether. Solvents include glycerin, sorbitol, ethyl alcohol, and syrup. Examples of non-aqueous liquids utilized in emulsions include mineral oil and cottonseed oil. Organic acids include citric and tartaric acid. Sources of carbon dioxide include sodium bicarbonate and sodium carbonate.

It should be understood that many carriers and excipients may serve several functions, even within the same formulation.

The pharmaceutical compositions provided herein can be provided as compressed tablets, tablet triturates, chewable lozenges, rapidly dissolving tablets, multiple compressed tablets, or enteric-coating tablets, sugar-coated, or film-coated tablets. Enteric-coated tablets are compressed tablets coated with substances that resist the action of stomach acid but dissolve or disintegrate in the intestine, thus protecting the active ingredients from the acidic environment of the stomach. Enteric-coatings include, but arc not limited to, fatty acids, fats, phenyl salicylate, waxes, shellac, ammoniatcd shellac, and cellulose acetate phthalates. Sugar-coated tablets are compressed tablets surrounded by a sugar coating, which may be beneficial in covering up objectionable tastes or odors and in protecting the tablets from oxidation. Film-coated tablets are compressed tablets that are covered with a thin layer or film of a water-soluble material. Film coatings include, but arc not limited to, hydroxyethylcellulose, sodium carboxymethylcellulose, polyethylene glycol 4000, and cellulose acetate phthalate. Film coating imparts the same general characteristics as sugar coating. Multiple compressed tablets are compressed tablets made by more than one compression cycle, including layered tablets, and press-coated or dry-coated tablets.

The tablet dosage forms can be prepared from the active ingredient in powdered, crystalline, or granular forms, alone or in combination with one or more carriers or excipients described herein, including binders, disintegrants, controlled-release polymers, lubricants, diluents, and/or colorants. Flavoring and sweetening agents are especially useful in the formation of chewable tablets and lozenges.

The pharmaceutical compositions provided herein can be provided as soft or hard capsules, which can be made from gelatin, methylcellulose, starch, or calcium alginate. The hard gelatin capsule, also known as the dry-filled capsule (DFC), consists of two sections, one slipping over the other, thus completely enclosing the active ingredient. The soft elastic capsule (SEC) is a soft, globular shell, such as a gelatin shell, which is plasticized by the addition of glycerin, sorbitol, or a similar polyol. The soft gelatin shells may contain a preservative to prevent the growth of microorganisms. Suitable preservatives are those as described herein, including methyl- and prapyl-parabens, and sorbic acid. The liquid, semisolid, and solid dosage forms provided herein may be encapsulated in a capsule. Suitable liquid and semisolid dosage forms include solutions and suspensions in propylene carbonate, vegetable oils, or triglycerides. Capsules containing such solutions can be prepared as described in U.S. Pat. Nos. 4,328,245; 4,409,239; and 4,410,545. The capsules may also be coated as known by those of skill in the art in order to modify or sustain dissolution of the active ingredient.

The pharmaceutical compositions provided herein can be provided in liquid and semisolid dosage forms, including emulsions, solutions, suspensions, elixirs, and syrups. An emulsion is a two-phase system, in which one liquid is dispersed in the form of small globules throughout another liquid, which can be oil-in-water or water-in-oil. Emulsions may include a pharmaceutically acceptable non-aqueous liquid or solvent, emulsifying agent, and preservative. Suspensions may include a pharmaceutically acceptable suspending agent and preservative. Aqueous alcoholic solutions may include a pharmaceutically acceptable acetal, such as a di(lower alkyl) acetal of a lower alkyl aldehyde, e.g., acetaldehyde diethyl acetal; and a water-miscible solvent having one or more hydroxyl groups, such as propylene glycol and ethanol. Elixirs are clear, sweetened, and hydroalcoholic solutions. Syrups are concentrated aqueous solutions of a sugar, for example, sucrose, and may also contain a preservative. For a liquid dosage form, for example, a solution in a polyethylene glycol may be diluted with a sufficient quantity of a pharmaceutically acceptable liquid carrier, e.g., water, to be measured conveniently for administration.

Other useful liquid and semisolid dosage forms include, but are not limited to, those containing the active ingredient(s) provided herein, and a dialkylated mono- or poly-alkylene glycol, including, 1,2-dimethoxymethane, diglyme, triglyme, tetraglyme, polyethylene glycol-350-dimethyl ether, polyethylene glycol-550-dimethyl ether, polyethylene glycol-750-dimethyl ether, wherein 350, 550, and 750 refer to the approximate average molecular weight of the polyethylene glycol. These formulations can further comprise one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, bisulfite, sodium metabisulfite, thiodipropionic acid and its esters, and dithiocarbamates.

The pharmaceutical compositions provided herein for oral administration can be also provided in the forms of liposomes, micelles, microspheres, or nanosystems. Micellar dosage forms can be prepared as described in U.S. Pat. No. 6,350,458.

The pharmaceutical compositions provided herein can be provided as non- effervescent or effervescent, granules and powders, to be reconstituted into a liquid dosage form. Pharmaceutically acceptable carriers and excipients used in the non-cffcrvcscent granules or powders may include diluents, sweeteners, and wetting agents. Pharmaceutically acceptable carriers and excipients used in the effervescent granules or powders may include organic acids and a source of carbon dioxide.

Coloring and flavoring agents can be used in all of the above dosage forms.

The pharmaceutical compositions provided herein can be formulated as immediate or modified release dosage forms, including delayed-, sustained, pulsed-, controlled, targeted-, and programmed-release forms.

The pharmaceutical compositions provided herein can be co-formulated with other active ingredients which do not impair the desired therapeutic action, or with substances that supplement the desired action.

### Parenteral Administration

The pharmaceutical compositions provided herein can be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial, intravesical, and subcutaneous administration.

The pharmaceutical compositions provided herein can be formulated in any dosage forms that are suitable for parenteral administration, including solutions, suspensions, emulsions, micelles, liposomes, microspheres, nanosystems, and solid forms suitable for solutions or suspensions in liquid prior to injection. Such dosage forms can be prepared according to conventional methods known to those skilled in the art of pharmaceutical science (*see, Remington: The Science and Practice of Pharmacy*, supra).

The pharmaceutical compositions intended for parenteral administration can include one or more pharmaceutically acceptable carriers and excipients, including, but not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotcctants, lyoprotectants, thickening agents, pH adjusting agents, and inert gases.

Suitable aqueous vehicles include, but are not limited to, water, saline, physiological saline or phosphate buffered saline (PBS), sodium chloride injection, Ringers injection, isotonic dextrose injection, sterile water injection, dextrose and lactated Ringers injection. Non-aqueous vehicles include, but are not limited to, fixed oils of vegetable origin, castor oil, corn oil, cottonseed oil, olive oil, peanut oil, peppermint oil, safflower oil, sesame oil, soybean oil, hydrogenated vegetable oils, hydrogenated soybean oil, and medium-chain triglycerides of coconut oil, and palm seed oil. Water-miscible vehicles include, but are not limited to, ethanol, 1,3-butanediol, liquid polyethylene glycol (e.g., polyethylene glycol 300 and polyethylene glycol 400), propylene glycol, glycerin, *N*-methyl-2-pyrrolidone, *N,N-*dimethylacetamide, and dimethyl sulfoxide.

Suitable antimicrobial agents or preservatives include, but are not limited to, phenols, cresols, mercurials, benzyl alcohol, chlorobutanol, methyl and propyl p-hydroxybenzoates, thimerosal, benzalkonium chloride (e.g., benzethonium chloride), methyl- and propyl-parabens, and sorbic acid. Suitable isotonic agents include, but are not limited to, sodium chloride, glycerin, and dextrose. Suitable buffering agents include, but are not limited to, phosphate and citrate. Suitable antioxidants are those as described herein, including bisulfite and sodium metabisulfite. Suitable local anesthetics include, but are not limited to, procaine hydrochloride. Suitable suspending and dispersing agents are those as described herein, including sodium carboxymethylcelluose, hydroxypropyl methylcellulose, and polyvinylpyrrolidone. Suitable emulsifying agents include those described herein, including polyoxyethylene sorbitan monolaurate, polyoxyethylene sorbitan monooleate 80, and triethanolamine oleate. Suitable sequestering or chelating agents include, but are not limited to EDTA. Suitable pH adjusting agents include, but are not limited to, sodium hydroxide, hydrochloric acid, citric acid, and lactic acid. Suitable complexing agents include, but are not limited to, cyclodextrins, including α-cyclodextrin, β-cyclodextrin, hydroxypropyl-β-cyclodextrin, sulfobutylether-β-cyclodextrin, and sulfobutylether 7-β-cyclodextrin (CAPTISOL^{®}, CyDex, Lenexa, KS).

The pharmaceutical compositions provided herein can be formulated for single or multiple dosage administration. The single dosage formulations are packaged in an ampoule, a vial, or a syringe. The multiple dosage parenteral formulations must contain an antimicrobial agent at bacteriostatic or fungistatic concentrations. All parenteral formulations must be sterile, as known and practiced in the art.

In one embodiment, the pharmaceutical compositions are provided as ready-to-use sterile solutions. In another embodiment, the pharmaceutical compositions are provided as sterile dry soluble products, including lyophilized powders and hypodermic tablets, to be reconstituted with a vehicle prior to use. In yet another embodiment, the pharmaceutical compositions are provided as ready-to-use sterile suspensions. In yet another embodiment, the pharmaceutical compositions are provided as sterile dry insoluble products to be reconstituted with a vehicle prior to use. In still another embodiment, the pharmaceutical compositions are provided as ready-to-use sterile emulsions.

The pharmaceutical compositions provided herein can be formulated as immediate or modified release dosage forms, including delayed-, sustained, pulsed-, controlled, targeted-, and programmed-release forms.

The pharmaceutical compositions can be formulated as a suspension, solid, semi-solid, or thixotropic liquid, for administration as an implanted depot. In one embodiment, the pharmaceutical compositions provided herein are dispersed in a solid inner matrix, which is surrounded by an outer polymeric membrane that is insoluble in body fluids but allows the active ingredient in the pharmaceutical compositions diffuse through.

Suitable inner matrixes include polymethylmethacrylate, polybutylmethacrylate, plasticized or unplasticized polyvinylchloride, plasticized nylon, plasticized polyethylene terephthalate, natural rubber, polyisoprene, polyisobutylene, polybutadiene, polyethylene, ethylene-vinyl acetate copolymers, silicone rubbers, polydimethylsiloxanes, silicone carbonate copolymers, hydrophilic polymers, such as hydrogels of esters of acrylic and methacrylic acid, collagen, cross-linked polyvinyl alcohol, and cross-linked partially hydrolyzed polyvinyl acetate.

Suitable outer polymeric membranes include polyethylene, polypropylene, ethylene/propylene copolymers, ethylenelethyl acrylate copolymers, ethylene/vinyl acetate copolymers, silicone rubbers, polydimethyl siloxanes, neoprene rubber, chlorinated polyethylene, polyvinylchloride, vinyl chloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer.

### Topical Administration

The pharmaceutical compositions provided herein can be administered topically to the skin, orifices, or mucosa. The topical administration, as used herein, includes (intra)dermal, conjunctival, intracorneal, intraocular, ophthalmic, auricular, transdermal, nasal, vaginal, urethral, respiratory, and rectal administration.

The pharmaceutical compositions provided herein can be formulated in any dosage forms that are suitable for topical administration for local or systemic effect, including emulsions, solutions, suspensions, creams, gels, hydrogels, ointments, dusting powders, dressings, elixirs, lotions, suspensions, tinctures, pastes, foams, films, aerosols, irrigations, sprays, suppositories, bandages, dermal patches. The topical formulation of the pharmaceutical compositions provided herein can also comprise liposomes, micelles, microspheres, nanosystems, and mixtures thereof.

Pharmaceutically acceptable carriers and excipients suitable for use in the topical formulations provided herein include, but are not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, penetration enhancers, cryoprotectants, lyoprotectants, thickening agents, and inert gases.

The pharmaceutical compositions can also be administered topically by electroporation, iontophoresis, phonophoresis, sonophoresis, or microneedle or needle-free injection, such as POWDERJECT™ (Chiron Corp., Emeryville, CA), and BIOJECT™ (Bioject Medical Technologies Inc., Tualatin, OR).

The pharmaceutical compositions provided herein can be provided in the forms of ointments, creams, and gels. Suitable ointment vehicles include oleaginous or hydrocarbon vehicles, including lard, benzoinated lard, olive oil, cottonseed oil, and other oils, white petrolatum; emulsifiable or absorption vehicles, such as hydrophilic petrolatum, hydroxystearin sulfate, and anhydrous lanolin; water-removable vehicles, such as hydrophilic ointment; water-soluble ointment vehicles, including polyethylene glycols of varying molecular weight; emulsion vehicles, either water-in-oil (W/O) emulsions or oil-in-water (O/W) emulsions, including cetyl alcohol, glyceryl monostearate, lanolin, and stearic acid (*see, Remington: The Science and Practice of Pharmacy,* supra). These vehicles are emollient but generally require addition of antioxidants and preservatives.

Suitable cream base can be oil-in-water or water-in-oil. Cream vehicles may be water-washable, and contain an oil phase, an emulsifier, and an aqueous phase. The oil phase is also called the "internal" phase, which is generally comprised of petrolatum and a fatty alcohol such as cetyl or stearyl alcohol. The aqueous phase usually, although not necessarily, exceeds the oil phase in volume, and generally contains a humectant. The emulsifier in a cream formulation may be a nonionic, anionic, cationic, or amphoteric surfactant.

Gels arc semisolid, suspension-type systems. Single-phase gels contain organic macromolecules distributed substantially uniformly throughout the liquid carrier. Suitable gelling agents include crosslinked acrylic acid polymers, such as carbomers, carboxypolyalkylenes, CARBOPOL^{®}; hydrophilic polymers, such as polyethylene oxides, polyoxyethylene-polyoxypropylene copolymers, and polyvinylalcohol; cellulosic polymers, such as hydroxypropyl cellulose, hydroxyethyl cellulose, hydroxypropyl methyl cellulose, hydroxypropyl methylcellulose phthalate, and methylcellulose; gums, such as tragacanth and xanthan gum; sodium alginate; and gelatin. In order to prepare a uniform gel, dispersing agents such as alcohol or glycerin can be added, or the gelling agent can be dispersed by trituration, mechanical mixing, and/or stirring.

The pharmaceutical compositions provided herein can be administered rectally, urethrally, vaginally, or perivaginally in the forms of suppositories, pessaries, bougies, poultices or cataplasm, pastes, powders, dressings, creams, plasters, contraceptives, ointments, solutions, emulsions, suspensions, tampons, gels, foams, sprays, or enemas. These dosage forms can be manufactured using conventional processes as described in *Remington: The Science and Practise of Pharmacy,* supra.

Rectal, urethral, and vaginal suppositories are solid bodies for insertion into body orifices, which arc solid at ordinary temperatures but melt or soften at body temperature to release the active ingredient(s) inside the orifices. Pharmaceutically acceptable carriers utilized in rectal and vaginal suppositories include bases or vehicles, such as stiffening agents, which produce a melting point in the proximity of body temperature, when formulated with the pharmaceutical compositions provided herein; and antioxidants as described herein, including bisulfite and sodium metabisulfite. Suitable vehicles include, but are not limited to, cocoa butter (theobroma oil), glycerin-gelatin, carbowax (polyoxyethylene glycol), spermaceti, paraffin, white and yellow wax, and appropriate mixtures of mono-, di- and triglycerides of fatty acids, hydrogels, such as polyvinyl alcohol, hydroxy methyl methacrylate, polyacrylic acid; glycerinated gelatin. Combinations of the various vehicles may be used. Rectal and vaginal suppositories may be prepared by the compressed method or molding. The typical weight of a rectal and vaginal suppository is about 2 to about 3 g.

The pharmaceutical compositions provided herein can be administered ophthalmically in the forms of solutions, suspensions, ointments, emulsions, gel-forming solutions, powders for solutions, gels, ocular inserts, and implants.

The pharmaceutical compositions provided herein can be administered intranasally or by inhalation to the respiratory tract. The pharmaceutical compositions can be provided in the form of an aerosol or solution for delivery using a pressurized container, pump, spray, atomizer, such as an atomizer using electrohydrodynamics to produce a fine mist, or nebulizer, alone or in combination with a suitable propellant, such as 1,1,1,2-tetrafluorocthanc or 1,1,1,2,3,3,3-hcptafluoropropane. The pharmaceutical compositions can also be provided as a dry powder for insufflation, alone or in combination with an inert carrier such as lactose or phospholipids; and nasal drops. For intranasal use, the powder can comprise a bioadhesive agent, including chitosan or cyclodextrin.

Solutions or suspensions for use in a pressurized container, pump, spray, atomizer, or nebulizer can be formulated to contain ethanol, aqueous ethanol, or a suitable alternative agent for dispersing, solubilizing, or extending release of the active ingredient provided herein, a propellant as solvent; and/or a surfactant, such as sorbitan trioleate, oleic acid, or an aligolactic acid.

The pharmaceutical compositions provided herein can be micronized to a size suitable for delivery by inhalation, such as about 50 micrometers or less, or about 10 micrometers or less. Particles of such sizes can be prepared using a comminuting method known to those skilled in the art, such as spiral jet milling, fluid bed jet milling, supercritical fluid processing to form nanoparticles, high pressure homogenization, or spray drying.

Capsules, blisters and cartridges for use in an inhaler or insufflator can be formulated to contain a powder mix of the pharmaceutical compositions provided herein; a suitable powder base, such as lactose or starch; and a performance modifier, such as /-leucine, mannitol, or magnesium stearate. The lactose may be anhydrous or in the form of the monohydrate. Other suitable excipients or carriers include dextran, glucose, maltose, sorbitol, xylitol, fructose, sucrose, and trehalose. The pharmaceutical compositions provided herein for inhaled/intranasal administration can further comprise a suitable flavor, such as menthol and levomenthol, or sweeteners, such as saccharin or saccharin sodium.

The pharmaceutical compositions provided herein for topical administration can be formulated to be immediate release or modified release, including delayed-, sustained-, pulsed-, controlled-, targeted, and programmed release.

### Modified Release

The pharmaceutical compositions provided herein can be formulated as a modified release dosage form. As used herein, the term "modified release" refers to a dosage form in which the rate or place of release of the active ingredient(s) is different from that of an immediate dosage form when administered by the same route. Modified release dosage forms include delayed-, extended-, prolonged-, sustained-, pulsatile-, controlled-, accelerated- and fast-, targeted-, programmed-release, and gastric retention dosage forms. The pharmaceutical compositions in modified release dosage forms can be prepared using a variety of modified release devices and methods known to those skilled in the art, including, but not limited to, matrix controlled release devices, osmotic controlled release devices, multiparticulate controlled release devices, ion-exchange resins, enteric coatings, multilayered coatings, microspheres, liposomes, and combinations thereof. The release rate of the active ingredient(s) can also be modified by varying the particle sizes and polymorphorism of the active ingredient(s).

Examples of modified release include, but are not limited to, those described in U.S. Pat. Nos.: 3,845,770; 3,916,899; 3,536,809; 3,598,123; 4,008,719; 5,674,533; 5,059,595; 5,591,767; 5,120,548; 5,073,543; 5,639,476; 5,354,556; 5,639,480; 5,733,566; 5,739,108; 5,891,474; 5,922,356; 5,972,891; 5,980,945; 5,993,855; 6,045,830; 6,087,324; 6,113,943; 6,197,350; 6,248,363; 6,264,970; 6,267,981; 6,376,461; 6,419,961; 6,589,548; 6,613,358; and 6,699,500.

### 1. Matrix Controlled Release Devices

The pharmaceutical compositions provided herein in a modified release dosage form can be fabricated using a matrix controlled release device known to those skilled in the art (*see,* Takada et al in "Encyclopedia of Controlled Drug Delivery," Vol. 2, Mathiowitz Ed., Wiley, 1999).

In one embodiment, the pharmaceutical compositions provided herein in a modified release dosage form is formulated using an erodible matrix device, which is water-swellable, erodible, or soluble polymers, including synthetic polymers, and naturally occurring polymers and derivatives, such as polysaccharides and proteins.

Materials useful in forming an erodible matrix include, but are not limited to, chitin, chitosan, dextran, and pullulan; gum agar, gum arabic, gum karaya, locust bean gum, gum tragacanth, carrageenans, gum ghatti, guar gum, xanthan gum, and scleroglucan; starches, such as dextrin and maltodextrin; hydrophilic colloids, such as pectin; phosphatides, such as lecithin; alginates; propylene glycol alginate; gelatin; collagen; and cellulosics, such as ethyl cellulose (EC), methylethyl cellulose (MEC), carboxymethyl cellulose (CMC), CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, hydroxypropyl methyl cellulose (HPMC), HPMCP, HPMCAS, hydroxypropyl methyl cellulose acetate trimellitate (HPMCAT), and ethylhydroxy ethylcellulose (EHEC); polyvinyl pyrrolidone; polyvinyl alcohol; polyvinyl acetate; glycerol fatty acid esters; polyacrylamide; polyacrylic acid; copolymers of ethacrylic acid or methacrylic acid (EUDRAGIT^{®}, Rohm America, Inc., Piscataway, NJ); poly(2-hydroxyethylmethacrylate); polylactides; copolymers of L-glutamic acid and ethyl-L-glutamate; degradable lactic acid-glycolic acid copolymers; poly-D-(-)-3-hydroxybutyric acid; and other acrylic acid derivatives, such as homopolymers and copolymers of butylmethacrylate, methylmethacrylate, ethylmethacrylate, ethylacrylate, (2-dimethylaminoethyl)methacrylate, and (trimethylaminoethyl)methacrylate chloride.

In further embodiments, the pharmaceutical compositions are formulated with a non-erodible matrix device. The active ingredient(s) is dissolved or dispersed in an inert matrix and is released primarily by diffusion through the inert matrix once administered. Materials suitable for use as a non-erodible matrix device included, but are not limited to, insoluble plastics, such as polyethylene, polypropylene, polyisoprene, polyisobutylene, polybutadiene, polymethylmethacrylate, polybutylmethacrylate, chlorinated polyethylene, polyvinylchloride, methyl acrylate-methyl methacrylate copolymers, ethylene-vinyl acetate copolymers, ethylene/propylene copolymers, ethylene/ethyl acrylate copolymers, vinyl chloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, polyvinyl chloride, plasticized nylon, plasticized polyethylene terephthalate, natural rubber, silicone rubbers, polydimethylsiloxanes, silicone carbonate copolymers, and; hydrophilic polymers, such as ethyl cellulose, cellulose acetate, crospovidone, and cross-linked partially hydrolyzed polyvinyl acetate; and fatty compounds, such as carnauba wax, microcrystalline wax, and triglycerides.

In a matrix controlled release system, the desired release kinetics can be controlled, for example, via the polymer type employed, the polymer viscosity, the particle sizes of the polymer and/or the active ingredient(s), the ratio of the active ingrcdicnt(s) versus the polymer, and other excipients or carriers in the compositions.

The pharmaceutical compositions provided herein in a modified release dosage form can be prepared by methods known to those skilled in the art, including direct compression, dry or wet granulation followed by compression, melt-granulation followed by compression.

### 2. Osmotic Controlled Release Devices

The pharmaceutical compositions provided herein in a modified release dosage form can be fabricated using an osmotic controlled release device, including one-chamber system, two-chamber system, asymmetric membrane technology (AMT), and extruding core system (ECS). In general, such devices have at least two components: (a) the core which contains the active ingredient(s); and (b) a semipermeable membrane with at least one delivery port, which encapsulates the core. The semipermeable membrane controls the influx of water to the core from an aqueous environment of use so as to cause drug release by extrusion through the delivery port(s).

In addition to the active ingredient(s), the core of the osmotic device optionally includes an osmotic agent, which creates a driving force for transport of water from the environment of use into the core of the device. One class of osmotic agents water-swellable hydrophilic polymers, which are also referred to as "osmopolymers" and "hydrogels," including, but not limited to, hydrophilic vinyl and acrylic polymers, polysaccharides such as calcium alginate, polyethylene oxide (PEO), polyethylene glycol (PEG), polypropylene glycol (PPG), poly(2-hydroxyethyl methacrylate), poly(acrylic) acid, poly(methacrylic) acid, polyvinylpyrrolidone (PVP), crosslinked PVP, polyvinyl alcohol (PVA), PVA/PVP copolymers, PVA/PVP copolymers with hydrophobic monomers such as methyl methacrylate and vinyl acetate, hydrophilic polyurethanes containing large PEO blocks, sodium croscarmellose, carrageenan, hydroxyethyl cellulose (HEC), hydroxypropyl cellulose (HPC), hydroxypropyl methyl cellulose (HPMC), carboxymethyl cellulose (CMC) and carboxycthyl, cellulose (CEC), sodium alginate, polycarbophil, gelatin, xanthan gum, and sodium starch glycolate.

The other class of osmotic agents is osmogens, which are capable of imbibing water to affect an osmotic pressure gradient across the barrier of the surrounding coating. Suitable osmogens include, but are not limited to, inorganic salts, such as magnesium sulfate, magnesium chloride, calcium chloride, sodium chloride, lithium chloride, potassium sulfate, potassium phosphates, sodium carbonate, sodium sulfite, lithium sulfate, potassium chloride, and sodium sulfate; sugars, such as dextrose, fructose, glucose, inositol, lactose, maltose, mannitol, raffinose, sorbitol, sucrose, trehalose, and xylitol; organic acids, such as ascorbic acid, benzoic acid, fumaric acid, citric acid, maleic acid, sebacic acid, sorbic acid, adipic acid, edetic acid, glutamic acid, p-toluenesulfonic acid, succinic acid, and tartaric acid; urea; and mixtures thereof.

Osmotic agents of different dissolution rates can be employed to influence how rapidly the active ingredient(s) is initially delivered from the dosage form. For example, amorphous sugars, such as MANNOGEM^{™} EZ (SPI Pharma, Lewes, DE) can be used to provide faster delivery during the first couple of hours to promptly produce the desired therapeutic effect, and gradually and continually release of the remaining amount to maintain the desired level of therapeutic or prophylactic effect over an extended period of time. In this case, the active ingredient(s) is released at such a rate to replace the amount of the active ingredient metabolized and excreted.

The core can also include a wide variety of other excipients and carriers as described herein to enhance the performance of the dosage form or to promote stability or processing.

Materials useful in forming the semipermeable membrane include various grades of acrylics, vinyls, ethers, polyamides, polyesters, and cellulosic derivatives that arc water-permeable and water-insoluble at physiologically relevant pHs, or are susceptible to being rendered water-insoluble by chemical alteration, such as crosslinking. Examples of suitable polymers useful in forming the coating, include plasticized, unplasticized, and reinforced cellulose acetate (CA), cellulose diacetate, cellulose triacetate, CA propionate, cellulose nitrate, cellulose acetate butyrate (CAB), CA ethyl carbamate, CAP, CA methyl carbamate, CA succinate, cellulose acetate trimellitate (CAT), CA dimcthylaminoacctatc, CA ethyl carbonate, CA chloroacetate, CA ethyl oxalate, CA methyl sulfonate, CA butyl sulfonate, CA p-toluene sulfonate, agar acetate, amylose triacetate, beta glucan acetate, beta glucan triacetate, acetaldehyde dimethyl acetate, triacetate of locust bean gum, hydroxylated ethylenevinylacetate, EC, PEG, PPG, PEG/PPG copolymers, PVP, HEC, HPC, CMC, CMEC, HPMC, HPMCP, HPMCAS, HPMCAT, poly(acrylic) acids and esters and poly-(methacrylic) acids and esters and copolymers thereof, starch, dextran, dextrin, chitosan, collagen, gelatin, polyalkenes, polyethers, polysulfones, polyethersulfones, polystyrenes, polyvinyl halides, polyvinyl esters and ethers, natural waxes, and synthetic waxes.

Semipermeable membrane can also be a hydrophobic microporous membrane, wherein the pores are substantially filled with a gas and are not wetted by the aqueous medium but are permeable to water vapor, as disclosed in U.S. Pat. No. 5,798,119. Such hydrophobic but water-vapor permeable membrane are typically composed of hydrophobic polymers such as polyalkenes, polyethylene, polypropylene, polytetrafluoroethylene, polyacrylic acid derivatives, polyethers, polysulfones, polyethersulfones, polystyrenes, polyvinyl halides, polyvinylidene fluoride, polyvinyl esters and ethers, natural waxes, and synthetic waxes.

The delivery port(s) on the semipermeable membrane can be formed post-coating by mechanical or laser drilling. Delivery port(s) can also be formed in situ by erosion of a plug of water-soluble material or by rupture of a thinner portion of the membrane over an indentation in the core. In addition, delivery ports can be formed during coating process, as in the case of asymmetric membrane coatings of the type disclosed in U.S. Pat. Nos. 5,612,059 and 5,698,220.

The total amount of the active ingredient(s) released and the release rate can substantially by modulated via the thickness and porosity of the semipermeable membrane, the composition of the core, and the number, size, and position of the delivery ports.

The pharmaceutical compositions in an osmotic controlled-release dosage form can further comprise additional conventional excipients or carriers as described herein to promote performance or processing of the formulation.

The osmotic controlled-release dosage forms can be prepared according to conventional methods and techniques known to those skilled in the art (*see, Remington: The Science and Practice of Pharmacy,* supra; Santus and Baker, J. Controller Release 1995, 35, 1-21; Verma et al., Drug Development and Industrial Pharmacy 2000, 26, 695-708; Verma et al., J. Controlled Release 2002, 79, 7-27).

In certain embodiments, the pharmaceutical compositions provided herein are formulated as AMT controlled-release dosage form, which comprises an asymmetric osmotic membrane that coats a core comprising the active ingredient(s) and other pharmaceutically acceptable excipients or carriers. *See,* U.S. Pat. No. 5,612,059 and WO 2002/17918. The AMT controlled-release dosage forms can be prepared according to conventional methods and techniques known to those skilled in the art, including direct compression, dry granulation, wet granulation, and a dip-coating method.

In certain embodiments, the pharmaceutical compositions provided herein are formulated as ESC controlled-release dosage form, which comprises an osmotic membrane that coats a core comprising the active ingredient(s), a hydroxylethyl cellulose, and other pharmaceutically acceptable excipients or carriers.

### 3. Multiparticulate Controlled Release Devices

The pharmaceutical compositions provided herein in a modified release dosage form can be fabricated as a multiparticulate controlled release device, which comprises a multiplicity of particles, granules, or pellets, ranging from about 10 µm to about 3 mm, about 50 µm to about 2.5 mm, or from about 100 µm to about 1 mm in diameter. Such multiparticulates can be made by the processes known to those skilled in the art, including wet-and dry-granulation, extrusion/spheronization, roller-compaction, melt-congealing, and by spray-coating seed cores. *See,* for example, Multiparticulate Oral Drug Delivery; Marcel Dekker: 1994; and Pharmaceutical Pelletization Technology; Marcel Dekker: 1989.

Other excipients or carriers as described herein can be blended with the pharmaceutical compositions to aid in processing and forming the multiparticulates. The resulting particles can themselves constitute the multiparticulate device or can be coated by various film-forming materials, such as enteric polymers, water-swellable, and water-soluble polymers. The multiparticulates can be further processed as a capsule or a tablet.

### 4. Targeted Delivery

The pharmaceutical compositions provided herein can also be formulated to be targeted to a particular tissue, receptor, or other area of the body of the subject to be treated, including liposome-, resealed erythrocyte-, and antibody-based delivery systems. Examples include, but are not limited to, U.S. Pat. Nos. 6,316,652; 6,274,552; 6,271,359; 6,253,872; 6,139,865; 6,131,570; 6,120,751; 6,071,495; 6,060,082; 6,048,736; 6,039,975; 6,004,534; 5,985,307; 5,972,366; 5,900,252; 5,840,674; 5,759,542; and 5,709,874.

### D. EVALUATION OF THE ACTIVITY OF THE COMPOUNDS

Standard physiological, pharmacological and biochemical procedures are available for testing the compounds to identify those that possess biological activities that modulate the activity of JAK kinases, including wild type and mutant JAK kinases. Such assays include, for example, biochemical assays such as binding assays, see, Fabian et al., Nature Biotechnology 2005, 23,329-336, radioactivity incorporation assays, as well as a variety of cell based assays.

Exemplary cell based assay methodologies include measurement of STAT5A phosphorylation, for example, by ELISA or the measurement of proliferation in leukemic cell lines such as TF-1 or HEL-2, for example, by BrdU incorporation, by fluorescent staining or by a reporter assay activated by the transcription factor STAT5. Cells useful in the assays include cells with wildtype JAK such as TF-1 or mutated JAK such as the cell line HEL-2 which express a constitutively active JAK2 carrying the V617F mutation. Suitable cells include those derived through cell culture from patient samples as well as cells derived using routine molecular biology techniques, e.g., retroviral transduction, transfcction, mutagenesis, etc.

### E. THE COMPOUNDS AND COMPOSITIONS FOR USE IN METHODS

Also provided herein are the disclosed compounds and compositions, or pharmaceutically acceptable salts, solvates or hydrates thereof for use in methods for the treatment, prevention, or amelioration of a disease or disorder that is mediated or otherwise affected via JAK kinase, including JAK2 kinase activiy or one or more symptoms of diseases or disorders that are mediated or otherwise affected via JAK kinase, including JAK2 kinase, activity. JAK kinase can be wild type and/or mutant form of JAK2 kinase. Consistent with the description above, such diseases or disorders include without limitation: myeloproliferative disorders such as polycythemia vera (PCV), essential thrombocythemia and idiopathic myelofibrosis (IMF); leukemia such as myeloid leukemia including chronic myeloid leukemia (CML), imatinib-resistant forms of CML, acute myeloid leukemia (AML), and a subtype of AML, acute megakaryoblastic leukemia (AMKL); lymphoproliferative diseases such as myeloma; cancer including head and neck cancer, prostate cancer, breast cancer, ovarian cancer, melanoma, lung cancer, brain tumor, pancreatic cancer and renal carcinoma; and inflammatory diseases or disorders related to immune dysfunction, immunodeficiency, immunomodulation, autoimmune diseases, tissue transplant rejection, graft-versus-host disease, wound healing, kidney disease, diabetic neuropathy, multiple sclerosis, thyroiditis, type 1 diabetes, sarcoidosis, psoriasis, allergic rhinitis, inflammatory bowel disease including Crohn's disease and ulcerative colitis (UC), systemic lupus erythematosis (SLE), arthritis, osteoarthritis, rheumatoid arthritis, osteoporosis, asthma and chronic obstructive pulmonary disease (COPD) and dry eye syndrome (or keratoconjunctivitis sicca (KCS)),

In certain embodiments, provided herein are the disclosed compounds and compositions, or pharmaceutically acceptable salts, solvates or hydrates thereof for use in methods for the treatment, prevention, or amelioration of a disease or disorder selected from myeloproliferative disorders such as polycythemia vera (PCV), essential thrombocythemia and idiopathic myelofibrosis (IMF) and hypercosinophilic syndrome (HES); leukemia such as myeloid leukemia including chronic myeloid leukemia (CML), imatinib-resistant forms of CML, acute myeloid leukemia (AML), acute lymphoblastic leukemia (ALL) and a subtype of AML, acute megakaryoblastic leukemia (AMKL); lymphoproliferative diseases such as myeloma; cancer including head and neck cancer, prostate cancer, breast cancer, ovarian cancer, melanoma, lung cancer, brain cancer, pancreatic cancer, gastric cancer, thyroid cancer, renal carcinoma, Kaposi's sarcoma, Castleman's disease, melanoma; and inflammatory diseases or disorders related to immune dysfunction, immunodeficiency or immunomodulation, such as tissue transplant rejection, graft-versus-host disease, wound healing, kidney disease including diabetic neuropathy; autoimmune diseases such as multiple sclerosis, thyroiditis, type I diabetes, sarcoidosis, psoriasis, allergic rhinitis, atopic dermatitis, myasthenia gravis, inflammatory bowel disease including Crohn's disease and ulcerative colitis (UC), systemic lupus erythematosis (SLE), arthritis, osteoarthritis, rheumatoid arthritis, osteoporosis, asthma and chronic obstructive pulmonary disease (COPD), inflammatory diseases of the eye including conjunctivitis, uveitis, iritis, scleritis, inflammatory diseases of the respiratory tract including the upper respiratory tract such as rhinitis and sinusitis and inflammatory diseases of the lower repiratory tract including bronchitis; inflammatory myopathy such as myocarditis, other inflammatory diseases such as ischemia reperfusion injuries related to an inflammatory ischemic event such as a stroke or cardiac arrest, and other inflammatory conditions such as systemic inflammatory response syndrome (SIRS) and sepsis.

In certain embodiments, JAK-mediated diseases and disorders include restenosis, fibrosis and scleroderma. In certain embodiments, JAK-mediated diseases include viral diseases such as Epstein Barr virus (EBV), hepatitis (hepatitis B or hepatitis C), human immunodeficiency virus (HIV), Human T-lymphotropic virus type 1 (HTLV-1), varicella-zoster virus and the human papilloma virus (HPV).

### F. COMBINATION THERAPY

Furthermore, it will be understood by those skilled in the art that the compounds, isomers, and pharmaceutically acceptable salts, solvates or hydrates provided herein, including pharmaceutical compositions and formulations containing these compounds, can be used in a wide variety of combination therapies to treat the conditions and diseases described above. Thus, also contemplated herein is the use of compounds, isomers and pharmaceutically acceptable salts, solvates or hydrates provided herein in combination with other active pharmaceutical agents for the treatment of the disease/conditions described herein.

In one embodiment, such additional pharmaceutical agents include without limitation anti-cancer agents, including chemotherapeutic agents and antiproliferative agents; anti-inflammatory agents and immunomodulatory agents or immunosuppressive agents.

In certain embodiments, the anti-cancer agents include anti-metabolites (*e.g.,* 5-fluoro-uracil, cytarabine, methotrexate, fludarabine and others), antimicrotubule agents (*e.g.,* vinca alkaloids such as vincristine, vinblastine; taxanes such as paclitaxel and docetaxel), alkylating agents (*e.g.,* cyclophosphamide, melphalan, carmustine, nitrosourcas such as bischlorocthylnitrosurca and hydroxyurea), platinum agents (*e.g.* cisplatin, carboplatin, oxaliplatin, satraplatin and CI-973), anthracyclines (*e.g.,* doxrubicin and daunorubicin), antitumor antibiotics (*e.g.,* mitomycin, idarubicin, adriamycin and daunomycin), topoisomerase inhibitors (*e.g.,* etoposide and camptothecins), anti-angiogenesis agents (*e.g.* Sutent®, sorafenib and Bevacizumab) or any other cytotoxic agents, (*e.g.* estramustine phosphate, prednimustine), hormones or hormone agonists, antagonists, partial agonists or partial antagonists, kinase inhibitors (such as imatinib), and radiation treatment.

In certain embodiments, the anti-inflammatory agents include methotrexate, matrix melalloproteinase inhibitors, inhibitors of pro-inflammatory cytokines (*e.g.,* anti-TNF molecules, TNF soluble receptors, and IL1) non-steroidal anti-inflammatory drugs (NSAIDs) such as prostaglandin synthase inhibitors (*e.g.,* choline magnesium salicylate and salicylsalicyclic acid), COX-1 or COX-2 inhibitors, or glucocorticoid receptor agonists such as corticosteroids, methylprednisone, prednisone, or cortisone.

The compound or composition provided herein, or pharmaceutically acceptable salts, solvates or hydrates thereof, may be administered simultaneously with, prior to, or after administration of one or more of the above agents.

Pharmaceutical compositions containing a compound provided herein or pharmaceutically acceptable salts, solvates or hydrates thereof, and one or more of the above agents are also provided.

Also provided are the compounds or compositions disclosed herein, or pharmaceutically acceptable salts, solvates or hydrates thereof, with one or more anti-cancer agents for use in a combination therapy that treats or prevents the onset of the symptoms, or associated complications of cancer and related diseases and disorders.

### G. PREPARATION OF COMPOUNDS

Starting materials in the synthesis examples provided herein are either available from commercial sources or via literature procedures (*e.g.,* March Advanced Organic Chemistry: Reactions, Mechanisms, and Structure, (1992) 4th Ed.; Wiley Interscience, New York). All commercially available compounds were used without further purification unless otherwise indicated. Proton (¹H) nuclear magnetic resonance (NMR) spectra were typically recorded at 300 MHz on a Bruker Avance 300 NMR spectrometer unless otherwise noted. Significant peaks arc tabulated and typically include: number of protons, and multiplicity (s, singlet; d, double; t, triplet; q, quartet; m, multiplet; br s, broad singlet). Chemical shifts are reported as parts per million (δ) relative to tetramethylsilane. Unless otherwise noted, low resolution mass spectra (MS) were obtained as electrospray ionization [ESI) mass spectra, which were typically recorded on a Shimadzu HPLC/MS instrument using reverse-phase conditions using a mobile phase gradients of either acetonitrile/water containing 0.05% acetic acid or MeOH/water containing 0.2% formic acid. Preparative reverse phase HPLC was typically performed using a Varian HPLC system equipped with a Phenomenex phenylhexyl, a Phenomenex Luna C18, or a Varian Pursuit diphenyl reverse phase column; typical elution conditions utilized a gradient of acetonitrile/water containing 0.05% acetic acid. Silica gel chromatography was either performed manually, typically following the published procedure for flash chromatography (Still et al. (1978) J. Org. Chem. 43:2923), or on an automated system (for example, on a Biotage SP instrument) using pre-packed silica gel columns.

It is understood that in the following description, combinations of substituents and/or variables of the depicted formulae are permissible only if such contributions result in stable compounds under standard conditions.

It will also be appreciated by those skilled in the art that in the process described below the functional groups of intermediate compounds may need to be protected by suitable protecting groups. Such functional groups include hydroxy, amino, mercapto and carboxylic acid. Suitable protecting groups for hydroxy include trialkylsilyl or diarylalkylsilyl (e.g., *t*-butyldimethylsilyl, *t*-butyldiphenylsilyl or trimcthylsilyl), tetrahydropyranyl, benzyl, and the like. Suitable protecting groups for amino, amidino and guanidino include t-butoxycarbonyl, benzyloxycarbonyl, and the like. Suitable protecting groups for mercapto include -C(O)-R (where R is alkyl, aryl or aralkyl), *p*-methoxybenzyl, trityl and the like. Suitable protecting groups for carboxylic acid include alkyl, aryl or aralkyl esters.

Protecting groups may be added or removed in accordance with standard techniques, which are well-known to those skilled in the art and as described herein. The use of protecting groups is described in detail in Green, T.W. and P.G.M. Wutz, Protective Groups in Organic Synthesis (1991), 2nd Ed., Wiley-Interscience.

One of ordinary skill in the art could readily ascertain which choices for each substitucnt are possible for the reaction conditions of each Scheme. Moreover, the substituents are selected from components as indicated in the specification heretofore, and may be attached to starting materials, intermediates, and/or final products according to schemes known to those of ordinary skill in the art.

Also it will be apparent that the compounds provided herein could exist as one or more isomers, that is E/Z isomers, enantiomers and/or diastereomers.

Compounds of formula (I) may be generally prepared as depicted in the following schemes, and unless otherwise noted, the various substituents are as defined elsewhere herein.

Standard abbreviations and acronyms as defined in J. Org. Chem. 2007 72(1): 23A-24A are used herein. Other abbreviations and acronyms used herein are as follows:

| | |
|---|---|
| DCM | dichloromethane |
| DIEA | diisopropylethylamine |
| EDCI | N-(3-Dimethylaminopropyl)-N'-ethylcarbodiimide hydrochloride |
| EtOAc | ethyl acetate |
| EtOH | ethanol |
| FBS | fetal bovine serum |
| HATU | O-(7-Azabenzotriazol-1-yl)-N,N,N',N'-tetramethyluronium hexafluorophosphate |
| HOAc | acetic acid |
| HOBt | N-hydroxybenzotriazole |
| MeOH | methanol |
| TEA | Triethylamine |
| Trityl | Triphenylmethyl |

Compounds provided herein are synthesized according to the following schemes and descriptions.

In Scheme **1** is illustrated synthetic methodology suitable for preparation of isoquinolines **6**. As described in the literature (see Hurtley, J. Chem. Soc. 1929, 1872) treatment of an appropriate ortho-halobenzoic acid **1** with dialkyl malonate and subsequent processing under conditions described affords diacid **2**. Condensation of **2** with ammonia with heating or other dehydrating conditions produces dihydroxyisoquinoline **3** (see Bailey, J. Chem. Soc. 1956, 2477; US2351391). The hydroxyl groups of **3** are converted to leaving groups X by treating with an appropriate phosphorous or phosphoryl halide reagent, for example phosphoryl chloride, to form 2,4-dihalo derivatives **4** (X = halo). Alternatively, X can be a different leaving group moiety, for example sulfonate, via treatment of **3** with an appropriate sulfonyl halide in the presence of base such as a tertiary amine. Treatment of **4** with an aminoazole with heating as required in the presence of base or in the presence of a suitable Pd catalyst with added Pd ligands as required affords **5**. Treatment of **5** with a suitable thiolate reagent with heating as required forms an intermediate sulfide, which is oxidized by treatment at 0 °C to rt with a stoichiometric or slight excess quantity of an oxidant such as a percarboxylic acid to give sulfoxide **6** (n = 1). Sulfone **6** (n = 2) is formed either from further oxidation of the sulfoxide using additional equivalents of oxidant at rt to elevated temperature as required, or can be formed directly from the sulfide by treatment with two to four equivalents of oxidant at rt to elevated temperature as required to drive the reaction to substantial completion. In some cases it may be advantageous to displace one of the X groups of **4** with a group "Prot" followed by reaction with a thiolate reagent to form **7**. "Prot" is intended to be a group, for example alkoxy, which can be subsequently conveniently reverted to a leaving group X, for example, to afford **8**. Conversion of **8** to **9** is effected under conditions analogous to, or if needed, more forcing than, those that used to effect conversion of **4** to **5**. Conversion of **9** to **6** is carried out under conditions analogous to those described above for the conversion of **5** to **6**. Alternatively, conversion of **8** to **6** may be achieved via treatment first with a stoichiometric or slight excess quantity of an oxidant such as a percarboxylic acid to give a sulfoxide intermediate (n = 1), or further oxidation of the sulfoxide using additional equivalents of oxidant at rt to elevated temperature as required to afford the sulfone intermediate (n = 2). Subsequent treatment of such sulfoxide or sulfone intermediates with an aminoazole with heating as required in the presence of base or in the presence of a suitable Pd catalyst with added Pd ligands as required affords **6**.

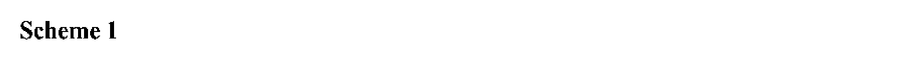

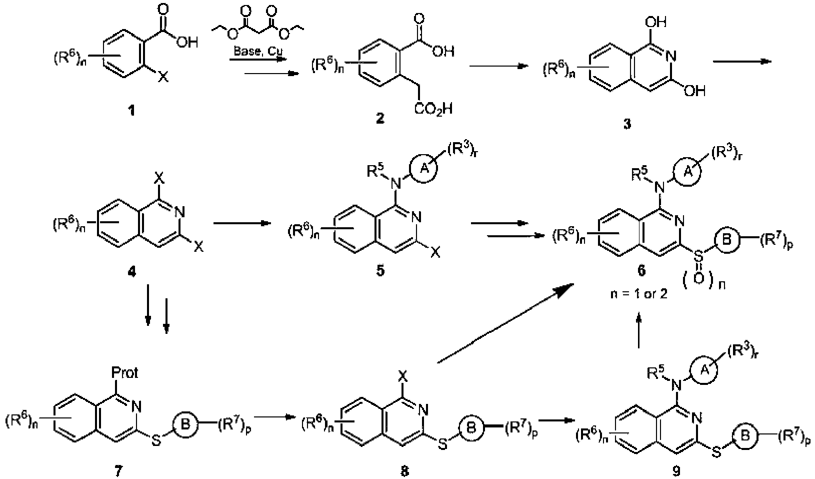

In Scheme 2 is illustrated synthetic methodology suitable for preparation of isoquinolines **15**. A suitable ortho-halobenzoate ester **10** is treated with 2-acetamidoacrylate ester in the presence of a Pd catalyst, for example palladium acetate, with heating in a suitable solvent such as DMF to form intermediate **11**, which upon further heating is transformed to **12**. The hydroxyl group of **12** is converted to a leaving group X by treating with an appropriate phosphorous or phosphoryl halide reagent, for example phosphoryl chloride, to form **13** (X = halo). Alternatively, X can be a different leaving group moiety, for example sulfonate, via treatment of **12** with an appropriate sulfonyl halide in the presence of base such as a tertiary amine. Treatment of **13** with a metalloarene or metalloheteroarene, for example an aryl or heteroaryl lithium or an aryl or heteroaryl Grignard reagent in a suitable solvent such diethyl ether, THF, or other ether solvent, produces ketone **14**. Treatment of **14** with an aminoazole with heating as required in the presence of acid or base or in the presence of a suitable Pd catalyst with added Pd ligands as required affords **15**.

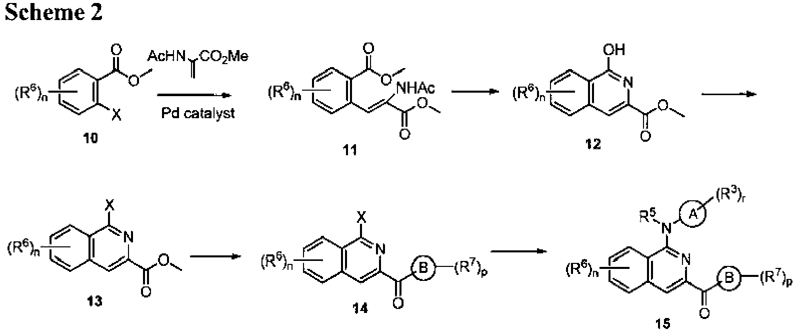

In Scheme 3 is illustrated synthetic methodology suitable for preparation of isoquinoliness **21**. In a manner analogous to procedures described in WO2003/106421, a suitable ortho-methybenzonitrile **16** is deprotonated with strong base and then treated with a suitably activated carboxylic acid **17**, wherein Y may be alkoxy or -N(Me)OMe, to form ketone **18**. Treatment of **18** under acidic conditions effects ring closure to the hydroxyisoquinoline derivative **19**. The hydroxyl group of **19** is converted to a leaving group X to form **20** in a manner analogous to that described in Scheme 2 for conversion of **12** to **13**. Treatment of **20** with an aminoazole with heating as required in the presence of acid or base or in the presence of a suitable Pd catalyst with added Pd ligands as required affords **21**.

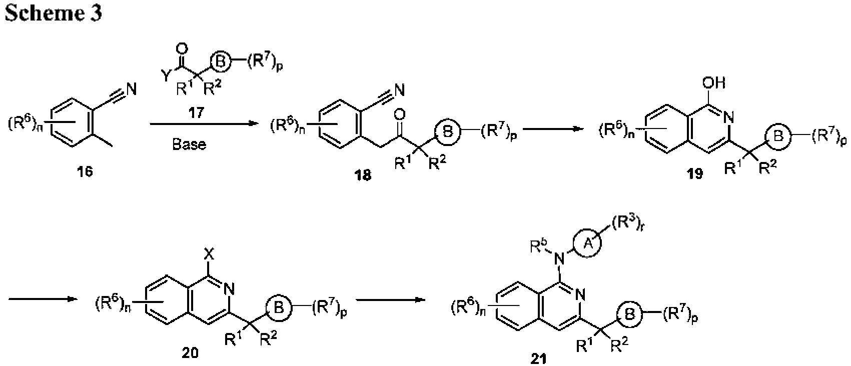

In Scheme 4 is illustrated synthetic methodology suitable for preparation of quinolines **27**. As described by Lutz, et al. (J. Am. Chem. Soc. 1946, 68, 1285), treatment of a suitable anthranilic ester **22** with a dialkyl malonate such as **23** affords amide **24**. Treatment of **24** with a base such as an alkoxide with heating effects ring closure, which is followed by ester hydrolysis and decarboxylation to afford **25**. Conversion of **25** to **27** via **26** is effected using methodology analogous to that described in Scheme 1 for conversion of **3** to **6**.

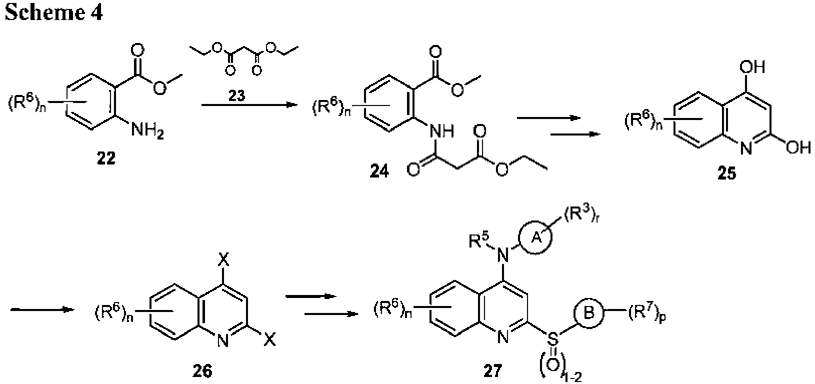

In Scheme 5 is illustrated synthetic methodology suitable for preparation of quinolines **32**. As described in US5026700, treatment of a suitable aniline **28** with dialkyl acetylenedicarboxylate in refluxing alcohol solvent affords hydroxyquinoline **29**. Alternatively, dialkyl 2-oxosuccinate may be substituted for dialkyl acetylenedicarboxylate following a procedure reported by Riegel, et al. J. Am. Chem. Soc. 1946, 68, 2685. Conversion of **29** to **32** via **30** and **31** may be effected using methodology analogous to that described in Scheme 2 for converting **12** to **15**.

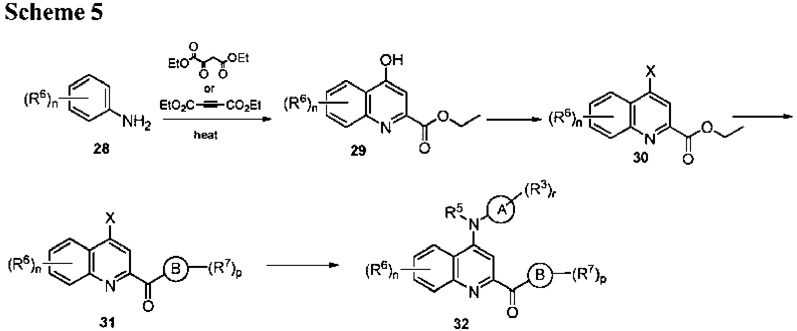

In Scheme 6 is illustrated synthetic methodology suitable for preparation of quinolines **36**. A suitable aniline **28** is acetylated under Friedel-Crafts conditions and then the amino group is acylated with a suitably activated carboxylic acid derivative **17** to afford amide **33**. Ring closure to **34** is effected by treatment with a base such as hydroxide or alkoxide with heating as required. Conversion of **34** to **36** via **35** is effected using methodology analogous to that described in Scheme 3 for conversion of **19** to **21**.

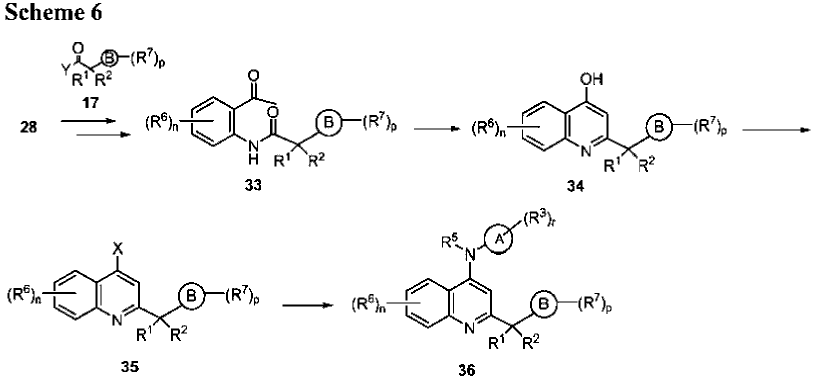

In Scheme **7** are illustrated representative examples by which the keto group in any of **15** (Scheme 2) or **32** (Scheme 5) can be further modified to afford additional compounds of the invention. Treatment of ketone with Lawcsson's reagent affords thioketones **37**. Treatment of kelone with an amine, hydroxylamine, or alkoxylamine under dehydrating conditions optionally in the presence of acid with heating affords, respectively, imines, oximes, or O-alkyl oximes **38**. Treatment of ketone with a Wittig reagent or Horner-Emmons reagent affords olefins **39**. Treatment of ketone with a reducing agent such as sodium borohydride or lithium borohydride affords secondary alcohols **40**. Treatment of ketone with an organometallic reagent such as a Grignard reagent or an organolithium compound affords tertiary alcohols **41**. Heating ketone with an alcohol in the presence of acid with removal of water affords ketals **42**. Heating ketone with a 1,2- 1,3- or 1, 4 diol in the presence of acid with removal of water affords cyclic ketals **43**.

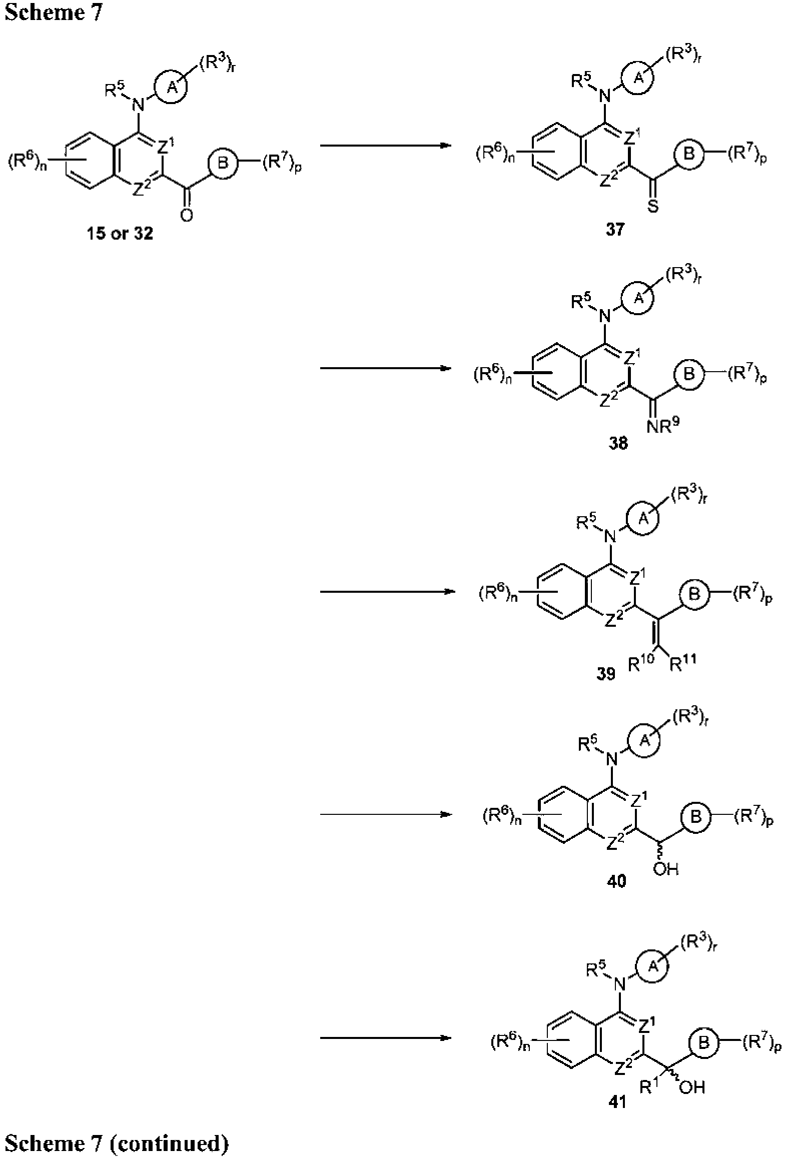

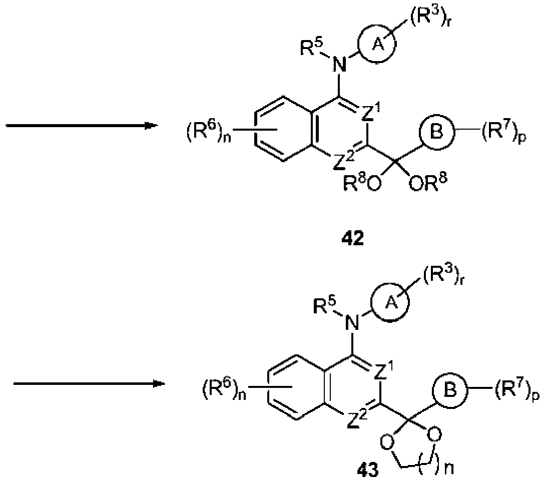

In Scheme 8 is illustrated a useful method for preparing acids **17** used in Schemes 3 and 6. A carboxylic acid derivative **44**, where Y' is for example alkoxy or a subsequently removable chiral auxiliary, is deprotonated at the alpha position with a strong base and treated with an alkylating agent to afford **45**. The sequence is repeated with the same or a different alkylating agent to form **46**. The Y' group of **46** is then converted by procedures well known in the art to the Y group of 17 that is suitable for use in Scheme 3 or 6.

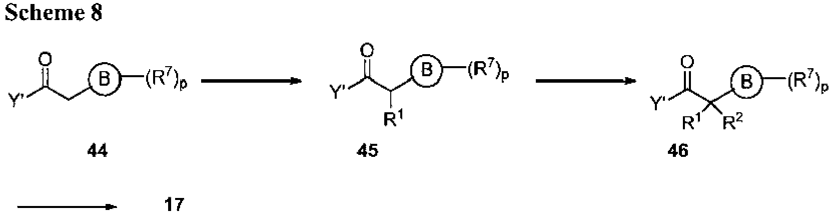

In Scheme 9 is illustrated an alternative method for preparing acids **17** used in Schemes 3 and 6. A suitable carboxylic acid derivative, following conversion with base to an enolate **47** or its equivalent is treated with an aryl halide, or more suitably with a heteroaryl halide to form **49**. The Y' group of **49** is then converted by procedures well known in the art to the Y group of **17** that is suitable for use in Scheme 3 or 6.

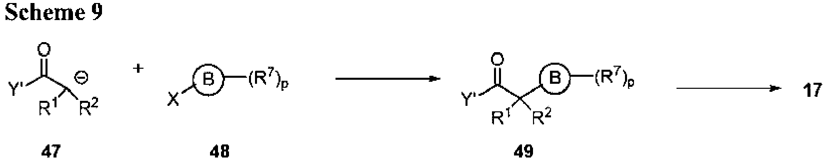

It will be appreciated by one skilled in the art that standard functional group manipulations may be used to prepare additional compounds of the invention from products or intermediates prepared as described by the foregoing methods. In Scheme 10 are shown representative examples that are intended to illustrate, but in no way to limit the scope of, such standard functional group manipulations.

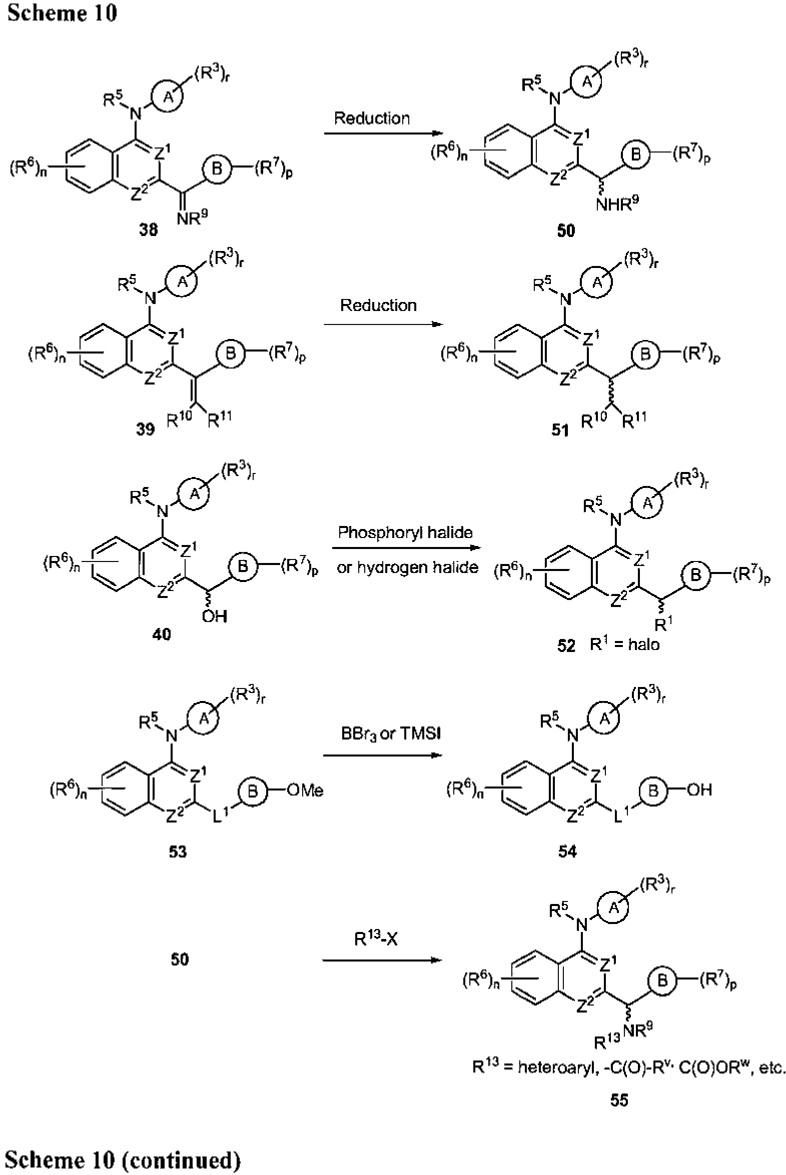

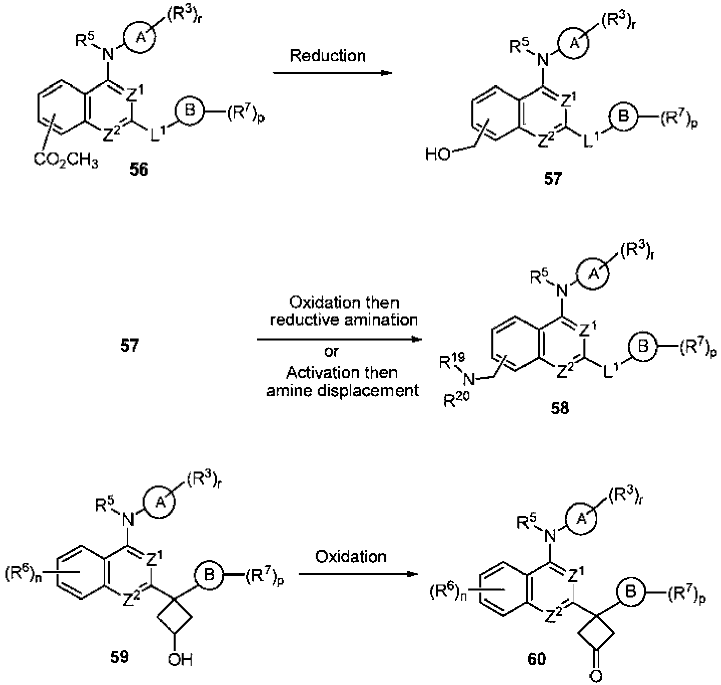

Aminoazole or azolyl amine intermediates employed herein may be obtained either via commercial sources or prepared using methods known to those skilled in the art. Scheme **11** illustrates representative methods that may be employed for the preparation of additional aminoazoles or azolyl amines. For example, nitroazoles **61** may be converted to aminoazoles **62** via treatment with a suitable reducing agent such as SnCl₂ in a suitable solvent such as DCE or EtOH optionally in the presence of HCl, with heating. Alternatively, treatment of **61** with activated iron or zinc metal in HOAc with heating will afford **62**. Alternatively, treatment of **61** with palladium metal on activated carbon in the presence of ≥ 1 atmosphere pressure of hydrogen gas, in a suitable solvent such as MeOH, EtOH, or EtOAc or mixtures of these, at rt or with heating as required, will afford **62**. Alternatively treatment of **61** with sodium hydrosulfite in a suitable solvent mixture such as THF and water at rt or with heating as required, will afford **62**. Alternatively, aminoazoles **62** may also be obtained from azole carboxylic acids **63** via initial treatment with diphenylphosphoryl azide in the presence of an organic base such as TEA, and in a suitable solvent such as toluene or THF, and with heating from 50 °C to 150 °C as required, followed by hydrolysis. Alternatively, treatment of **63** with diphenylphosphoryl azide in the presence of an organic base such as TEA, and in the presence of excess *tert*-butanol, and in a suitable solvent such as toluene or THF, and with heating from 50 °C to **150** °C as required, will afford a *tert*-butylcarbamoyl acole intermediate, which upon treatment with an acid such as TFA or HCl in a suitable solvent, will afford **62**. Aminoazoles **62** may also be obtained from azolyl bromides or iodides **64**, bearing (as required) suitable protecting groups on any azole ring N-H position, via initial treatment with a suitable amino containing reagent (where P = protecting group), such as benzophenone imine, 2,4-dimethoxybenzylamine, or *tert*-butyl carbamate, and in the presence of a catalytic amount of a suitable organopalladium-complex, and optionally in the presence of a suitable phosphine-ligand, and optionally in the presence of a suitable base, and in a suitable solvent at elevated temperature or under microwave conditions, to afford intermediate **65**. Subsequent N-deprotection of intermediate **65** (including azole ring N-deprotection, where required), employing appropriate methods known to those skilled in the art will afford **62**. Conversion of aminoazoles **62** to alkylated aminoazoles **66** may be achieved via treatment of **62** with an appropriate aldehyde or ketone substrate, in the presence of a suitable Lewis acid such as TMSCI or TiCl₄ and a suitable reducing agent such as sodium (triacetoxy)borohydride or sodium cyanoborohydride, in a suitable organic solvent such as DCM, DCE, THF, or MeOH, optionally in the presence of HOAc, at rt or with heating as required. Alternatively, **66** may be obtained via treatment of **62** with an alkyl halide in the presence of a suitable organic base such as pyridine or DIEA, and sodium or potassium iodide, and in a suitable solvent such as DMF or THF, at rt or with heating as required. Nitroazoles **61**, azole carboxylic acids **63**, and azole bromides or iodides **64** may be obtained from commercial sources or prepared using methods known to those skilled in the art.

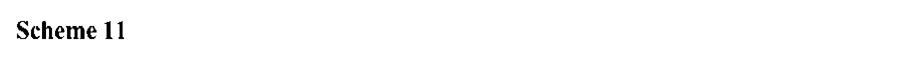

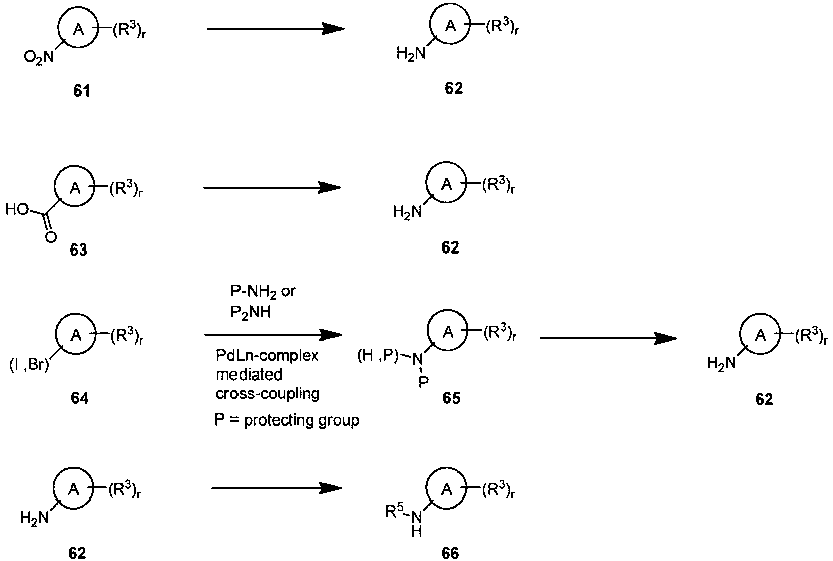

The subject matter has been described in an illustrative manner, and it is to be understood that the terminology used is intended to be in the nature of description rather than of limitation. Thus, it will be appreciated by those of skill in the art that conditions such as choice of solvent, temperature of reaction, volumes, reaction time may vary while still producing the desired compounds. In addition, one of skill in the art will also appreciate that many of the reagents provided in the following examples may be substituted with other suitable reagents. *See, e.g*., Smith & March, Advanced Organic Chemistry, 5th ed. (2001).

### EXAMPLES

The embodiments described above arc intended to be merely exemplary, and those skilled in the art will recognize, or will be able to ascertain using no more than routine experimentation, numerous equivalents of specific compounds, materials, and procedures. All such equivalents are considered to be within the scope of the claimed subject matter and are encompassed by the appended claims.

### Example 1

### Preparation of (4-fluorophenyl)(1-(5-methyl-1H-pyrazol-3-ylamino)isoquinolin-3-yl)methanone

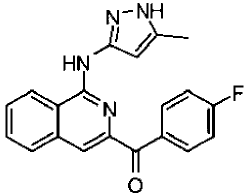

**Step A:** To a stirred mixture of methyl 2-iodobenzoate (1.46 g, 5.57 mmol), palladium (0) acetate (25 mg, 0.11 mmol), tetrabutylammonium chloride (1.54 g, 5.57 mmol) and sodium hydrogen carbonate (1.17 g, 13.92 mmol) in N, N-dimethylformamide (11 mL) was added methyl 2-acetamidoacrylate (1.19g, 8.35 mmol), and the mixture was heated at 55 °C for 15 h. After cooling to rt, the mixture was partitioned between water and EtOAc, and then the separated aqueous layer was extracted three times with EtOAc. The combined organic layers were dried over Na₂SO₄, filtered, and concentrated under reduced pressure. The residue was recrystallized from EtOAc to afford methyl 1-oxo-1,2-dihydroisoquinoline-3-carboxylate as a colorless solid (820 mg, 72%). ¹H NMR (400 MHz, DMSO-d₆) δ 11.21 (s, 1H), 8.26 (d, *J* = 8.0 Hz, I H), 7.92 (d, *J* = 7.6 Hz, 1H), 7.79-7.83 (m, 1H), 7.65-7.69 (m, 1H), 7.46 (s, 1H), 3.90 (s, 3H); LC-MS (ESI) *m*/*z* 204 (M+H)⁺.

**Step B:** A mixture of methyl 1-oxo-1,2-dihydroisoquinoline-3-carboxylate (2.03 g, 10 mmol) and 1N HCl/EtOH (20 mL) was stirred at 100 °C for 3 h. The mixture was cooled to rt, concentrated under reduced pressure, and adjusted to pH 7 with aq sodium hydrogen carbonate. The mixture was then extracted with EtOAc and the combined organic layers were dried over Na₂SO₄, filtered, and concentrated under reduced pressure to afford ethyl 1-oxo-1,2-dihydroisoquinoline-3-carboxylate as a tan solid (1.8 g, 83%). ¹H NMR (400 MHz, DMSO-d₆) δ 11.19 (s, 1H), 8.26 (d, *J* = 8.0 Hz, 1H), 7.92 (d, *J* = 8.0 Hz, 1H), 7.79-7.73 (m, 1H), 7.66-7.68 (m, 1 H), 7.44 (s, 1H), 4.36 (q, *J* = 7.2 Hz, 2H), 1.36 (t, *J* = 7.2 Hz, 3H); LC-MS (ESI) *m*/*z* 218 (M+H)⁺.

**Step C:** A stirred mixture of ethyl 1-oxo-1,2-dihydroisoquinoline-3-carboxylate (1.8 g, 0.23 mmol) in phosphorus oxychloride (15 mL) was heated under reflux for 2 h. After cooling to rt, the mixture was poured into ice water and adjusted to pH 7 with aq sodium hydrogen carbonate. The mixture was extracted with EtOAc and the combined organic layers were dried over Na₂SO₄, filtered, and concentrated under reduced pressure to afford ethyl 1-chloroisoquinoline-3-carboxylate as a tan solid (1.3 g, 66%). ¹H NMR (400 MHz, CDCl₃) δ 8.54 (s, 1H), 8.45 (d, *J*= 7.6 Hz, 1H), 8.03 (d, *J*= 7.2 Hz, 1H), 1.85-7.87 (m, 2H), 4.55 (q, *J* = 7.2 Hz, 2H), 1.50 (t, *J* = 7.2 Hz, 3H); LC-MS (ESI) *m*/*z* 236 (M+H)⁺.

**Step D:** To a stirred mixture of ethyl 1-chloroisoquinoline-3-carboxylate (940 mg, 4.0 mmol) in anhydrous THF (10 mL) at -30 °C was added 2M (4-fluorophenyl)magnesium bromide/diethyl ether (2.4 mL, 4.8 mmol), and the mixture was stirred at -30 °C for 2.5 h. 1N hydrochloric acid was then added and the mixture was extracted with EtOAc. The combined organic layers were dried over Na₂SO₄, filtered, and concentrated under reduced pressure. The residue was purified by silica gel flash chromatography and then by preparative reverse-phase HPLC to afford (1-chloroisoquinolin-3-yl)(4-fluorophenyl)methanone (130 mg, 11%). LC-MS (ESI) *m*/*z* 286 (M+H)⁺.

**Step E:** Preparation of tert-butyl 3-amino-5-methyl-1H-pyrazole-1-carboxylate.

To a stirred solution of 3-amino-5-mcthyl-pyrazole (1.94 g, 20 mmol) in N, N-dimethylformamide (20 mL) at 0 °C was added 60% sodium hydride/mineral oil (880 mg, 22 mmol) and the resulting mixture was stirred at rt for 30 min. Di-tert-butyl dicarbonatc (4.8 g, 22 mmol) was slowly added and the resulting mixture was stirred at rt for 2 h. The mixture was then poured into saturated aq sodium hydrogen carbonate and extracted with EtOAc. The combined organic layers were dried over Na₂SO₄, filtered and concentrated under reduced pressure. The crude product was purified by silica gel flash chromatography to afford tert-butyl 3-amino-5-methyl-1H-pyrazole-1-carboxylate (1.9 g, 48%) and tert-butyl 5-amino-3-methyl-1H-pyrazole-1-carboxylate (1.1 g, 28%). For tert-butyl 3-amino-5-methyl-1H-pyrazole-1-carboxylate ¹H NMR (400 MHz, DMSO-d₆) δ 5.60 (s, 1H), 5.28 (s, 2H), 2.34 (s, 3H), 1.51 (s, 3H). For tert-butyl 5-amino-3-methyl-1H-pyrazole-1-carboxylate ¹H NMR (400 MHz, DMSO-d₆) δ 6.22 (s, 2H), 5.15 (s, 1H), 2.00 (s, 3H), 1.54 (s, 3H); LC-MS (ESI) *m*/*z* 198 (M+H)⁺

**Step F:** A stirred mixture of (1-chloroisoquinolin-3-yl)(4-fluorophenyl)methanone from Step D (125 mg, 0.44 mmol), tert-butyl 3-amino-5-methyl-1H-pyrazole-1-carboxylate from Step E (130 mg, 0.66 mmol), tris(dibenzylideneacetone)dipalladium (0) (40 mg, 0.044 mmol), 4,5-bis(diphenylphosphino)-9,9-dimethylxanthene (25 mg, 0.044 mmol), and cesium carbonate (143 mg, 0.44 mmol) was heated at 70 °C for 2 h and then at 100 °C for 2 h. After cooling to rt, the mixture was concentrated under reduced pressure and purified by silica gel flash chromatography to afford tert-butyl 3-(3-(4-fluorobenzoyl)isoquinolin-1-ylamino)-5-methyl-1H-pyrazole-1-carboxylate as a yellow solid (100 mg, 51%). LC-MS (ESI) *m*/*z* 447 (M+H)⁺.

**Step G:** tert-Butyl 3-(3-(4-fluorobenzoyl)isoquinolin-1-ylamino)-5-methyl-1H-pyrazole-1-carboxylate (140 mg, 0.31 mmol) was treated with 2N hydrogen chloride/ 1,4-dioxane (2 mL) and the mixture was stirred at rt for 2 h, then concentrated under reduced pressure and adjusted to pH 7 with aq sodium hydrogen carbonate. The mixture was extracted with EtOAc and the combined organic layers were dried over Na₂SO₄, filtered, and concentrated under reduced pressure. The crude product was purified by silica gel chromatography to afford (4-fluorophenyl)(1-(5-methyl-1H-pyrazol-3-ylamino)isoquinolin-3-yl)methanone as a solid (30 mg, 28%). ¹H NMR (400 MHz, DMSO-d₆) δ 11.97 (s, 1H), 9.81 (br s, 1H), 8.69 (d, *J*= 7.2 Hz, 1H), 8.06-8.14 (m, 3H), 7.88 (s, 1H), 7.73-7.82 (m, 2H), 7.37 (t, *J* = 8.8 Hz, 2H), 6.24 (s, 1H), 2.12 (s, 3H); LC-MS (ESI) *m*/*z* 347 (M+H)⁺.

### Example 2

### Preparation of (R,S)-1-(4-fluorophenyl)(1-(5-methyl-1H-pyrazol-3-ylamino)isoquinolin-3-yl)methanol

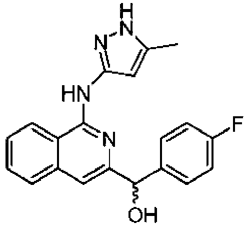

To a suspension of (4-fluorophenyl)(1-(5-methyl-1H-pyrazol-3-ylamino)isoquinolin-3-yl)methanone from Example 1 (90 mg, 0.26 mmol) in MeOH (5 mL) was added sodium borohydride (20 mg, 0.52 mmol) and the mixture was stirred at rt for 2 h. The mixture was concentrated under reduced pressure and the residue was partitioned between water and EtOAc. The separated aqueous phase was extracted with EtOAc and the combined organic layers were dried over magnesium sulfate, filtered, and concentrated under reduced pressure. The residue was purified by preparative reverse-phase HPLC to afford (*R*,*S*)-(4-fluorophenyl)(1-(5-methyl-1H-pyrazol-3-ylamino)isoquinolin-3-yl)methanol as a solid (40 mg, 44%). ¹H NMR (400 MHz, DMSO-d₆) δ 11.91 (br s, 1H), 9.68 (br s, 1H), 8.49 (d, *J* = 8.0 Hz, 1H), 7.80 (d, *J* = 6.8 Hz, 1H), 7.65 (t, *J* = 7.6 Hz, 1H), 7.48-7.51 (m, 3H), 7.1 (s, 1H), 7.14 (t, *J* = 8.8 Hz, 2H), 6.15-6.17 (m, 2H), 5.70 (s, 1H), 2.20 (s, 3H); LC-MS (ESI) *m*/*z* 347 [M+H)⁺.

### Example 3

### Preparation of (4-fluorophenyl)(4-(5-methyl-1H-pyrazol-3-ylamino)quinolin-2-yl)methanone

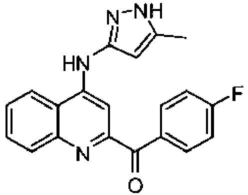

**Step A:** To a stirred solution of ethyl 4-chloroquinoline-2-carboxylate (WO 2006/87543) (1.37 g, 5.8 mmol) in THF at -30 °C under N₂ was added 2M (4-fluorophenyl)magnesium bromide/diethyl ether (2.9 mL, 5.8 mmol) dropwise over 20 min. The mixture was stirred at -40 °C for 1 h, and then allowed to warm for 10 min. The mixture was poured onto aqueous acid and extracted with EtOAc, and the combined organic layers were dried over Na₂SO₄, and concentrated under reduced pressure. The residue was chromatographed over silica gel eluting with 20:2 petroleum ether/EtOAc to afford (4-chloroquinolin-2-yl)(4-fluorophenyl]methanone as a white solid (1.60 g, 96%). Rr 0.67 (silica gel TLC, 15:1 petroleum ether/EtOAc).

**Step B:** A procedure analogous to that described in Example 1 Steps F and G is followed, substituting (4-chloroquinolin-2-yl)(4-fluorophenyl)methanone for the (1-chloroisoquinolin-3-yl)(4-fluorophenyl)methanone used in Example I Step F to afford (4-fluorophenyl)(4-(5-methyl-1H-pyrazol-3-ylamino)quinolin-2-yl)methanone.

### Example 4

### Preparation of (R,S)-(4-Fluorophenyl)(4-((5-methyl-1H-pyrazol-3-yl]amino]quinolin-2-yl)methanol

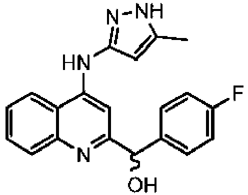

(R,S)-(4-Fluorophenyl)(4-((5-methyl-1H-pyrazol-3-yl)amino)quinolin-2-yl)methanol is prepared using a procedure analogous to that described in Example 2, substituting (4-fluorophenyl)(4-((5-methyl-1H-pyrazol-3-yl)amino)quinolin-2-yl)methanone for the (4-fluorophenyl)(1-(5-methyl-1H-pyrazol-3-ylamino)isoquinolin-3-yl)methanone used in Example 2.

### Example 5

### Preparation of (R,S)-2-((Cyclopropylamino)(4-fluorophenyl]methyl)-N-(5-methyl-1H-pyrazol-3-yl)quinolin-4-amine

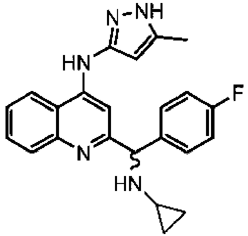

To (4-fluorophenyl)(4-(5-methyl-1H-pyrazol-3-ylamino)quinolin-2-yl)methanone from Example 3 (0.25 mmol) in 2-propanol (3 mL) are added cyclopropylamine (0.25 mL) and 3Å (8-12 mesh) molecular sieves, and the mixture is heated in a sealed vial at 90 °C for 4 d. Then AcOH (0.4 mL) is added followed by portionwise additions of sodium borohydride (340 mg) in MeOH (4 - 5 mL) and the mixture is stirred at rt until the product is substantially formed by LCMS. DMSO is added and the mixture is purified by preparative HPLC to afford (R,S)-2-((Cyclopropylamino)(4-fluorophenyl)methyl)-N-(5-methyl-1H-pyrazol-3-yl)quinolin-4-amine.

### Example 6

### Preparation of (R,S)- 2-(Amino(4-fluorophenyl)methyl)-N-(5-methyl-1H-pyrazol-3-yl)quinolin-4-amine

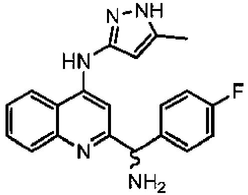

**Step A:** To a solution of 2-amino-2-(4-fluorophenyl)acetic acid (5 g, 29.5 mmol) in THF (50 mL) at 50 °C was added triphosgene (8.77 g, 29.5 mmol), then heating was continued for 3 h. The reaction mixture was then filtered and evaporated to a volume of about 10 mL, followed by addition of 150 mL of hexanes. The mixture was heated slightly, and then cooled to -20 °C for 1 h. The crude slurry was filtered to give 4-(4-fluorophenyl)oxazolidine-2,5-dione (5.03 g, 87%) which was used without further purification.

**Step B:** To a solution of 4-(4-fluorophenyl)oxazolidine-2,5-dione (2.5 g, 12.8 mmol) in THF (30 mL) cooled to -25 °C was added benzyl chloroformate (2.3 mL, 16.6 mmol), followed by the slow addition (∼ 10 min.) of N-methylmorpholine (2.11 mL, 19.2 mmol) as a solution in THF (5 mL). The solution was stirred at this temperature for 1 h. and then allowed to warm to rt overnight. The resulting solution was filtered through Celite, and the filtrate was concentrated. The resulting crude material was recrystallized from 2:2:1 t-butyl methyl ether : hexanes : THF to give benzyl 4-(4-fluorophenyl)-2,5-dioxooxazolidine-3-carboxylale (2.7 g, 64%) which was used without further purification.

**Step C:** To a solution of 2-aminoacetophenone (4.5 mmol) in THF (10 mL) is added benzyl 4-(4-fluorophonyl)-2,5-dioxooxazolidine-3-carboxylate (1.43 g, 4.34 mmol) and the mixture is heated at 50 - 70 °C for 3 - 5 h. Then potassium t-butoxide (10 mmol) in t-butanol is added and the reaction is heated at 65 - 100 °C for 6 - 12 h. The mixture is concentrated and the residue is purified by silica gel chromatography to afford (R,S)-2-(amino(4-fluorophenyl)methyl)-N-(5-methyl-1H-pyrazol-3-yl)quinolin-4-amine.

**Step D.** To a solution of (R,S)-2-(amino(4-fluorophenyl)methyl)-N-(5-methyl-1H-pyrazol-3-yl)quinolin-4-amine (1 mmol) in DCM (5 mL) at 0 °C arc added pyridine (5 mmol) and trifluoromethanesulfonic anhydride (1.1 mmol), and the mixture is stirred at 0 °C to rt for 2 - 4 h. The mixture is concentrated and the residue is purified by silica gel chromatography to afford 2-((benzyloxycarbonylamino)(4-fluorophenyl)methyl)quinolin-4-yl trifluormethanesulfonate.

**Step E.** The procedures of Example 1 Steps F and G is followed, substituting -((benzyloxycarbonylamino)(4-fluorophenyl)methyl)quinolin-4-yl trifluormethanesulfonate for the (1-chloroisoquinolin-3-yl)(4-fluorophenyl)methanone used in Example 1 Step F. The crude product isolated following aqueous work-up is subjected to catalytic hydrogenolysis to remove the Cbz protecting group. The product is purified by reverse-phase HPLC to afford (R,S)- 2-(amino(4-fluorophenyl)methyl)-N-(5-methyl-1H-pyrazol-3-yl)quinolin-4-amine.

### Example 7

### Preparation of 2-((4-fluorophenyl)(methoxy)methyl)-N-(5-methyl-1H-pyrazol-3-yl)quinolin-4-amine

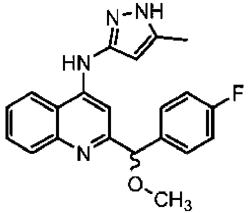

To (R,S)-(4-Fluorophenyl)(4-((5-methyl-1H-pyrazol-3-yl)amino)quinolin-2-yl)methanol from Example 4 (ca. 1 mmol) in MeOH (2 mL) is added methanesulfonic acid (0.2 mL) and the mixture is heated under microwave irradiation at 100 - 150°C for 0.4 - 3 h, or until the reaction is substantially complete. After concentration, the reaction mixture is purified by reverse-phase HPLC to afford 2-((4-fluorophenyl)(methoxy)methyl)-N-(5-methyl-1H-pyrazol-3-yl)quinolin-4-amine.

### Example 8

### Preparation of 2-(2-(4-fluorophenyl)-1,3-dioxolan-2-yl)-N-(5-methyl-1H-pyrazol-3-yl)quinolin-4-amine

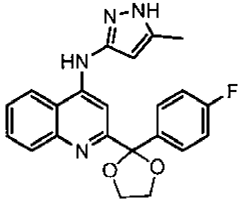

To (4-fluorophenyl)(4-(5-methyl-1H-pyrazol-3-ylamino)quinolin-2-yl)methanone from Example 3 (ca. 1 mmol) in toluene (ca. 30 mL) are added ethylene glycol (0.4 mL) and p-toluenesulfonic acid monohydrate (0.1 - 0.5 equiv). The mixture is heated under reflux overnight while collecting water in a Dean-Stark trap. As needed, additional ethylene glycol and p-toluenesulfonic acid monohydrate arc added, and heating at reflux with collection of water is continued, until product formation is substantially complete. After cooling, the mixture is evaporated to dryness, and the residue is dissolved in DMSO. The solution is purified by preparative reverse-phase HPLC to afford 2-(2-(4-fluorophenyl)-1,3-dioxolan-2-yl)-N-(5-methyl-1H-pyrazol-3-yl)quinolin-4-amine.

### Example 9

### Preparation of 3-(difluoro(4-fluorophenyl)methyl)-N-(5-methyl-1H-pyrazol-3-yl)isoquinolin-1-amine

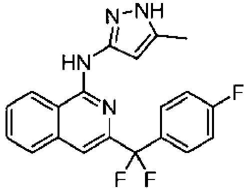

**Step A:** 2,2-Difluoro-2-(4-fluorophenyl)acetic acid was prepared according to Middleton et al., J. Org. Chcm., 1980, 45(14): 2883-2887) by reaction of ethyl 2-(4-fluorophenyl)-2-oxoacetate with (diethylamino)sulfur trifluoride followed by ester saponification and acidic workup. To a stirred solution of 2,2-difluoro-2-(4-fluorophenyl)acetic acid (1 g, 5.26 mmol) and N,O-dimethylhydroxylamine (616 mg, 6.31 mmol) in anhydrous DCM (14 mL) was added TEA (1.8 mL, 13.15 mmol) and the mixture was stirred at rt for 10 min. 2-(7-aza-1H-benzotriazole-1-yl)-1,1,3,3-tetramethyluronium hexafluorophosphate (3 g, 7.89 mmol) was added and the mixture was stirred at rt for 15 h. The mixture was partitioned between DCM and water while maintaining an aqueous phase at pH ∼8 by addition of saturated aq solution of sodium bicarbonate. The layers were separated and the aqueous phase was extracted three times with DCM. The organic phases were combined, dried over MgSO₄, filtered, and concentrated under reduced pressure. The residue was purified by silica gel flash chromatography eluting with 0 - 50% EtOAc/hexane to afford 2,2-difluoro-2-(4-fluorophenyl)-N-methoxy-N-methylacetamide (1.03 g, 84%) as a colorless oil. ¹H NMR (300 MHz, DMSO-d₆) δ 7.59-7.54 (m, 2H), 7.40-7.34 (m, 2H), 3.47 (s, 3H), 3.18 (s, 3H); LC-MS (ESI) *m*/*z* 234 (M + H)⁺.

**Step B:** To a solution of o-tolunitrile (46 µL, 0.39 mmol) in anhydrous THF (46 µL) and DMPU (52 µL, 0.429 mmol) at -78 °C was added dropwise 0.21 mL of 2 M LDA in hexane/THF/benzene. The resulting mixture was stirred at -78 °C for 15 min. A solution of 2,2-difluoro-2-(4-fluorophenyl)-N-methoxy-N-methylacetamide (100 mg, 0.43 mmol) in THF (43 µL) was added dropwise and the mixture was stirred at -78 °C for 2 h, then saturated aq ammonium chloride was added. The mixture was allowed to warm to rt and partitioned between EtOAc and water. The separated aqueous phase was extracted three times with EtOAc, and the combined organic phases were dried over MgSO₄, filtered, and concentrated under reduced pressure. The residue was purified by silica gel flash chromatography eluting with 0 - 50% EtOAc/hexane to afford 2-(3,3-difluoro-3-(4-fluorophenyl)-2-oxopropyl)benzonitrile (17 mg, 14%). ¹H NMR (300 MHz, CDCl₃) δ 7.67-7.53 (m, 3H), 7.45-7.42 (m, 2H), 7.30-7.13 (m, 3H), 4.26 (s, 2H); LC-MS (ESI) *m*/*z* 290 (M + H)⁺.

**Step C:** 2-(3,3-Difluoro-3-(4-fluorophenyl)-2-oxopropyl)benzonitrile (17 mg, 0.0588 mmol) was treated with few drops of 12 M sulfuric acid at 0 °C and the mixture was stirred for 20 min. A few drops or water were added and the mixture was stirred for 10 min. The mixture was partitioned between EtOAc and water basified to pH 8 with 5% sodium hydroxide. The separated aqueous phase was extracted three times with EtOAc and the combined organic phases were dried over MgSO₄, filtered, and concentrated under reduced pressure. The residue was purified by silica gel flash chromatography eluting with 0 - 50% EtOAc/hexane to afford 3-(difluoro(4-fluorophenyl)methyl)isoquinolin-1-ol (2 mg, 12%). ¹H NMR (300 MHz, DMSO-d₆) δ 11.86 (s, 1H), 8.20 (d, *J* = 8.1 Hz, 1H), 7.79-7.71 (m, 4H), 7.62-7.57 (m, 1H), 7.39 (t, *J* = 8.9 Hz, 2H), 6.85 (s, 1H); LC-MS (ESI) *m*/*z* 290 (M + H)⁺.

**Step D:** To a solution of 3-(difluoro(4-fluorophenyl)methyl)isoquinolin-1-ol (1 mmol) in toluene (2 mL) is added phosphorus (III) oxychloride (5 mmol) and the resulting mixture is heated at 120 °C for 1 h. The mixture is cooled to rt and concentrated under reduced pressure. The residue is partitioned between EtOAc and saturated aq sodium hydrogen carbonate and the separated aqueous phase is extracted three times with EtOAc. The combined organic phases are dried over MgSO₄, filtered, and concentrated under reduced pressure to afford 1-chloro-3-(difluoro(4-fluorophenyl)methyl)isoquinoline.

**Step E:** A procedure analogous to that described in Example 1 Step F is followed, substituting 1-chloro-3-(difluoro(4-fluorophenyl)methyl)isoquinoline for the (1-chloroisoquinolin-3-yl)(4-fluorophenyl)methanone used in Example 1; reaction time is extended and additional tert-butyl 3-amino-5-methyl-1H-pyrazole-1-carboxylate, tris(dibenzylideneacetone)dipalladium (0), 4,5-bis(diphenylphosphino)-9,9-dimethylxanthene, and cesium carbonate are added as needed to effect substantially complete reaction. Workup as in Example 1 Step F and further processing and purification under the conditions described in Example 1 Step G affords 3-(difluoro(4-fluorophenyl)methyl)-N-(5-methyl-1H-pyrazol-3-yl)isoquinolin-1-amine.

### Example 10

### Preparation of 3-(4-fluorophenylsulfonyl)-N-(5-methyl-1H-pyrazol-3-yl)isoquinolin-1-amine

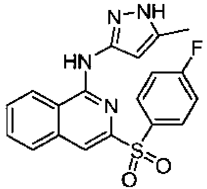

**Step A:** To anhydrous MeOH (25 mL) was added portionwise sodium metal (436 mg, 18 mmol), and after the mixture was homogeneous, 1,3-dichloroisoquinoline (2.4 g, 12.1 mmol) in acetonitrile (20 mL) was added. The resulting mixture was heated at 65 °C for 1.5 h, and then concentrated under reduced pressure. The residue was partitioned between EtOAc and water and the aqueous phase was extracted three times with EtOAc. The combined organic phases were dried over MgSO₄, filtered, and concentrated under reduced pressure to afford 3-chloro-1-methoxyisoquinoline as a pale yellow solid (2.2 g, 94%), which was used directly in the next step. ¹H NMR (300 MHz, DMSO-*d*₆) δ 8.16 (d, *J*= 9 Hz, 1H), 7.89 (d, *J* = 9 Hz, 1H), 7.81 (t, *J* = 7.0 Hz, 1H), 7.64 (t, *J* = 7.0 Hz, 1H), 7.57 (s, 1H), 4.08 (s, 3H); LC-MS (ESI) *m*/*z* 194 (M+H)⁺.

**Step B:** A mixture of 3-chloro-1-methoxyisoquinoline (1 g, 5.18 mmol), 4-fluorothiophenol (1.4 mL, 12.94 mmol) and Cs₂CO₃ (4.2 g, 12.94 mmol) in DMA (15 mL) in a 20 mL scaled tube was stirred and heated in a microwave synthesizer at 100 °C for 20 min. The mixture was partitioned between EtOAc and water, and the separated aqueous phase was extracted three times with EtOAc. The combined organic phases were dried over MgSO₄, filtered, and concentrated under reduced pressure. The residue was triturated with DCM/MeOH and the solid was collected by filtration to afford 3-(4-fluorophenylthio)isoquinolin-1-ol as a colorless solid (998 mg, 71%). ¹H NMR (300 MHz, DMSO-*d*₆) δ 11.74 (br s, 1H), 8.13 (d, *J* = 9 Hz, 1H), 7.72-7.64 (m, 1H), 7.61-7.46 (m, 3H), 7.32-7.24 (m, 3H), 6.61 (s, 1H); LC-MS (ESI) *m*/*z* 272 (M+H)⁺.

**Step C:** To a stirred solution of 3-(4-fluorophenylthio)isoquinolin-1-ol (745 mg, 2.75 mmol) in toluene (1 mL) was added phosphorus (V) oxybromide (3.1 g, 11 mmol). The mixture was heated at 120°C for 1 h, allowed to cool to rt, and partitioned between EtOAc and water basified with 10% aq sodium hydroxide to pH 8. The separated aqueous phase was extracted three times with EtOAc and twice with DCM. The combined organic phases were dried over MgSO₄, filtered, and concentrated under reduced pressure to afford 1-bromo-3-(4-fluorophenylthio)isoquinoline as a tan solid (920 mg, quantitative), which was used directly in the next step. ¹H NMR (300 MHz, DMSO-*d*₆) δ 8.14 (d, *J* = 6 Hz, 1H), 7.90-7.67 (m, 5H), 7.46 (s, 1H), 7.38 (t, *J* = 9.0 Hz, 2H); LC-MS (ESI) *m*/*z* 334/336 (Br isotopic pattern).

**Step D:** To a stirred solution of 1-bromo-3-(4-fluorophenylthio)isoquinoline (300 mg, 0.89 mmol) in DCM (9 mL) at -10 °C was added *m*-CPBA (200 mg, 1.15 mmol) and the mixture was stirred at 0 °C for 15 min. The mixture was filtered washing with EtOAc, and the filtrate was concentrated under reduced pressure. The residue was purified by silica gel flash chromatography eluting with EtOAc/ petroleum ether to afford 1-bromo-3-(4-fluorophenylsulfinyl)isoquinoline (273 mg, 87%). ¹H NMR (300 MHz, DMSO-*d*₆) δ 8.58 (s, 1H), 8.29 (dd, *J* = 16.8, 6.0 Hz, 2H), 8.00-7.93 (m, 2H), 7.88-7.85 (m, 2H), 7.43 (t, *J* = 6.0 Hz, 2H); LC-MS (ESI) *m*/*z* 349/351 (Br isotopic pattern).

**Step E:** A mixture of 1-bromo-3-(4-fluorophenylsulfinyl)isoquinoline (200 mg, 0.571 mmol), *tert*-butyl 3-amino-5-methyl-1H-pyrazole-1-carboxylate from Example I Step E (135 mg, 0.686 mmol), 4,5-bis-(diphenylphosphino)-9,9-dimethylxanthene (20 mg, 0.0343 mmol), tris(dibenzylideneacetone)dipalladium (0) (10 mg, 0.0114 mmol), 2M aq sodium carbonate (0.4 mL, 0.86 mmol), and toluene (4 mL) was degassed under Ar for 20 min, heated at 80 °C for 15 h, and allowed to cool to rt. The mixture was concentrated under reduced pressure and the residue was partitioned between EtOAc and water. The separated aqueous phase was extracted three times with EtOA, and the combined organic phases were dried over MgSO₄, filtered, and concentrated under reduced pressure. The residue was purified by silica gel flash chromatography eluting with 0-50% EtOAc/hexane to afford *tert*-butyl 3-(3-(4-fluorophenylsulfinyl)isoquinolin-1-ylamino)-5-methyl-1H-pyrazole-1-carboxylate as a pale yellow solid (216 mg, 81%). ¹H NMR (300 MHz, DMSO-*d*₆) δ 10.45 (s, 1H), 8.65 (d, *J* = 8.4 Hz, 1H), 8.06 (d, *J* = 7.5 Hz, 1H), 7.88-7.79 (m, 4H), 7.69 (t, *J* = 8.4 Hz, 1H), 7.41 (t, *J* = 9 Hz, 2H), 6.32 (s, 1H), 3.29 (s, 3H), 1.58 (s, 9H); LC-MS (ESI) *m*/*z* 367 ([M-Boc] + H)⁺.

**Step F:** To a stirred solution of *tert*-butyl 3-(3-(4-fluorophenylsulfinyl)isoquinolin-1-ylamino)-5-methyl-1H-pyrazole-1-carboxylate (216 mg, 0.463 mmol) in DCM (2 mL) at 0 °C was added *m*-CPBA (159 mg, 0.927 mmol) and the resulting mixture was stirred at 0 °C for 1 h. The precipitated solid was collected by filtration and dried to afford *tert*-butyl 3-(3-(4-fluorophenylsulfonyl)isoquinolin-1-ylamino)-5-methyl-1H-pyrazole-1-carboxylate (78 mg). The filtrate was purified by silica gel flash chromatography eluting with 0-50% EtOAc/hexane to afford additional *tert*-butyl 3-(3-(4-fluorophenylsulfonyl)isoquinolin-1-ylamino)-5-methyl-1H-pyrazole-1-carboxylate, which was combined with the previously obtained solid (combined yield 214 mg, 96%). ¹H NMR (300 MHz, DMSO-*d*₆) δ 10.61 (s, 1H), 8.73 (d, *J* = 8.3 Hz, 1H), 8.16-8.18 (m, 2H), 8.07 (dd, *J* = 8.9, 5.1 Hz, 2H), 7.88 (t, *J* = 7.3 Hz, 1H), 7.81-7.76 (m, 1H), 7.52 (t, *J* = 8.8 Hz, 2H), 6.40 (s, 1H), 3.29 (s, 3H), 1.59 (s, 9H); LC-MS (ESI) *m*/*z* 483 (M+H)⁺, 383 ([M-Boc] + H)⁺.

**Step G:** To a stirred solution of *tert*-butyl 3-(3-(4-fluorophenylsulfonyl)isoquinolin-1-ylamino)-5-methyl-1H-pyrazole-1-carboxylate (136 mg, 0.28 mmol) in DCM (1.4 mL) was added 4N HCl in 1,4-dioxane (0.21 mL, 0.843 mmol) and the mixture was stirred at rt for 1h. TFA (0.1 mL) was added dropwise and the mixture was stirred for 6 h. The mixture was then poured into saturated aq sodium hydrogen carbonate and extracted three times with DCM. The combined organic layers were dried over MgSO₄, filtered, and concentrated under reduced pressure to afford 3-(4-fluorophenylsulfonyl)-N-(5-methyl-1H-pyrazol-3-yl)isoquinolin-1-amine as an off-white solid (69.8 mg, 65%). ¹H NMR (300 MHz, DMSO-*d*₆) δ 10.09 (s, 1H), 8.67 (d, *J* = 8.3 Hz, 1H), 8.12-8.03 (m, 5H), 7.86-7.81 (m, 1 H), 7.77-7.72 (m, 1H), 7.50 (t, *J* = 8.8 Hz, 2H), 6.11 (s, 1H), 2.26 (s, 3H); LC-MS (ESI) *m*/*z* 383 (M+H)⁺.

### Example 11

### Preparation of N-(3-(4-fluorophenylsulfinyl)isoquinolin-1-yl)thiazol-2-amine

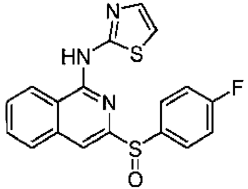

**Step A:** To a mixture of 1-bromo-3-(4-fluorophenylsulfinyl)isoquinoline from Example 10 Step D (100 mg, 0.283 mmol) in toluene (2 mL) were added Pd₂(dibenzylideneacetone)₃ (11 mg, 0.012 mmol), 4,5-bis(diphenylphosphino)-9,9-dimethylxanthene (20 mg, 0.036 mmol), 2-aminothiazole (35 mg, 0.34 mmol), and Na₂CO₃ (43 mg, 0.40 mmol), and then water (1 drop) was added with stirring. The reaction vessel was sealed under nitrogen and the mixture was heated in a microwave synthesizer at 100 °C for 1.5 h. The mixture was dissolved in 1:1 DCM/MeOH and filtered. The filtrate was concentrated and the residue was triturated with Et₂O and EtOAc. The residue was purified by silica gel chromatography eluting with 20:1 DCM/MeOH to afford N-(3-(4-fluorophenylsulfinyl)isoquinolin-1-yl)thiazol-2-amine (35 mg, 16 %) as a yellow solid. ¹H NMR (300 MHz, DMSO-*d*₆) δ 7.15 (br s, 1H), 7.38 (t, 2H), 7.51 (s, 1H), 7.74 (t, 1H), 7.86 (t, 1H), 7.94-7.98 (m, 3H), 8.12 (d, 1H), 8.78 (d, 1H), 12.09 (br s, I H); LC-MS (ESI) *m*/*z* 370 (M + H)⁺.

### Example 12

### Preparation N-(3-(4-fluorophenylsulfinyl)isoquinolin-1-yl)-5-methylthiazol-2-amine

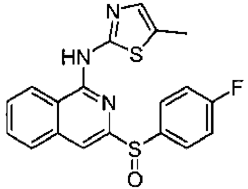

To 1-bromo-3-(4-fluorophenylsulfinyl)isoquinoline from Example 10 Step D (200 mg, 0.57 mmol) in toluene (4 mL) were added Pd₂(dibenzylideneacetone)₃ (23 mg, 0.025 mmol), 4,5-bis(diphenylphosphino)-9,9-dimethylxanthene (45 mg, 0.075 mmol), 5-methylthiazol-2-amine (35 mg, 0.34 mmol), and Na₂CO₃ (87 mg, 0.80 mmol), and then water (1 drop) was added with stirring. The mixture was heated in a microwave synthesizer at 100 °C for 1.5 h. The mixture was dissolved in 1:1 DCM/MeOH and filtered. The filtrate was concentrated and the residue was triturated with Et₂O and EtOAc to afford N-(3-(4-fluorophenylsulfinyl)isoquinolin-1-yl)-5-methylthiazol-2-amine (145 mg, 66 %) as a yellow solid. ¹H NMR (300 MHz, DMSO-*d*₆) δ 2.3 (s, 3H) 7.14 (s, 1H) 7.39 (t, 2H) 7.71 (t, 1H) 7.83 (t, 1H) 7.92-7.96 (m, 3H) 8.08 (d, 1H) 8.70 (d, 1H) 11.88 (br s, 1H); LC-MS (ESI) *m*/*z* 384 (M + H)⁺.

### Example 13

### Preparation of 3-(4-fluorophenylsulfinyl)-N-(5-methyl-1H-pyrazol-3-yl)isoquinolin-1-amine

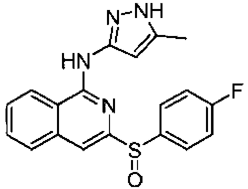

A sealed tube was charged with Pd₂(dibenzylidencacetone)₃ (14 mg, 0.016 mmol), 4,5-bis(diphenylphosphino)-9,9-dimethylxanthene (26 mg, 0.047 mmol), tert-butyl 3-amino-5-methyl-1H-pyrazole-1-carboxylate from Example 1 Step D (177 mg, 0.89 mmol), Na₂CO₃ (110 mg, 1.04 mmol), 1-bromo-3-(4-fluorophenylsulfinyl)isoquinoline from Example 10 Step D (260 mg, 0.74 mmol) and degassed toluene (5.2 mL), and then water (0.014 mL) was added with stirring. The sealed tube was heated in an oil bath at 100 °C for 2 h, and then in a microwave synthesizer at 100 °C for 1.5 h. The mixture was filtered and the filtrate was concentrated. The solid residue was triturated with MeOH and collected by filtration and dried under reduced pressure to afford tert-butyl 3-(3-(4-fluorophenylsulfinyl)isoquinolin-1-ylamino)-5-methyl-1H-pyrazole-1-carboxylate (166 mg, 40 %) as a white solid. ¹H NMR (300 MHz, DMSD-*d*₆) δ 1.52 (s, 9H), 2.25 (s, 3H), 6.46 (s, 1H), 7.40 (t, 2H), 7.83-7.90 (m, 4H), 8.00-8.03 (m, 2H), 8.17 (d, 1H), 10.61 (s, 1H); LC-MS (ESI) *m*/*z* 467 (M + H)⁺.

**Step B:** To a suspension of tert-butyl 3-(3-(4-fluorophenylsulfinyl)isoquinolin-1-ylamino)-5-methyl-1H-pyrazole-1-carboxylate (160 mg, 0.34 mmol) in EtOAc (1.5 mL) was added 4 M HCl/dioxane (1 mL), and the mixture was stirred at rt for 30 min. The mixture was concentrated and the residue was dissolved in water. The pH was adjusted to 7-8 with NaHCO₃, then the solution was extracted with 20:1 DCM/MeOH (3×20 mL). The organic layer was concentrated, and the residue was triturated with 50:1 Et₂O/MeOH, then collected by filtration and dried under reduced pressure to afford 3-(4-fluorophenylsulfinyl)-N-(5-methyl-1H-pyrazol-3-yl)isoquinolin-1-amine as a white solid (30 mg, 24 %). ¹H NMR (300 MHz, DMSO-*d*₆) δ 2.27 (s, 3H), 6.09 (s, 1H), 7.40 (t, 2H), 7.63-7.85 (m, 5H), 8.00-8.02 (m, 1H), 8.61 (d, 1H), 9.93 (s, 1H), 12.05 (s, 1H); LC-MS (ESI) *m*/*z* 367 (M + H)⁺.

### Example 14

### Preparation of 3-(4-fluorophenylsulfonyl)-N-(1H-pyrazol-3-yl)isoquinolin-1-amine

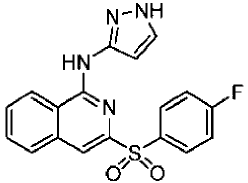

**Step A:** To a stirred solution of acrylonitrile (7.96 g, 150 mmol) in THF at 0 °C was added hydrazine monohydrate (9.70 g, 155 mmol) portionwise. The resulting mixture was stirred at 0 °C for 15 min, then warmed to rt and stirred for 1 h. To the mixture was added portionwise p-anisaldehyde (21.10 g, 155 mmol) and the mixture was stirred at rt for 17 h. The mixture was concentrated and n-butanol (30 mL) was added followed by a solution of sodium *tert*-butoxide (14.42 g, 150 mmol) in n-butanol (70 mL). The mixture was stirred at rt for 10 min, heated at 120 °C in a sealed tube for 3.5 h, then cooled to rt and stirred for 15 h. The mixture was poured into 1N HCl (600 mL) and extracted with diethyl ether (3x 100 mL). The aqueous phase was basified with 1N sodium hydroxide and extracted with diethyl ether (3x 120 mL). The latter organic layers were combined, dried over MgSO₄, filtered, and concentrated under reduced pressure. The residue was triturated with hexane, filtered, and dried to afford 1-(4-methoxybenzyl)-1H-pyrazol-5-amine as a yellow solid (14 g, 46 %). ¹H NMR (300 MHz, CDCl₃) δ 7.30-7.26 (m, 1H), 7.12 (d, *J* = 8.5 Hz, 2H), 6.86 (d, *J* = 8.5 Hz, 2H), 5.55 (s, 1H), 5.15 (s, 2H), 3.78 (s, 3H), 3.37 (s, 2H); LC-MS (ESI) *m*/*z* 204 (M + H)⁺.

**Step B:** 3-(4-fluorophenylsulfinyl)-N-(1-(4-methoxybenzyl)-1H-pyrazol-5-yl)isoquinolin-1-amine was prepared following a procedure analogous to that described in Example 10 Step E, substituting -(4-methoxybenzyl)-1H-pyrazol-5-amine (139 mg, 0.685 mmol) for the *tert*-butyl 3-amino-5-methyl-1H-pyrazole-1-carboxylate used in Example 10. The crude product was purified by silica gel flash chromatography eluting with 0 - 60% EtOAc/hexane to afford 3-(4-fluorophenylsulfinyl)-N-(1-(4-methoxybenzyl)-1H-pyrazol-5-yl)isoquinolin-1-amine as a pale yellow oil (240 mg, 89%). ¹H NMR (300 MHz, CDCl₃) δ 7.85-7.80 (m, 2H), 7.73-7.63 (m, 3H), 7.58-7.53 (m, 3H), 7.10-7.06 (m, 5H), 6.79 (d, *J*= 8.1 Hz, 2H), 6.22 (s, 1H), 5.13-5.01 (m, 2H), 3.74 (s, 3H); LC-MS (ESI) *m*/*z* 473 (M + H)⁺.

**Step C:** 3-(4-Fluorophenylsulfonyl)-N-(1-(4-methoxybenzyl)-1H-pyrazol-5-yl)isoquinolin-1-amine (163 mg, 66%) was obtained following a procedure analogous to that described in Example 10 Step F, substituting 3-(4-fluorophenylsulfinyl)-N-(1-(4-methoxybenzyl)-1H-pyrazol-5-yl)isoquinolin-1-amine for the *tert*-butyl 3-(3-(4-fluorophenylsulfonyl)isoquinolin-1-ylamino)-5-methyl-1H-pyrazole-1-carboxylate used in Example 10. ¹H NMR (300 MHz, DMSO-*d*₆) δ 9.61 (s, 1H), 8.43 (d, *J* = 8.3 Hz, 1H), 8.15 (d, *J* = 7.7 Hz, 1H), 8.07 (s, 1H), 7.90-7.79 (m, 4H), 7.56 (d, *J* = 1.7 Hz, 1H), 7.40 (t, *J* = 8.8 Hz, 2H), 6.91 (d, *J* = 8.7 Hz, 2H), 6.66 (d, *J* = 8.7 Hz, 2H), 6.14 (d, *J* = 1.7 Hz, 1H), 5.76 (s, 2H), 4.93 (s, 3H); LC-MS (ESI) *m*/*z* 489 (M+H)⁺.

**Step D:** To a stirred solution of 3-(4-fluorophenylsulfonyl)-N-(1-(4-methoxybenzyl)-1H-pyrazol-5-yl)isoquinolin-1-amine (154 mg, 0.315 mmol) in DCM (0.6 mL) at 0 °C was added dropwise TFA (0.24 mL), 3.15 mmol). The resulting mixture was stirred at rt for 5 h. Additional TFA (0.24 mL, 3.15 mmol) was added and the mixture was stirred at rt for a further 15 h. The mixture was partitioned between DCM/chloroform and cold saturated aq sodium hydrogen carbonate. The separated aqueous phase was extracted four times with chloroform. The combined organic phases were dried over MgSO₄, filtered, and concentrated under reduced pressure. The residue was purified by silica gel flash chromatography eluting with 0 - 10% MeOH /DCM to afford 3-(4-fluorophenylsulfonyl)-N-(1H-pyrazol-5-yl)isoquinolin-1-amine as a solid (66.7 mg, 57%). ¹H NMR (300 MHz, DMSO-*d*₆) δ 12.40 (s, 1H), 10.24 (s, 1H), 8.70 (d, *J* = 8.3 Hz, 1H), 8.12 (d, *J =* 7.9 Hz, 1H), 8.07-8.02 (m, 3H), 7.87-7.82 (m, 1H), 7.78-7.73 (m, 1H), 7.68 (br s, 1H), 7.47 (t, *J* = 8.8 Hz, 2H), 6.61 (br s, 1H); LC-MS (ESI) *m*/*z* 369 (M+H)⁺.

### Example 15

### Preparation of 3-(4-fluorophenylsulfonyl)-N-(5-methoxy-1H-pyrazol-3-yl)isoquinolin-1-amine

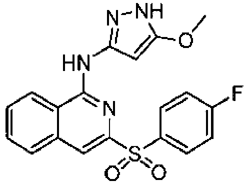

**Step A:** A stirred mixture of 1-nitropyrazole (3.45 g, 30.5 mmol) in benzonitrile (33 mL) was heated at 180°C for 3 h. The mixture was cooled to rt, diluted with hexane and stirred at rt for 20 min. The precipitated solid was collected by filtration to afford 3-nitro-1H-pyrazole as a tan solid (3.16 g, 91%). ¹H NMR (300 MHz, DMSO-*d*₆) δ 13.94 (br s, 1H), 8.03 (d, *J* = 2.4 Hz, 1H), 7.03 (t, *J* = 2.4 Hz, 1H).

**Step B:** To a stirred mixture of 3-nitro-1H-pyrazole (3.16 g, 27.9 mmol) in glacial acetic acid (20 mL) at 0 °C was added fuming nitric acid (2.6 mL, 58.69 mmol) dropwise, followed by acetic anhydride (6.6 mL, 69.87 mmol). The mixture was stirred and allowed to warm to rt over 3 h, then poured into ice water (50 mL) and stirred for 20 h. The mixture was extracted with EtOAc combined organic layers were dried over MgSO₄, filtered and concentrated to dryness to afford 1,3-dinitro-1H-pyrazole (4.3 g, 97%). ¹H NMR (300 MHz, DMSO-*d*₆) δ 8.00 (br s, 1H), 6.44 (br s, 1H).

**Step C:** A stirred mixture of 1,3-dinitro-1H-pyrazole (4.3 g, 27.20 mmol) in benzonitrile (60 mL) was heated at 180 °C for 3 h. The mixture was cooled to rt and partitioned between 1N sodium hydroxide and hexane. The organic layer was separated and the solid precipitate in the aqueous layer was filtered and triturated with toluene to afford 1.2 g of a pale yellow solid. The filtrate was neutralized with 1N HCl and extracted with EtOAc. The combined organic layers were dried over MgSO₄, filtered and concentrated to dryness. The residue was purified by silica gel flash chromatography elutingwith 0-30% EtOAc/hexane and then with 0-10% DCM/MeOH. The obtained solid was triturated with diethyl ether to afford 1.36 g of solid, which was combined with the previously obtained soled to afford 3,5-dinitro-1H-pyrazole (2.56 g, 59%). ¹H NMR (300 MHz, DMSO-*d*₆) δ 7.28 (s, 1H).

**Step D:** To a stirred mixture of 3,5-dinitro-1H-pyrazole (2.5 g, 15.81 mmol) and potassium carbonate (4.36 g, 31.62 mmol) in DMF (50 mL) at 0 °C was added (2-(chloromethoxy)ethyl)trimethylsilane (3.07 mL, 17.39 mmol) and the mixture was stirred at rt for 6 h. The mixture was concentrated under reduced pressure and the residue was purified by silica gel flash chromatography eluting with 0-20% EtOAc/hexane to afford 3,5-dinitro-1-((2-(trimethylsilyl)ethoxy)methyl)-1H-pyrazole as a colorless oil (2.7 g, 59%). ¹H NMR (300 MHz, CDCl₃) δ 7.68 (s, 1H), 6.00 (s, 2H), 3.72-3.67 (m, 2H), 0.97-0.91 (m, 2H), 0.00 (s, 9H).

**Step E:** To a stirred solution of anhydrous MeOH (25 mL) was added sodium (300 mg, 13.04 mmol) portionwise. To the clear solution was added SEM-protected 3,5-dinitropyrazole from Step D (1 g, 3.47 mmol) and the mixture was stirred at 60 °C for 2 h. The mixture was allowed to cool to rt and was concentrated under reduced pressure. The residue was purified by silica gel flash chromatography eluting with 0-30% EtOAc/hexane to afford a single regioisomer of SEM-protected 3-methoxy-5-nitropyrazole (SEM = ((2-(trimethylsilyl)ethoxy)methyl) as a clear oil (723 mg, 76%). ¹H NMR (300 MHz, CDCl₃) δ 6.23 (s, 1H), 5.41 (s, 2H), 4.02 (s, 3H), 3.70-3.65 (m, 2H), 0.96-0.91 (m, 2H), 0.00 (s, 9H).

**Step F:** To a stirred solution of SEM-protected 3-methoxy-5-nitropyrazole from Step E (723 mg, 2.65 mmol) in ethanol (20 mL) was added palladium on activated carbon (100 mg) and the resulting suspension was degassed and filled with hydrogen. After stirring at rt for 1 h, additional palladium on activated carbon (200 mg) was added and the mixture degassed and filled with hydrogen. The reaction mixture was stirred at rt for 75 h, filtered through Celite, and the filtrate was concentrated under reduced pressure. The residue was purified by silica gel flash chromatography eluting with 0-50% EtOAc/hexane and then with 0-20% DCM/MeOH to afford SEM-protected 3-amino-5-methoxypyrazole (478 mg, 74%). ¹H NMR (300 MHz, DMSO-*d*₆) δ 4.93-4.92 (m, 3H), 4.62 (s, 2H), 3.78 (s, 3H), 3.47 (t, *J* = 8.1 Hz, 2H), 0.88 (t, *J* = 8.1 Hz, 2H), -0.04 (s, 9H).

**Step G:** To a stirred solution of 1-bromo-3-(4-fluorophenylsulfinyl)isoquinoline from Example 10 Step D (200 mg, 0.571 mmol) in DCM (2 mL) at 0 °C was added *m*-CPBA (118 mg, 0.686 mmol) and the resulting mixture was stirred at 0 °C for 3 h. Additional *m*-CPBA (118 mg, 0.686 mmol) was then added and the mixture stirred for a further 3 h. The mixture was partitioned between DCM and saturated aq sodium hydrogen carbonate. The layers were separated and the water phase extracted three times with DCM. The combined organic phases were dried over MgSO₄, filtered, and concentrated under reduced pressure. The residue was purified by silica gel flash chromatography eluting with 50-100 % EtOAc/hexane to afford 1-bromo-3-(4-fluorophenylsulfonyl)isoquinoline as a colorless solid (200 mg, 96%). ¹H NMR (300 MHz, DMSO-d₆) δ 8.93 (s, 1H), 8.43-8.40 (m, 1H), 8.32 (d, *J* = 7.3 Hz, 1H), 8.13-8.03 (m, 4H), 7.53 (t, *J =* 8.9 Hz, 2H); LC-MS (ESI) *mlz* 366/368 (Br isotopic pattern).

**Step H:** A procedure analogous to that described in Example 10 Step E was followed, substituting 1-bromo-3-(4-fluorophenylsulfonyl)isoquinoline for the 1-bromo-3-(4-fluorophenylsulfinyl)isoquinoline used in Example 10, and substituting SEM-protected 3-amino-5-methoxypyrazole from Step F for the *tert*-butyl 3-amino-5-methyl-1H-pyrazole-1-carboxylate used in Example 10, to afford the expected SEM-protected intermediate (234 mg, 82%). ¹H NMR (300 MHz, CDCl₃) δ 8.16-8.11 (m, 3H), 8.06 (s, 1H), 7.94-7.92 (m, 2H), 7.79-7.67 (m, 2H), 7.20-7.14 (m, 2H), 6.56 (s, 1H), 5.24 (s, 2H), 4.06 (s, 3H), 3.63-3.57 (m, 2H), 0.97-0.91 (m, 2H), 0.01 (s, 9H); LC-MS (ESI) *m*/*z* 529 (M + H)⁺.

**Step I:** To a stirred solution of the SEM-protected intermediate (234 mg, 0.443 mmol) in DCM (0.2 mL) at 0 °C was added TFA (0.34 mL, 4.43 mmol) dropwise. The resulting mixture was stirred at rt for 3 h, then additional TFA (0.34 mL, 4.43 mmol) was then added and the mixture was stirred at rt for a further 2 h. The mixture was partitioned between DCM and a cold saturated sodium hydrogen carbonate. The layers were separated and the aqueous phase was extracted with DCM (4X). The combined organic phases were dried over MgSO₄, filtered, and concentrated under reduced pressure. The residue was purified by silica gel flash chromatography eluting with 0-5% MeOH/DCM and then by reverse phase HPLC eluting with 30% to 70% acctonitrile/0.5% aq HOAc to afford 3-(4-fluorophenylsulfonyl)-N-(5-methoxy-1H-pyrazol-3-yl)isoquinolin-1-amine (40 mg, 23%). ¹H NMR (300 MHz, DMSO-*d*₆) δ 11.29 (br s, 1H), 10.46 (br s, 1H), 8.53 (br s, 1H), 8.18-8.16 (m, 2H), 8.05 (dd, *J*= 8.8, 5.2 Hz, 2H), 7.92-7.87 (m, 2H), 7.49 (t, *J* = 8.8 Hz, 2H), 5.74 (br s, 1H), 3.83 (s, 3H); LC-MS (ESI) *m*/*z* 399 (M + H)⁺.

### Example 16

### Preparation of N-(3-(4-fluorophenylsulfonyl)isoquinolin-1-yl)-5-methylthiazol-2-amine

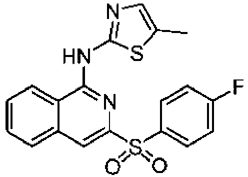

N-(3-(4-fluorophenylsulfonyl)isoquinolin-1-yl)-5-methylthiazol-2-amine was prepared following a procedure analogous to that described in Example 10 Step E, substituting 1-bromo-3-(4-fluorophenylsulfonyl)isoquinoline for the 1-bromo-3-(4-fluorophenylsulfinyl)isoquinoline used in Example 10, and 5-methylthiazol-2-aminc for the *tert*-butyl 3-amino-5-methyl-1H-pyrazole-1-carboxylate used in Example 10. The crude product was purified by silica gel flash chromatography eluting with 0 - 10% MeOH/DCM and the isolated product was further purified by reverse phase HPLC eluting with 30% - 80% acetonitrile/0.05% aq HOAc to afford N-(3-(4-fluorophenylsulfonyl)isoquinolin-1-yl)-5-methylthiazol-2-amine as a solid (27 mg, 49%). ¹H NMR (300 MHz, DMSO-*d*₆) δ 12.00 (br s, 1H), 8.72 (d, *J*= 8.1 Hz, 1H), 8.18-8.14 (m, 4H), 7.90-7.76 (m, 2H), 7.50 (t, *J* = 8.8 Hz, 2H), 7.13 (s, 1H), 2.36 (s, 3H); LC-MS (ESI) *m*/*z* 400 (M + H)⁺.

### Example 17

### Preparation of N-(3-(4-fluorophenylsulfonyl)isoquinolin-1-yl)thiazol-2-amine

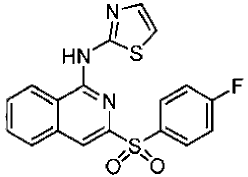

N-(3-(4-fluorophenylsulfonyl)isoquinolin-1-yl)thiazol-2-amine was prepared using a procedure analogous to that described in Example 10 Step E, substituting 1-bromo-3-(4-fluorophenylsulfonyl)isoquinoline for the 1-bromo-3-(4-fluorophenylsulfinyl)isoquinoline used in Example 10, and substituting thiazol-2-amine for the *tert*-butyl 3-amino-5-methyl-1H-pyrazole-1-carboxylate used in Example 10. The crude product was purified by silica gel flash chromatography eluting with 0 - 10 % MeOH/DCM and the isolated product was triturated with acetonitrile to afford N-(3-(4-fluorophenylsulfonyl)isoquinolin-1-yl)thiazol-2-amine as a solid (36 mg, 23%). ¹H NMR (300 MHz, DMSO-*d*₆) δ 12.20 (br s, 1H), 8.80 (br s, 1H), 8.27-8.16 (m, 4H), 7.94-7.81 (m, 2H), 7.50-7.44 (m, 3H), 7.17 (s, 1H); LC-MS (ESI) *m*/*z* 386 (M + H)⁺.

### Example 18

### Preparation of N-(3-(4-fluorophenylsulfonyl)isoquinolin-1-yl)-1,2,4-thiadiazol-5-amine

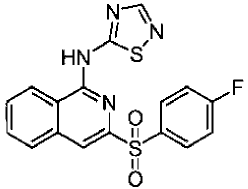

A mixture of 1-bromo-3-(4-fluorophenylsulfonyl)isoquinoline from Example 15 Step A (100 mg, 0.27 mmol), Pd₂(dibenzylideneacetone)₃ (10 mg, 0.011 mmol), 4,5-bis(diphenylphosphino)-9,9-dimethylxanthene (19 mg, 0.03 mmol), 1,2,4-thiadiazol-5-amine (34 mg, 0.33 mmol), and Na₂CO₃ (40 mg, 0.38 mmol) and toluene (4 mL) was evacuated and flushed with argon three times, and then heated at 100 °C overnight. The mixture was concentrated under reduced pressure and additional Pd₂(dibenzylideneacetone)₃ (10 mg, 0.011 mmol), 4,5-Bis(diphenylphosphino)-9,9-dimethylxanthene (19 mg, 0.03 mmol), and Na₂CO₃ (40 mg, 0.38 mmol) were added, followed by dioxane (3 mL). The reaction mixture was evacuated and flushed with argon three times, and then r heated in a microwave synthesizer at 140 °C for 30 min. The resulting mixture was purified by preparative HPLC (Varian diphenyl reverse phase column eluting with gradient of solvent B = 0.05% HOAC/ACN and solvent A = 0.05% HOAc/H₂O) to afford N-(3-(4-fluorophenylsulfonyl)isoquinolin-1-yl)-1,2,4-thiadiazol-5-amine (35 mg, 33 %). ¹H NMR (300 MHz, DMSO-*d*₆) δ 13.06 (br s, 1H), 8.91 (d, *J* = 8.1 Hz, 1H), 8.49 (s, 2H), 8.32 (d, *J*= 7.9 Hz, 1H), 8.18 (dd, *J* = 5.2, 8.8 Hz, 2H), 7.88 - 8.06 (m, 2H), 7.50 (t, *J* = 8.9 Hz, 2H); LC-MS (ESI) *m*/*z* 387 (M + H)⁺.

### Example 19

### Preparation of 3-(3-(4-fluorophenylsulfonyl)isoquinolin-1-ylamino)-1H-pyrazol-5-ol

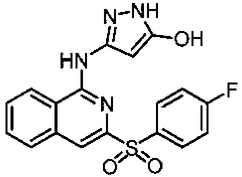

To a stirred solution of 3-(4-fluorophenylsulfonyl)-N-(5-methoxy-1H-pyrazol-3-yl)isoquinolin-1-amine from Example 15 (46 mg, 0.115 mmol), in DCM (0.6 mL) at 0 °C was added dropwise boron tribromide (0.23 mL, 0.23 mmol) and the resulting mixture was stirred at rt for 1 h. Additional boron tribromide (0.23 mL, 0.23 mmol) was then added dropwise and the mixture stirred for a further 5 h. Additional boron tribromide (0.23 mL, 0.23 mmol) was added and the mixture stirred for a further 15 h. Two additional portions (cach 0.23 mL, 0.231 mmol) were added followed by stirring for several hours. Then water was added at 0°C and the mixture was extracted three times with chloroform/lisopropano, and the combined organic layers were dried over MgSO₄, filtered, and concentrated under reduced pressure. The residue was purified by reverse phase HPLC eluting with 30% to 80% acetonitrile/0.05% aq HOAc to afford 3-(3-(4-fluorophenylsulfonyl)isoquinolin-1-ylamino)-1H-pyrazol-5-ol as a colorless solid (14 mg, 32%). ¹H NMR (300 MHz, DMSO-*d*₆) δ 11.59 (br s, 1H), 10.14 (br s, 2H), 8.59 (br s, 1H), 8.14-8.07 (m, 4H), 7.86-7.78 (m, 2H), 7.46 (t, *J*= 8.8 Hz, 2H), 5.84 (br s, 1H); LC-MS (ESI) *m*/*z* 385 (M + H)⁺.

### Example 20

### Preparation of 3-(4-fluorophenylsulfonyl)-N-(1-methyl-1H-imidazol-4-yl)isoquinolin-1-amine

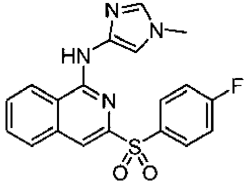

**Step A:** To a mixture of 4-nitro-1H-imidazole (2.0 g, 17.7 mmol) and K₂CO₃ (3.67 g, 26.6 mmol) in acetonitrile (18 mL) was added iodomethane (1.32 mL, 21.2 mmol) and the mixture was heated in a sealed vial at 60 °C overnight. The mixture was filtered washing with acetone. The filtrate was concentrated under reduced pressure, and the residue was diluted with hot isopropanol and cooled, and the precipitated solid was collected by filtration. The solid was dissolved in chloroform and filtered, and the filtrate was concentrated under reduced pressure. The residue was triturated with propan-2-ol and collected by filtration to afford 1-methyl-4-nitro-1H-imidazole (1.03 g, 46%) as a tan solid. ¹H NMR (300 MHz, DMSO-*d*₆) δ 8.37 (d, *J* = 1.1 Hz, 1H), 7.82 (s, 1H), 3.76 (s, 3H).

**Step B:** To 1-methyl-4-nitro-1H-imidazole (354 mg, 2.8 mmol) in EtOH (20 mL) was added 10% Pd-C (90 mg) and the mixture was stirred under a hydrogen atmosphere for 1.5 h. The mixture was filtered and the filtrate was concentrated under reduced pressure to afford 1-methyl-1H-imidazol-4-amine (250 mg, 92%) as a yellow oil that darkened upon standing. ¹H NMR (300 MHz, DMSO-*d₆*) δ 7.05 (s, 1H), 6.08 (d, *J* = 1.3 Hz, 1H), 4.06 (br s, 2H), 3.47 (s, 3H).

**Step C:** To a mixture of 1-bromo-3-(4-fluorophenylsulfonyl)isoquinoline from Example 15 Step G (150 mg, 0.4 mmol), Pd₂(dibenzylideneacetone)₃ (18 mg, 0.02 mmol), 4,5-Bis(diphenylphosphino)-9,9-dimethylxanthene (35 mg, 0.06 mmol), 1-methyl-1H-imidazol-4-amine (58 mg, 0.6 mmol), and 3M aq Na₂CO₃ (0.2 mL, 0.6 mmol) was added toluene (3 mL). The mixture evacuated and flushed with argon three times, and then heated at 90 °C overnight. The mixture was diluted with DCM and MeOH and concentrated under reduced pressure. The residue was purified by preparative HPLC (Varian diphenyl reverse phase column, eluted with gradient of solvent B = 0.05% HOAC/ACN and solvent A = 0.05% HOAc/H₂O). The obtained material was then further purified by preparative HPLC (Varian Pursuit XRs C-18 reverse phase column, eluted with gradient of solvent B = 0.05% formic acid/ACN and solvent A = 0.05% formic acid/ 5% ACN/H₂O) to afford 3-(4-fluorophenylsulfonyl)-N-(1-methyl-1H-imidazol-4-yl)isoquinolin-1-amine (14 mg, 9 %). ¹H NMR (300 MHz, DMSO-*d*₆) δ 10.23 (s, 1H), 8.75 (d, *J* = 8.3 Hz, 1H), 8.07 - 8.18 (m, 3H), 8.02 (s, 1H), 7.83 (t, *J* = 7.3 Hz, 1H), 7.69 - 7.77 (m, 1H), 7.52 (t, *J* = 8.8 Hz, 2H), 7.46 (s, 1H), 7.20 (s, 1H), 3.70 (s, 3H). LCMS (ESI) *m*/*z* 383 (M + H)⁺.

### Example 21

### Preparation of 3-(5-fluoropyridin-2-ylsulfonyl)-N-(1-methyl-1H-imidaxol-4-yl)isoquinolin-1-amine

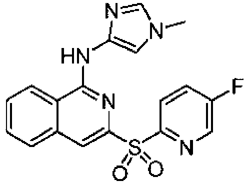

**Step A:** 5-Fluoropyridine-2-thiol is prepared using a procedure analogous to that described for preparation of pyridine-2-thiol (Thirtle, J. Am. Chem. Soc. 1946, 68, 342), substituting 2-bromo-5-fluoropyridine for the 2-bromopyridine used in the reported procedure.

**Step B:** A mixture of 5-fluoropyridine-2-thiol (2.5 equiv), 3-chloro-1-methoxyisoquinoline from Example 10 Step A (1 equiv), and Cs₂CO₃ (2.5 equiv) in DMA (15 mL) in a sealed tube is stirred and heated in a microwave synthesizer at 100 - 150°C for 20 - 60 min or until the reaction is substantially complete. The mixture is partitioned between EtOAc and water, and the separated aqueous phase is extracted several times with EtOAc. The combined organic phases are dried over MgSO₄, filtered, and concentrated under reduced pressure. The residue is purified by chromatography to afford 3-(5-fluoropyridin-2-ylthio)isoquinolin-1-ol.

**Step C:** To a stirred solution of 3-(5-fluoropyridin-2-ylthio)isoquinolin-1-ol. (1 equiv) in toluene is added phosphorus (V) oxybromide (4 equiv). The mixture is heated at 120°C for 1 h or until the reaction is substantially complet, then allowed to cool to rt. The mixture is partitioned between EtOAc and water to which 10% aq sodium hydroxide is added to pH 8. The separated aqueous phase is extracted several times with EtOAc and DCM, and the combined organic phases are dried over MgSO₄, filtered, and concentrated under reduced pressure. To the residue in DCM at 0 °C is added 3-chloroperbenzoic acid (2.3 equiv) and the mixture is stirred at 0 °C for 3 - 6 h or until the reaction is substantially complete, with addition of small additional quantities of 3-chloroperbenzoic acid as required. The mixture is partitioned between DCM and saturated aq sodium hydrogen carbonate. The separated aqueous phase is extracted three times with DCM, and the combined organic layers are dried over MgSO₄, filtered, and concentrated under reduced pressure. The residue is purified by silica gel chromatography to afford 1-bromo-3-(5-fluoropyridin-2-ylsulfonyl)isoquinoline.

**Step D:** 3-(5-fluoropyridin-2-ylsulfonyl)-N-(1-methyl-1H-imidazol-4-yl)isoquinolin-1-amine is prepared following a procedure analogous to that described in Example 20 Step C, substituting 1-bromo-3-(5-fluoropyridin-2-ylsulfonyl)isoquinoline for the 1-bromo-3-(4-fluorophenylsulfonyl)isoquinoline used in Example 20.

### Example 22

### Preparation of (R,S)-N-(3-(4-fluorophenylsulfinyl)isoquinolin-1-yl)-4-methyloxazol-2-amine

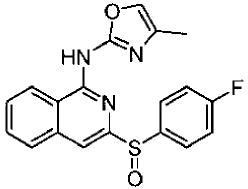

To a mixture of (R,S)-1-bromo-3-(4-fluorophcnylsulfinyl)isoquinoline from Example 13 Step D (150 mg, 0.43 mmol), Pd₂(dibenzylideneacetone)₃ (16 mg, 0.017 mmol), 4,5-Bis(diphenylphosphino)-9,9-dimethylxanthene (30 mg, 0.052 mmol), 4-methyloxazol-2-amine (50 mg, 0.52 mmol), and Na₂CO₃ (64 mg, 0.61 mmol) was added toluene (3 mL) and water (1 drop). The mixture was evacuated and flushed with argon three times and then heated at 100 °C overnight. The mixture was concentrated under reduced pressured and the residue was purified by preparative HPLC (Varian diphenyl reverse phase column, eluted with gradient of solvent B = 0.05% HOAC/ACN and solvent A = 0.05% HOAc/H₂O) to afford N-(3-(4-fluorophenylsulfinyl)isoquinolin-1-yl)-4-methyloxazol-2-amine (2 mg, 1%). ¹H NMR (300 MHz, DMSO-*d*₆) δ 11.41 (br s, 1H), 8.45 (d, *J =* 8.3 Hz, 1H), 8.02 (d, *J* = 8.1 Hz, 1H), 7.91 (dd, *J* = 5.4, 8.6 Hz, 2H), 7.77 - 7.86 (m, 2H), 7.64 - 7.74 (m, 1H), 7.59 (br s, 1H), 7.41 (t, *J*= 8.9 Hz, 2H), 2.18 (s, 3H). LCMS (ESI) *m*/*z* 368 (M + H)⁺.

### Example 23

### Preparation of N-(3-(4-fluorophenylsulfonyl)isoquinolin-1-yl)oxazol-2-amine

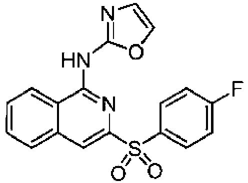

N-(3-(4-fluorophenylsulfonyl)isoquinolin-1-yl)oxazol-2-amine was prepared following a procedure analogous to that described in Example 10 Step E, substituting 1-bromo-3-(4-fluorophenylsulfonyl)isoquinoline for the 1-bromo-3-(4-fluorophenylsulfinyl)isoquinoline used in Example 10, and oxazol-2-amine for the *tert*-butyl 3-amino-5-methyl-1H-pyrazole-1-carboxylate used in Example 10. The crude product was purified by reverse phase HPLC eluting with 30% - 80% acetonitrile/0.05% aq HOAc to afford N-(3-(4-fluorophenylsulfonyl)isoquinolin-1-yl)oxazol-2-amine as a solid (19 mg, 37%). ¹H NMR (300 MHz, DMSO-d₆) δ 11.14 (br s, 2H), 8.51 (d, *J* = 8.1 Hz, 1H), 8.18-8.07 (m, 3H), 7.93-7.78 (m, 3H), 7.50 (t, *J*= 8.9 Hz, 2H), 7.33 (br s, 1H); LC-MS (ESI) *m*/*z* 370 (M + H)⁺.

### Example 24

### Preparation of 3-((4-fluorophenyl)sulfonyl)-N-(1-methyl-d₃-1H-imidazol-4-yl)isoquinolin-1-amine

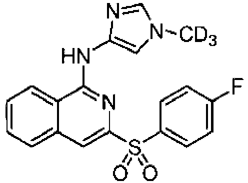

**Step A:** To 3-((4-fluorophenyl)thio)isoquinolin-1-ol from Example 10 Step B (998 mg, 0.57 mmol) in toluene (1 mL) was added POCl₃ (1.35 mL, 14.73 mmol) and the mixture was heated in a sealed tube at 120 °C for 1 h. The mixture was concentrated under reduced pressure and partitioned between DCM and saturated aq sodium bicarbonate. The organic layer was dried over magnesium sulfate and concentrated under reduced pressure to afford 1-chloro-3-((4-fluorophenyl)thio)isoquinoline (1.1 g, quantitative) as a yellow solid. ¹H NMR (300 MHz, CHLOROFORM-*d*) δ ppm 7.07 (s, 1 H) 7.17 (t, *J*=8.67 Hz, 2 H) 7.54 - 7.71 (m, 5 H) 8.25 (d, *J*=8.10 Hz, 1 H).

**Step B:** To 1-chloro-3-((4-fluorophenyl)thio)isoquinoline (1.1 g, 3.81 mmol) in DCM (20 mL) at 0 °C was added 70% 3-chloroperbenzoic acid (1.64 g, 9.51 mmol) and the mixture was stirred for 5 h. The mixture was partitioned between DCM and saturated aq sodium bicarbonate. The organic layer was concentrated under reduced pressure and the residue was purified by silica gel chromatography eluting with 0 - 10% MeOH/DCM to afford 1-chloro-3-((4-fluorophenyl)sulfonyl)isoquinoline (1.06 g, 87%). ¹H NMR (300 MHz, DMSO-*d*₆) δ ppm 7.52 (t, *J*=8.85 Hz, 2 H) 8.00 - 8.17 (m, 4 H) 8.34 - 8.48 (m, 2 H) 8.93 (s, 1 H).

**Step C:** To a mixture of 4-nitro-1H-imidazole (2 g, 17.7 mmol) and K₂CO₃ (3.67 g, 26.6 mmol) in acetonitrile (18 mL) was added iodomethane-*d₃* (1.32 mL, 21.15 mmol) and the mixture was heated in a sealed tube at 60 °C overnight. The mixture was allowed to cool, and then was filtered, washing with acetone. The filtrate was concentrated under reduced pressure and the residue was suspended in hot 2-propanol. The mixture was allowed to cool and the precipitated solid was collected by filtration, washed with 2-propanol, and then suspended in chloroform. The mixture was filtered and the filtrate was concentrated under reduced pressure to afford 1-methyl-*d₃*-4-nitro-1H-imidazole (870 mg, 38%). ¹H NMR (300 MHz, DMSO-*d*₆) δ ppm 7.81 (d, *J*=1.32 Hz, 1 H) 8.36 (d, *J*=1.32 Hz, 1 H); LC-MS (ESI) *m*/*z* 131 (M+H)⁺.

**Step D:** To 1-methyl-*d₃*-4-nitro-1H-imidazole (420 mg, 3.2 mmol) in EtOH (20 mL) was added palladium hydroxide (20% on carbon, 75 mg) and the mixture was stirred under an atmosphere of hydrogen for 1 h at rt and 2 h at 60 °C. The mixture was filtered and the filtrate was concentrated under reduced pressure to afford crude 1-methyl-D₃-1H-imidazol-4-amine (370 mg, quantitative) as a dark oil. LC-MS (ESI) *m*/*z* 101 (M+H)⁺.

**Step E:** To a mixture of 1-chloro-3-((4-fluorophenyl)sulfonyl)isoquinoline from step B (100 mg, 0.31 mmol), tris(dibenzylidencacetone)dipalladium (15 mg, 0.015 mmol), 4,5-bis(diphenylphosphino)-9,9-dimethylxanthene (27 mg, 0.047 mmol), 1-methyl-*d₃*-1H-imidazol-4-amine (47 mg, 0.46 mmol) and 3M aq Na₂CO₃ (0.16 mL, 0.46 mmol) was added toluene (3 mL). The reaction vessel was evacuated and flushed with argon (3X), and the mixture was heated in a microwave synthesizer at 130 °C for 20 min. The mixture was diluted with MeOH, concentrated under reduced pressure, and then purified by preparative HPLC (Varian C-18 reverse phase column, eluted with gradient of solvent B = 0.05% formic acid/ACN and solvent A = 5% ACN/0.05% formic acid/H₂O) to afford 3-((4-fluorophenyl)sulfonyl)-N-(1-methyl-*d₃*-1H-imidazol-4-yl)isoquinolin-1-amine (35 mg, 29%). ¹H NMR (300 MHz, DMSO-*d*₆) δ ppm 7.21 (d, *J*=1.51 Hz, 1 H) 7.47 (d, *J*=1.51 Hz, 1 H) 7.52 (t, *J*=8.85 Hz, 2 H) 7.69-7.77 (m, 1 H) 7.79 - 7.87 (m, 1 H) 8.03 (s, 1 H) 8.12 (dd, *J*=8.85, 5.46 Hz, 3 H) 8.76 (d, *J*=8.29 Hz, 1 H) 10.26 (s, 1 H); LC-MS (ESI) *m*/*z* 386 (M+H)⁺.

### Example 25

### Preparation of N-(1-ethyl-1H-imidazol-4-yl)-3-((4-fluorophenyl)sulfonyl)isoquinolin-1-amine

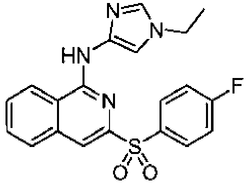

**Step A:** To a mixture of 4-nitro-1H-imidazole (2 g, 17.7 mmol) and K₂CO₃ (3.67 g, 26.6 mmol) in acetonitrile (18 mL) was added iodoethane (1.7 mL, 21.15 mmol) and the mixture was heated in a sealed tube at 60 °C overnight. The mixture was allowed to cool and then was filtered, washing with acetone. The filtrate was concentrated under reduced pressure, the residue was suspended in hot 2-propanol, and then the mixture was allowed to cool. The mixture was filtered and the filtrate was concentrated under reduced pressure to afford 1-ethyl-4-nitro-1H-imidazole (810 mg, 32%) as an orange oil. ¹H NMR (300 MHz, DMSO-*d*₆) δ ppm 1.41 (t, *J*=7.25 Hz, 3 H) 4.12 (q, *J*=7.35 Hz, 2 H) 7.91 (d, *J*=0.94 Hz, 1 H) 8.48 (d, *J*=1.13 Hz, 1 H); LC-MS (ESI) *m*/*z* 142 (M+H)⁺.

**Step B:** To 1-ethyl-4-nitro-1H-imidazole (810 mg, 5.7 mmol) in EtOH (20 mL) was added palladium hydroxide (20% on carbon, 150 mg) and the mixture was stirred under an atmosphere of hydrogen for 1 h at rt and 3 h at 60 °C. The mixture was filtered and the filtrate was concentrated under reduced pressure to afford crude 1-ethyl-1H-imidazol-4-amine (740 mg, quantitative) as a dark oil. LC-MS (ESI) *m*/*z* 112 (M+H)⁺.

**Step C:** To a mixture of 1-chloro-3-((4-fluorophenyl)sulfonyl)isoquinoline from Example 24 step B (150 mg, 0.46 mmol), tris(dibenzylideneacetone)dipalladium (22 mg, 0.023 mmol), 4,5-bis(diphenylphosphino)-9,9-dimethylxanthene (40 mg, 0.07 mmol), 1-ethyl-1H-imidazol-4-amine (100 mg, 0.9 mmol) and Na₂CO₃ (3 M, 0.235 mL, 0.7 mmol) was added toluene (4 mL). The reaction vessel was evacuated and flushed with argon (3X). The mixture was heated in a microwave synthesizer at 130°C for 20 min, and then diluted with McOH and concentrated under reduced pressure. The residue was purified by preparative HPLC (Varian C-18 reverse phase column, eluted with gradient of solvent B = 0.05% formic acid/ACN and solvent A = 5% ACN/0.05% formic acid/H₂O) to afford N-(1-ethyl-1H-imidazol-4-yl)-3-((4-fluorophenyl)sulfonyl)isoquinolin-1-amine (40 mg, 22%). ¹H NMR (300 MHz, DMSO-*d*₆) δ ppm 1.39 (t, *J*=7.25 Hz, 3 H) 4.02 (q, *J*=7.22 Hz, 2 H) 7.30 (d, *J*=1.32 Hz, 1 H) 7.46 - 7.56 (m, 3 H) 7.70 - 7.77 (m, 1 H) 7.79 - 7.86 (m, 1 H) 8.03 (s, 1 H) 8.07 - 8.14 (m, 3 H) 8.76 (d, *J*=8.29 Hz, 1 H) 10.26 (s, 1 H); LC-MS (ESI) *m*/*z* 397 (M+H)⁺.

### Example 26

### Preparation 5-bromo-3-((4-fluorophenyl)sulfonyl)-N-(5-methyl-1H-pyrazol-3-yl)isoquinolin-1-amine

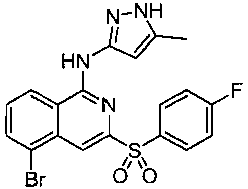

**Step A:** To 1,3-dichloroisoquinoline (5 g, 25.25 mmol) in acetonitrile (125 mL) were added concentrated sulfuric acid (5 mL) and N-bromosuccinimide (5.5 g. 29.41 mmol). The mixture was stirred at rt for 3 days, and then the solid precipitate was collected by filtration to afford 5-bromo-1,3-dichloroisoquinoline (2.8 g, 39%) as a white solid. ¹H NMR (300 MHz, CHLOROFORM-*d*) δ ppm 7.54 (t, *J*=8.01 Hz, 1 H) 7.99 - 8.10 (m, 2 H) 8.32 (d, *J*=8.67 Hz, 1 H); LC-MS (ESI) *m*/*z* 278 (M+H)⁺.

**Step B:** To 5-bromo-1,3-dichloroisoquinoline (1.5 g, 5.4 mmol) in MeOH (15 mL) was added 25% sodium methoxide/McOH (1.4 mL, 6.4 mmol), and the mixture was heated at 70°C for 30 min. The mixture was allowed to cool, and then water was added. The solid was collected by filtration to afford crude 5-bromo-3-chloro-1-methoxyisoquinoline (2.3 g, quantitative) which was used without further purification. LC-MS (ESI) *m*/*z* 274 (M+H)⁺.

**Step C:** To a mixture of crude 5-bromo-3-chloro-1-methoxyisoquinoline (1 g, 3.65 mmol) and Cs₂CO₃ (2.73 g, 8.4 mmol) in N,N-dimethylacetamide (12 mL) was added 4-fluorobenzenethiol (0.9 mL, 8.4 mmol) and the mixture was heated in a microwave synthesizer at 100°C. The mixture was allowed to cool and then diluted with 4 N HCl. The precipitated solid was collected by filtration and washed with water and hexanes to afford crude 5-bromo-3-((4-fluorophenyl)thio)isoquinolin-1-ol (667 mg, 53%) as a white solid. LC-MS (ESI) *m*/*z* 350/352 (M+H)⁺.

**Step D:** To crude 5-bromo-3-((4-fluorophenyl)thio)isoquinolin-1-ol (300 mg, 0.86 mmol) was added POCl₃ (3 mL) and the mixture was heated at 110 °C for 2 h. The mixture was concentrated under reduced pressure and then partitioned between EtOAc and saturated aq sodium bicarbonate. The organic layer was dried over sodium sulfate and then concentrated under reduced pressure. To the residue were added DCM (10 mL) and 3-chloroperbenzoic acid (70%, 368 mg, 1.49 mmol). The mixture was stirred at rt for 2 h and then concentrated under reduced pressure onto Celite. The mixture was purified by silica gel chromatography eluting with 10-100% EtOAc/hexanes to give a product that was triturated with a small amount of MeOH to afford 5-bromo-1-chloro-3-((4-fluorophenyl)sulfonyl)isoquinoline (143 mg, 41%). ¹H NMR (300 MHz, DMSO-d₆) δ ppm 7.53 (t, *J*=8.76 Hz, 1 H) 7.95 (t, *J*=8.10 Hz, 1 H) 8.15 (dd, *J*=8.85, 5.27 Hz, 1 H) 8.44 (t, *J*=8.01 Hz, 1 H) 8.77 (s, 1 H); LC-MS (ESI) *m*/*z* 424 (M+Na)⁺.

**Step E:** To 5-bromo-1-chloro-3-((4-fluorophenyl)sulfonyl)isoquinoline (62 mg, 0.16 mmol) in 1-methylpyrrolidin-2-one (2 mL) was added 5-methyl-1H-pyrazol-3-amine (172 mg, 1.77 mmol). The mixture was heated at 60 °C for 3 days, then at 90 °C overnight. The mixture was purified by preparative HPLC (Varian C-18 reverse phase column, eluted with gradient of solvent B = 0.05% formic acid/ACN and solvent A = 5% ACN/0.05% formic acid/H₂O) to afford 5-bromo-3-((4-fluorophenyl)sulfonyl)-N-(5-methyl-1H-pyrazol-3-yl)isoquinolin-1-amine (20 mg, 25%). ¹H NMR (300 MHz, DMSO-*d*₆) δ ppm 2.26 (s, 3 H) 6.05 (s, 1 H) 7.51 (t, *J*=8.85 Hz, 2 H) 7.66 (t, *J*=8.10 Hz, 1 H) 8.02 - 8.13 (m, 3 H) 8.19 (d, *J*=7.54 Hz, 1 H) 8.73 (d, *J*=8.29 Hz, 1 H) 10.30 (br s, 1 H) 12.12 (br s, 1 H); LC-MS (ESI) *m*/*z* 461/463 (M+H)⁺.

### Example 27

### Competition binding assay to determine binding constants (K_{d}) of the compounds against JAK kinases

Competition binding assays used herein were developed, validated and performed as described in Fabian et al., Nature Biotechnology 2005, 23, 329-336. Kinases were produced as fusions to T7 phage (*See*, Fabian et al. or WO04/015142) or alternatively, the kinases were expressed in HEK-293 cells and subsequently tagged with DNA for PCR detection (*See*, WO08/0005310). For the binding assays, streptavidin-coated magnetic beads were treated with biotinylated affinity ligands for 30 min at rt to generate affinity resins. The liganded beads were blocked with excess biotin and washed with blocking buffer (SeaBlock (Pierce), 1 % BSA, 0.05 % Tween 20, 1 mM DTT) to remove unbound ligand and to reduce non-specific binding. Binding reactions were assembled by combining kinase, liganded affinity beads, and test compounds in 1 x binding buffer (20 % SeaBlock, 0.17x PBS, 0.05 % Tween 20, 6 mM DTT). Test compounds were prepared as 100 x stocks in DMSO and rapidly diluted into the aqueous environment. DMSO was added to control assays lacking a test compound. Primary screen interactions were performed in polypropylene 384-well plates in a final volume of 34 µL, while Kd determinations were performed in polystyrene 96-well plates in a final volume of 135 µL. The assay plates were incubated at room temperature with shaking for 1 hour, long enough for binding reactions to reach equilibrium, and the affinity beads were washed extensively with wash buffer (1x PBS, 0.05 % Tween 20) to remove unbound protein. The beads were then resuspended in elution buffer (1x PBS, 0.05 % Tween 20, 2 µM non-biotinylated affinity ligand) and incubated at room temperature with shaking for 30 min. The kinase concentration in the eluates was measured by quantitative PCR. Each kinase was tested individually against each compound. Kds were determined using eleven serial threefold dilutions. A selectivity score, which is a quantitative measure of selectivity of a compound against a panel of enzymes, may be calculated for a compound by dividing the number of enzymes for which a compound meets a set criteria, (for example, a binding constant of 100 nM or less), by the total number of enzymes tested. A kinase selectivity score, S10, for example, is calculated for each compound by dividing the number of kinases for which a compound at a certain concentration (for example, 10 µM) displayed inhibition of 90% or greater compared to negative control lacking inhibitors (DMSO only), divided by the number of distinct kinases tested excluding mutant variants, typically 359 or 386 kinases.

In one embodiment, the compounds provided herein were found to have Kds of less than about 20 µM against JAK2. In another embodiment, the compounds provided herein were found to have Kds of less than about 10 µM against JAK2. In another embodiment, the compounds provided herein were found to have Kds of less than about 1 µM against JAK2.

In another embodiment, the compounds provided herein were found to have Kds of less than about 20 µM against JAK3. In another embodiment, the compounds provided herein were found to have Kds of less than about 10 µM against JAK3. In another embodiment, the compounds provided herein were found to have Kds of less than about 1 µM against JAK3.

### Example 28

### CSTF-1 cell-based reporter assay

CSTF-1 cells are derived from the human erythroleukemia cell line that is growth dependent on GM-CSF and has an intact GM-CSFR/JAK2/STAT5 pathway. The cell line contains stably integrated beta-lactamase reporter gene under the control of the regulatory factor 1 (irf 1) response element recognized by the activated transcription factor STAT5. csTF-1 cells (Invitrogen K1219) were washed with assay media (97%OPTIMEM/ 0.5%dialyzed FBS/ 0.1mM NEAA/ 1mM Na pyr/ P/S) and seeded in the same media at 5x10⁵ cell/mL in T150 flask. After 16 hour incubation, cells were seeded at 2x 10⁵ cell/well in 50 µl volume, into Costar, clear bottom, 96-well assay plates. Serial dilutions of compounds were added to the plates with final DMSO concentration at 0.5% and GM-CSF at 2ng/mL and the plates were then incubated at 30°C and 5% CO₂ for 4 hours. The plates were brought to room temperature before adding Substrate Mixture according to manufacturer's protocol (Invitrogen, Catalog # K1085). The assay plates containing the substrate mixture were incubated in the dark at room temperature for 2 hours. Blue and green fluorescence was measured with excitation at 409nm and emission at 460nm (for blue) and excitation at 409nm and emission at 530nm (for green) using Spectra Max Gemini EM. The compounds provided herein were found to have IC₅₀ of less than about 5 µM. In another embodiment, the compounds provided herein were found to have activity IC₅₀ of less than about 500 nM.

The compounds provided herein were found to have the following activity shown in Table 1:

**Table 1**

| Compound | Cell Assay: CS TF-1 reporter assay IC50 (nM) | Binding Assay: JAK2 Kd (nM) | Binding Assay: JAK3 Kd (nM) | Binding Assay: TYK2 Kd (nM) | S-Score: S(10) at 10µM |
|---|---|---|---|---|---|
| Example 1 | B | A | B | A | A |
| Example 2 | A | A | A | A | A |
| Example 10 | A | A | A | A | D |
| Example 11 | A | A | B | ND | A |
| Example 12 | A | A | B | A | C |
| Example 13 | A | A | A | A | D |
| Example 14 | A | A | A | A | B |
| Example 15 | A | A | A | A | D |
| Example 16 | A | A | A | A | B |
| Example 17 | B | A | B | B | A |
| Example 18 | B | A | B | A | B |
| Example 19 | C | B | C | B | A |
| Example 20 | A | A | A | A | C |
| Example 22 | C | C | C | ND | A |
| Example 23 | C | C | C | C | A |
| Example 24 | A | A | A | A | B |
| Example 25 | A | A | A | A | C |
| Example 26 | A | A | A | A | A |

In Table 1,
CSTF-1 reporter assay IC₅₀ (nM): A ≤100, 100<B≤500, C>500;
JAK2 Kd (nM): A≤5, 5<B≤50, C>50; JAK3 Kd (nM): A ≤10, 10<B≤100, C>100; TYK2 Kd (nM) A ≤10, 10<B≤100, C>100;
S score: A ≤0.3, 0.3<B≤0.4, 0.4<C≤0.5, D > 0.5; and ND= no data.

In certain embodiments, the compounds provided herein bind to JAK2 kinase with higher specificity as compared to non-mutant and non-JAK family kinases. For certain compounds provided herein, binding constants for less than 10 non-mutant and non-JAK family kinases are within 100-fold of the binding constant for JAK2 kinase for compounds provided herein. For certain compounds provided herein, binding constants for less than 8 non-mutant and non-JAK family kinases are within 100-fold of the binding constant for JAK2 kinase for compounds provided herein. For certain compounds provided herein, binding constants for 6 non-mutant and non-JAK family kinases arc within 100-fold of the binding constant for JAK2 kinase.

## Claims

1. A compound having formula (I): 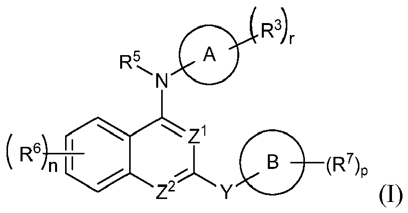 or a pharmaceutically acceptable salt, solvate or hydrate thereof, wherein
Z¹ and Z² are each independently N or CR⁰, such that one of Z¹ or Z² is N and the other is CR⁰;
ring A is azolyl;
ring B is aryl or heteroaryl;
Y is a linker selected from -C(R¹)(R²)-, -S(O)- and -S(O)₂-;
R⁰ is hydrogen, cyano or alkyl;
R¹ and R² are selected from (i), (ii), (iii), (iv) and (v) as follows:
(i) R¹ and R² together form =O, =S, =NR⁹ or =CR¹⁰R¹¹;
(ii) R¹ and R² are both -OR⁸, or R¹ and R², together with the carbon atom to which they are attached, form cycloalkyl or heterocyclyl wherein the cycloalkyl is substituted with one to four substituents selected from halo, deutero, alkyl, cycloalkyl, heterocyclyl, aryl, heteroaryl, cyano, =O, =N-OR²¹, -R^{x}OR²¹, -R^{x}N(R²²)₂, -R^{x}S(O)_{q}R²³, -C(O)R²¹, -C(O)OR²¹ and -C(O)N(R²²)₂, and wherein the heterocyclyl contains one to two heteroatoms selected from O, NR²⁴, S, S(O) and S(O)₂;
(iii) R¹ is hydrogen or halo; and R² is halo;
(iv) R¹ is alkyl, alkenyl, alkynyl, cycloalkyl or aryl, wherein the alkyl, alkenyl, alkynyl, cycloalkyl and aryl are each optionally substituted with one to four substitutents selected from halo, cyano, alkyl, -R^{x}OR^{w}, -R^{x}S(O)_{q}R^{v}, -R^{x}NR^{y}R^{z} and - C(O)OR^{w}; and R² is hydrogen, halo or -OR⁸; and
(v) R¹ is halo, deutero, -OR¹², -NR¹³R¹⁴, or -S(O)_{q}R¹⁵; and R² is hydrogen, deutero, alkyl, alkenyl, alkynyl, cycloalkyl or aryl, wherein the alkyl, alkenyl, alkynyl, cycloalkyl and aryl are each optionally substituted with one to four substitutents selected from halo, cyano, alkyl, -R^{x}OR^{w}, -R^{x}S(O)_{q}R^{v} and -R^{x}NR^{y}R^{z};
R³ is hydrogen, deutero, halo, alkyl, cyano, haloalkyl, deuteroalkyl, cycloalkyl, cycloalkylalkyl, hydroxy or alkoxy;
R⁵ is hydrogen or alkyl;
each R⁶ is independently selected from deutero, halo, nitro, cyano, alkyl, alkenyl, alkynyl, haloalkyl, cycloalkyl, aryl, arylalkyl, heteroaryl, heteroarylalkyl, heterocyclyl, heterocyclylalkyl, -R^{x}OR¹⁸, -R^{x}NR¹⁹R²⁰, -R^{x}C(O)NR^{y}R^{z}, -R^{x}S(O)_{q}R^{v}, -R^{x}NR¹⁹C(O)R¹⁸, -R^{x}C(O)OR¹⁸ and -R^{x} NR¹⁹S(O)_{q}R^{v}; where the alkyl, alkenyl, alkynyl, haloalkyl, cycloalkyl, aryl, heteroaryl and heterocyclyl groups are optionally substituted with one, two or three halo, oxo, hydroxy, alkoxy, alkyl, alkenyl, alkynyl, haloalkyl, or cycloalkyl groups;
each R⁷ is independently halo, alkyl, haloalkyl or -R^{x}OR^{w};
R⁸ is alkyl, alkenyl or alkynyl;
R⁹ is hydrogen, alkyl, haloalkyl, hydroxy, alkoxy or amino;
R¹⁰ is hydrogen or alkyl;
R¹¹ is hydrogen, alkyl, haloalkyl or -C(O)OR⁸;
R¹² is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, -C(O)R^{v}, -C(O)OR^{w} and -C(O)NR^{y}R^{z}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one to four substituents independently selected from halo, oxo, alkyl, hydroxy, alkoxy, amino and alkylthio;
R¹³ and R¹⁴ are selected as follows:
(i) R¹³ is hydrogen or alkyl; and R¹⁴ is selected from hydrogen, alkyl, alkenyl, alkynyl, cycloalkyl, cycloalkylalkyl, heterocyclyl, heterocyclylalkyl, aryl, aralkyl, heteroaryl, heteroaralkyl, alkoxy, -C(O)R^{v}, -C(O)OR^{w}, -C(O)NR^{y}R^{z} and -S(O)_{q}R^{v}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one to four substituents independently selected from halo, oxo, alkyl, hydroxy, alkoxy, amino and alkylthio; or
(ii) R¹³ and R¹⁴, together with the nitrogen atom to which they are attached, form heterocyclyl or heteroaryl wherein the heterocyclyl and heteroaryl are substituted with one to four substituents independently selected from halo, alkyl, hydroxy, alkoxy, amino and alkylthio, and wherein the heterocyclyl is optionally substituted with oxo;
R¹⁵ is alkyl, alkenyl, alkynyl, cycloalkyl, cycloalkylalkyl, heterocyclyl, heterocyclylalkyl, aryl, aralkyl, heteroaryl, heteroaralkyl, -C(O)NR^{y}R^{z} or -NR^{y}R^{z}, wherein the alkyl, alkenyl, alkynyl, cycloalkyl, cycloalkylalkyl, heterocyclyl, heterocyclylalkyl, aryl, aralkyl, heteroaryl and heteroaralkyl are each optionally substituted with one to four substituents independently selected from halo, oxo, alkyl, hydroxy, alkoxy, amino and alkylthio;
R¹⁸ is hydrogen, alkyl, haloalkyl, hydroxyalkyl, alkenyl, alkynyl, cycloalkyl, cycloalkylalkyl, heterocyclyl, heterocyclylalkyl, aryl, aralkyl, heteroaryl or heteroarylalkyl; wherein R¹⁸ is optionally substituted with 1 to 3 groups Q¹, each Q¹ independently selected from alkyl, hydroxy, halo, haloalkyl, alkoxy, aryloxy, alkoxyalkyl, alkoxycarbonyl, alkoxysulfonyl, carboxyl, cycloalkyl, heterocyclyl, aryl, heteroaryl, haloaryl and amino;
R¹⁹ and R²⁰ are selected as follows:
(i) R¹⁹ and R²⁰ are each independently hydrogen or alkyl; or
(ii) R¹⁹ and R²⁰, together with the nitrogen atom to which they are attached, form a heterocyclyl or heteroaryl which are each optionally substituted with 1 to 2 groups each independently selected from halo, oxo, alkyl, haloalkyl, hydroxyl and alkoxy;
R²¹ is hydrogen, alkyl, alkenyl, alkynyl, haloalkyl or cycloalkyl;
each R²² is independently hydrogen, alkyl, alkenyl, alkynyl, haloalkyl or cycloalkyl; or both R²², together with the nitrogen atom to which they are attached, form a heterocyclyl optionally substituted with oxo;
R²³ is alkyl, alkenyl, alkynyl or haloalkyl;
R²⁴ is hydrogen or alkyl;
each R^{x} is independently alkylene or a direct bond;
R^{v} is hydrogen, alkyl, alkenyl or alkynyl;
R^{w} is independently hydrogen, alkyl, alkenyl, alkynyl or haloalkyl;
R^{y} and R^{z} are selected as follows:
(i) R^{y} and R^{z} are each independently hydrogen, alkyl, alkenyl, alkynyl, cycloalkyl, haloalkyl or heterocyclyl;
(ii) R^{y} and R^{z}, together with the nitrogen atom to which they are attached, form a heterocyclyl or heteroaryl which are optionally substituted with 1 to 2 groups each independently selected from halo, alkyl, haloalkyl, hydroxyl and alkoxy;
n is 0-4;
p is 0-5;
each q is independently 0, 1 or 2; and
r is 1-3.

2. The compound of claim 1, wherein p is 1 or 2.

3. The compound of claim 1 having formula (II) 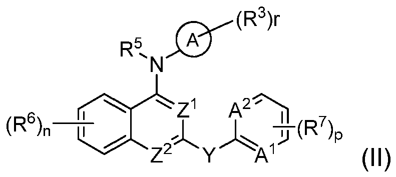 or a pharmaceutically acceptable salt, solvate or hydrate thereof, where A¹ and A² are CH, and p is 1 or 2.

4. The compound of claim 1 having formula (IIIa) or (IIIb) 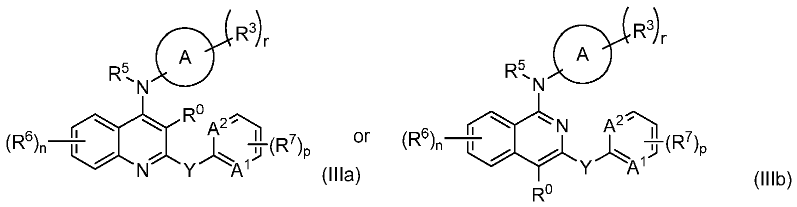 or a pharmaceutically acceptable salt, solvate or hydrate thereof, where
A¹ and A² are each independently selected from N and CH;
A is azolyl;
Y is a linker selected from -C(R¹)(R²)-, -S(O)- and -S(O)₂-;
R⁰ is hydrogen, cyano or alkyl;
R¹ and R² are selected as follows:
(i) R¹ and R² together form =O;
(ii) R¹ is hydrogen or halo; and R² is halo;
(iii) R¹ is alkyl, and R² is hydrogen, alkyl, halo, hydroxy or alkoxy; or
(iv) R¹ is halo, NR¹³R¹⁴, hydroxy or alkoxy; and R² is hydrogen or alkyl;
R³ is hydrogen, halo, alkyl, deuteroalkyl, hydroxy or alkoxy;
R⁵ is hydrogen or alkyl;
R¹³ is hydrogen or alkyl;
R¹⁴ is hydrogen, alkyl or cycloalkyl;
each R⁶ is independently deutero, halo or alkyl;
R⁷ halo;
n is 0 or 1;
p is 1; and
r is 1-3.

5. The compound of claim 1 having formula (Va) or (Vb) 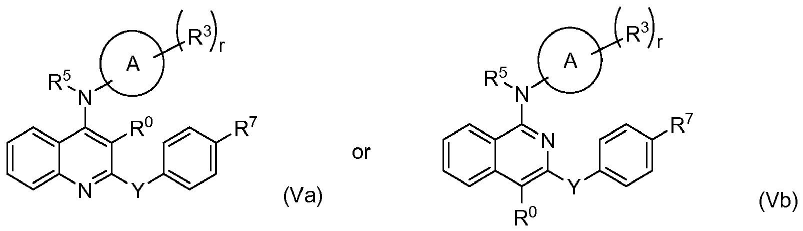 or a pharmaceutically acceptable salt, solvate or hydrate thereof, where
A is pyrazolyl, imidazolyl, oxazolyl or thiazolyl;
Y is a linker selected from -C(R¹)(R²)-, -S(O)- and -S(O)₂-;
R⁰ is hydrogen, cyano or alkyl;
R¹ and R² are selected as follows:
(i) R¹ and R² together form =O;
(ii) R¹ is hydrogen or halo; and R² is halo;
(iii) R¹ is alkyl, and R² is hydrogen, alkyl, halo, hydroxy or alkoxy; or
(iv) R¹ is halo, NR¹³R¹⁴, hydroxy or alkoxy; and R² is hydrogen or alkyl;
R³ is hydrogen, halo, alkyl, deuteroalkyl, hydroxy or alkoxy;
R⁵ is hydrogen or alkyl;
R¹³ is hydrogen or alkyl;
R¹⁴ is hydrogen, alkyl or cycloalkyl;
R⁷ halo; and
r is 1-3.

6. The compound of any of claims 1-4,
wherein A is pyrazolyl, imidazolyl, oxazolyl, thiazolyl, thiadiazolyl, or triazolyl, and p is 1 or 2; and/or
wherein Y is -S(O)₂-, and p is 1 or 2.

7. The compound of any of claims 1-6,
wherein R⁷ is halo, and p is 1 or 2; or
wherein R⁷ is fluoro, and p is 1 or 2.

8. The compound of claim 2 having formula ((VIIa) or (VIIb): 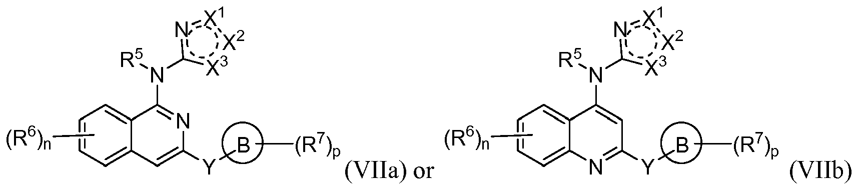 or a pharmaceutically acceptable salt, solvate or hydrate thereof, where
X¹, X² and X³ are selected from (i) and (ii) as follows
(i) X¹ is NR⁴, X² is CR³ and X³ is CH; and
(ii) X¹ is CH, X² is CR³ and X³ is S;
R³ is hydrogen or alkyl;
R⁴ is hydrogen or alkyl;
R⁶ is deutero, halo or alkyl;
R⁷ is deutero, halo or alkyl and
p is 1 or 2.

9. The compound of claim 8, wherein X¹ is NR⁴, X² is CR³ and X³ is CH.

10. The compound of any of claims 1-8, wherein each R³ is independently hydrogen, halo, alkyl, deuteroalkyl, hydroxy or alkoxy.

11. The compound of any of claims 1-7, wherein R⁰ is hydrogen.

12. The compound of claim 1 selected from:
3-((4-fluorophenyl)sulfinyl)-N-(5-methyl-1H-pyrazol-3-yl)isoquinolin-1-amine;
(4-fluorophenyl)(1-((5-methyl-1H-pyrazol-3-yl)amino)isoquinolin-3-yl)methanone;
(4-fluorophenyl)(1-((5-methyl-1H-pyrazol-3-yl)amino)isoquinolin-3-yl)methanol;
N-(3-((4-fluorophenyl)sulfinyl)isoquinolin-1-yl)thiazol-2-amine;
N-(3-((4-fluorophenyl)sulfinyl)isoquinolin-1-yl)-5-methylthiazol-2-amine;
N-(3-((4-fluorophenyl)sulfinyl)isoquinolin-1-yl)-4-methyloxazol-2-amine;
3-((4-fluorophenyl)sulfinyl)-N-(4-methoxypyridin-2-yl)isoquinolin-1-amine;
(4-fluorophenyl)(4-(4-fluorophenyl)-1-((5-methyl-1H-pyrazol-3-yl)amino)isoquinolin-3-yl)methanone;
(4-fluorophenyl)(4-(4-fluorophenyl)-1-((5-methyl-1H-pyrazol-3-yl)amino)isoquinolin-3-yl)methanol;
(4-fluorophenyl)(4-(4-fluorophenyl)-1-((5-methyl-1H-pyrazol-3-yl)amino)isoquinolin-3-yl)methyl formate;
(4-fluorophenyl)(4-(2-methoxyethoxy)-1-((5-methyl-1H-pyrazol-3-yl)amino)isoquinolin-3-yl)methanone;
3-((4-fluorophenyl)sulfinyl)-1-(1H-pyrazol-4-yl)isoquinoline;
3-((4-fluorophenyl)sulfonyl)-N-(5-methyl-1H-pyrazol-3-yl)isoquinolin-1-amine;
3-((4-fluorophenyl)sulfonyl)-N-(5-methyl-1H-pyrazol-3-yl)-4-nitroisoquinolin-1-amine;
3-((4-fluorophenyl)sulfonyl)-N-(1H-pyrazol-3-yl)isoquinolin-1-amine;
3-((4-fluorophenyl)sulfonyl)-N-(5-methoxy-1H-pyrazol-3-yl)isoquinolin-1-amine;
N-(3-((4-fluorophenyl)sulfonyl)isoquinolin-1-yl)-5-methylthiazol-2-amine;
N-(3-((4-fluorophenyl)sulfonyl)isoquinolin-1-yl)thiazol-2-amine;
N-(3-((4-fluorophenyl)sulfonyl)isoquinolin-1-yl)oxazol-2-amine;
3-((3-((4-fluorophenyl)sulfonyl)isoquinolin-1-yl)amino)-1H-pyrazol-5-ol;
N-(3-((4-fluorophenyl)sulfonyl)isoquinolin-1-yl)-1,2,4-thiadiazol-5-amine;
3-((4-fluorophenyl)sulfonyl)-N-(1-methyl-1H-imidazol-4-yl)isoquinolin-1-amine;
3-((4-fluorophenyl)sulfonyl)-N-(1-methyl-*d₃*-1-imidazol-4-yl)isoquinolin-1-amine;
N-(1-ethyl-1H-imidazol-4-yl)-3-((4-fluorophenyl)sulfonyl)isoquinolin-1-amine; and
5-bromo-3-((4-fluorophenyl)sulfonyl)-N-(5-methyl-1H-pyrazol-3-yl)isoquinolin-1-amine;
or a pharmaceutically acceptable salt, solvate or hydrate thereof.

13. A pharmaceutical composition comprising a compound of any of claims 1-12 and a pharmaceutically acceptable carrier, diluent or excipient.

14. The compound of any of claims 1-12 for use in:
a method for treatment of a JAK modulated disease; or
a method for treatment of a JAK2 modulated disease, optionally wherein JAK2 is wild type or mutant JAK2;
optionally wherein the disease is cancer, myeloproliferative disorder, inflammation or autoimmune disease.

15. The compound for use of claim 14, wherein the method further comprises administering a second pharmaceutical agent selected from anti-proliferative agent, anti-inflammatory agent, immunomodulatory agent and immunosuppressive agent.

## Patentansprüche

1. Verbindung mit der Formel (I): 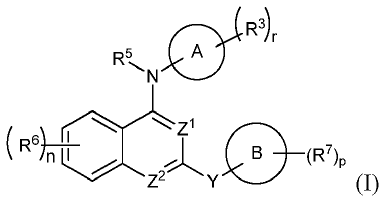 oder ein pharmazeutisch annehmbares Salz, Solvat oder Hydrat davon, wobei
Z¹ und Z² jeweils unabhängig N oder CR⁰ sind, so dass eines von Z¹ oder Z² N ist und das andere CR⁰ ist;
Ring A Azolyl ist;
Ring B Aryl oder Heteroaryl ist;
Y ein aus -C(R¹)(R²)-, -S(O)- und -S(O)₂- ausgewählter Linker ist;
R⁰ Wasserstoff, Cyano oder Alkyl ist;
R¹ und R² wie folgt aus (i), (ii), (iii), (iv) und (v) ausgewählt sind:
(i) R¹ und R² bilden zusammen =O, =S, =NR⁹ oder =CR¹⁰R¹¹;
(ii) R¹ und R² sind beide -OR⁸ oder R¹ und R² bilden mit dem Kohlenstoffatom, an das sie gebunden sind, Cycloalkyl oder Heterocyclyl, wobei das Cycloalkyl mit einem bis vier Substituenten ausgewählt aus Halo, Deutero, Alkyl, Cycloalkyl, Heterocyclyl, Aryl, Heteroaryl, Cyano, =O, =N-OR²¹, -R^{x}OR²¹,-R^{x}N(R²²)₂, -R^{x}S(O)_{q}R²³, -C(O)R²¹, -C(O)OR²¹ und -C(O)N(R²²)₂ substituiert ist, und wobei das Heterocyclyl ein bis zwei aus O, NR²⁴, S, S(O) und S(O)₂ ausgewählte Heteroatome enthält;
(iii) R¹ ist Wasserstoff oder Halo; und R² ist Halo;
(iv) R¹ ist Alkyl, Alkenyl, Alkynyl, Cycloalkyl oder Aryl, wobei das Alkyl, Alkenyl, Alkynyl, Cycloalkyl und Aryl jeweils optional mit einem bis vier Substituenten ausgewählt aus Halo, Cyano, Alkyl, -R^{x}OR^{w}, -R^{x}S(O)_{q}R^{v}, -R^{x}NR^{y}R^{z} und -C(O)OR^{w} substituiert ist; und R² ist Wasserstoff, Halo oder -OR⁸; und
(v) R¹ ist Halo, Deutero, -OR¹², -NR¹³R¹⁴ oder -S(O)_{q}R¹⁵; und R² ist Wasserstoff, Deutero, Alkyl, Alkenyl, Alkynyl, Cycloalkyl oder Aryl, wobei das Alkyl, Alkenyl, Alkynyl, Cycloalkyl und Aryl jeweils optional mit einem bis vier Substituenten ausgewählt aus Halo, Cyano, Alkyl, -R^{x}OR^{w}, -R^{x}S(O)_{q}R^{v} und - R^{x}NR^{y}R^{z} substituiert ist;
R³ Wasserstoff, Deutero, Halo, Alkyl, Cyano, Haloalkyl, Deuteroalkyl, Cycloalkyl, Cycloalkylalkyl, Hydroxy oder Alkoxy ist;
R⁵ Wasserstoff oder Alkyl ist;
jedes R⁶ unabhängig aus Deutero, Halo, Nitro, Cyano, Alkyl, Alkenyl, Alkynyl, Haloalkyl, Cycloalkyl, Aryl, Arylalkyl, Heteroaryl, Heteroarylalkyl, Heterocyclyl, Heterocyclylalkyl, -R^{x}OR¹⁸, -R^{x}NR¹⁹R²⁰, -R^{x}C(O)NR^{y}R^{z}, -R^{x}S(O)_{q}R^{v}, -R^{x}NR¹⁹C(O)R¹⁸, -R^{x}C(O)OR¹⁸ und -R^{x}NR¹⁹S(O)_{q}R^{v} ausgewählt ist; wobei die Alkyl, Alkenyl, Alkynyl, Haloalkyl, Cycloalkyl, Aryl, Heteroaryl und Heterocyclyl Gruppen optional mit einer, zwei oder drei Halo, Oxo, Hydroxy, Alkoxy, Alkyl, Alkenyl, Alkynyl, Haloalkyl oder Cycloalkyl Gruppen substituiert sind;
jedes R⁷ unabhängig Halo, Alkyl, Haloalkyl oder -R^{x}OR^{w} ist;
R⁸ Alkyl, Alkenyl oder Alkynyl ist;
R⁹ Wasserstoff, Alkyl, Haloalkyl, Hydroxy, Alkoxy oder Amino ist;
R¹⁰ Wasserstoff oder Alkyl ist;
R¹¹ Wasserstoff, Alkyl, Haloalkyl oder -C(O)OR⁸ ist;
R¹² aus Wasserstoff, Alkyl, Alkenyl, Alkynyl, Cycloalkyl, Cycloalkylalkyl, Heterocyclyl, Heterocyclylalkyl, Aryl, Aralkyl, Heteroaryl, Heteroaralkyl, -C(O)R^{v},-C(O)OR^{w} und -C(O)NR^{y}R^{z} ausgewählt ist, wobei das Alkyl, Alkenyl, Alkynyl, Cycloalkyl, Cycloalkylalkyl, Heterocyclyl, Heterocyclylalkyl, Aryl, Aralkyl, Heteroaryl und Heteroaralkyl jeweils optional mit einem bis vier Substituenten unabhängig ausgewählt aus Halo, Oxo, Alkyl, Hydroxy, Alkoxy, Amino und Alkylthio substituiert ist;
R¹³ und R¹⁴ wie folgt ausgewählt sind:
(i) R¹³ ist Wasserstoff oder Alkyl; und R¹⁴ ist ausgewählt aus Wasserstoff, Alkyl, Alkenyl, Alkynyl, Cycloalkyl, Cycloalkylalkyl, Heterocyclyl, Heterocyclylalkyl, Aryl, Aralkyl, Heteroaryl, Heteroaralkyl, Alkoxy, -C(O)R^{v}, - C(O)OR^{w}, -C(O)NR^{y}R^{z} und -S(O)_{q}R^{v}, wobei das Alkyl, Alkenyl, Alkynyl, Cycloalkyl, Cycloalkylalkyl, Heterocyclyl, Heterocyclylalkyl, Aryl, Aralkyl, Heteroaryl und Heteroaralkyl jeweils optional mit einem bis vier Substituenten unabhängig ausgewählt aus Halo, Oxo, Alkyl, Hydroxy, Alkoxy, Amino und Alkylthio substituiert ist; oder
(ii) R¹³ und R¹⁴ bilden zusammen mit dem Stickstoffatom, an das sie gebunden sind, Heterocyclyl oder Heteroaryl, wobei das Heterocyclyl und Heteroaryl mit einem bis vier Substituenten unabhängig ausgewählt aus Halo, Alkyl, Hydroxy, Alkoxy, Amino und Alkylthio substituiert sind, und wobei das Heterocyclyl optional mit Oxo substituiert ist;
R¹⁵ Alkyl, Alkenyl, Alkynyl, Cycloalkyl, Cycloalkylalkyl, Heterocyclyl, Heterocyclylalkyl, Aryl, Aralkyl, Heteroaryl, Heteroaralkyl, -C(O)NR^{y}R^{z} oder - NR^{y}R^{z} ist, wobei das Alkyl, Alkenyl, Alkynyl, Cycloalkyl, Cycloalkylalkyl, Heterocyclyl, Heterocyclylalkyl, Aryl, Aralkyl, Heteroaryl und Heteroaralkyl jeweils optional mit einem bis vier Substituenten unabhängig ausgewählt aus Halo, Oxo, Alkyl, Hydroxy, Alkoxy, Amino und Alkylthio substituiert ist;
R¹⁸ Wasserstoff, Alkyl, Haloalkyl, Hydroxyalkyl, Alkenyl, Alkynyl, Cycloalkyl, Cycloalkylalkyl, Heterocyclyl, Heterocyclylalkyl, Aryl, Aralkyl, Heteroaryl oder Heteroarylalkyl ist; wobei R¹⁸ optional mit 1 bis 3 Q¹ Gruppen substituiert ist, wobei jedes Q¹ unabhängig aus Alkyl, Hydroxy, Halo, Haloalkyl, Alkoxy, Aryloxy, Alkoxyalkyl, Alkoxycarbonyl, Alkoxysulfonyl, Carboxyl, Cycloalkyl, Heterocyclyl, Aryl, Heteroaryl, Haloaryl und Amino ausgewählt ist;
R¹⁹ und R²⁰ wie folgt ausgewählt sind:
(i) R¹⁹ und R²⁰ sind jeweils unabhängig Wasserstoff oder Alkyl; oder
(ii) R¹⁹ und R²⁰ bilden zusammen mit dem Stickstoffatom, an das sie gebunden sind, ein Heterocyclyl oder Heteroaryl, welches jeweils optional mit 1 bis 2 Gruppen substituiert ist, die jeweils unabhängig aus Halo, Oxo, Alkyl, Haloalkyl, Hydroxyl und Alkoxy ausgewählt sind;
R²¹ Wasserstoff, Alkyl, Alkenyl, Alkynyl, Haloalkyl oder Cycloalkyl ist;
jedes R²² unabhängig Wasserstoff, Alkyl, Alkenyl, Alkynyl, Haloalkyl oder Cycloalkyl ist; oder beide R²² zusammen mit dem Stickstoffatom, an das sie gebunden sind, ein Heterocyclyl bilden, das optional mit Oxo substituiert ist;
R²³ Alkyl, Alkenyl, Alkynyl oder Haloalkyl ist;
R²⁴ Wasserstoff oder Alkyl ist;
jedes R^{x} unabhängig Alkylen oder eine direkte Bindung ist;
R^{v} Wasserstoff, Alkyl, Alkenyl oder Alkynyl ist;
R^{w} unabhängig Wasserstoff, Alkyl, Alkenyl, Alkynyl oder Haloalkyl ist;
R^{y} und R^{z} wie folgt ausgewählt sind:
(i) R^{y} und R^{z} sind jeweils unabhängig Wasserstoff, Alkyl, Alkenyl, Alkynyl, Cycloalkyl, Haloalkyl oder Heterocyclyl;
(ii) R^{y} und R^{z} bilden zusammen mit dem Stickstoffatom, an das sie gebunden sind, ein Heterocyclyl oder Heteroaryl, die optional mit 1 bis 2 Gruppen substituiert sind, die jeweils unabhängig aus Halo, Alkyl, Haloalkyl, Hydroxyl und Alkoxy ausgewählt sind;
n 0-4 ist;
p 0-5 ist;
jedes q unabhängig 0, 1 oder 2 ist; und
r 1-3 ist.

2. Verbindung nach Anspruch 1, wobei p 1 oder 2 ist.

3. Verbindung nach Anspruch 1 mit der Formel (II) 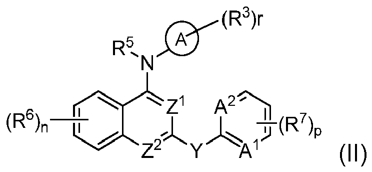 oder ein pharmazeutisch annehmbares Salz, Solvat oder Hydrat davon, wobei A¹ und A² CH sind, und p 1 oder 2 ist.

4. Verbindung nach Anspruch 1 mit der Formel (IIIa) oder (IIIb) 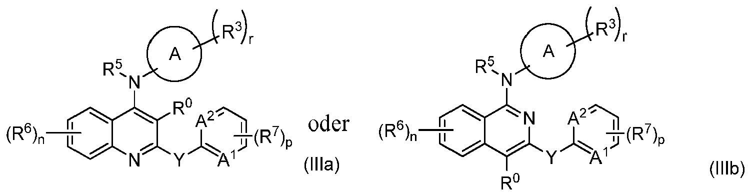 oder ein pharmazeutisch annehmbares Salz, Solvat oder Hydrat davon, wobei
A¹ und A² jeweils unabhängig ausgewählt sind aus N und CH;
A Azolyl ist;
Y ein aus -C(R¹)(R²)-, -S(O)- und -S(O)₂- ausgewählter Linker ist;
R⁰ Wasserstoff, Cyano oder Alkyl ist;
R¹ und R² wie folgt ausgewählt sind:
(i) R¹ und R² bilden zusammen =O;
(ii) R¹ ist Wasserstoff oder Halo; und R² ist Halo;
(iii) R¹ ist Alkyl, und R² ist Wasserstoff, Alkyl, Halo, Hydroxy oder Alkoxy;
oder
(iv) R¹ ist Halo, NR¹³R¹⁴, Hydroxy oder Alkoxy; und R² ist Wasserstoff oder Alkyl;
R³ Wasserstoff, Halo, Alkyl, Deuteroalkyl, Hydroxy oder Alkoxy ist;
R⁵ Wasserstoff oder Alkyl ist;
R¹³ Wasserstoff oder Alkyl ist;
R¹⁴ Wasserstoff, Alkyl oder Cycloalkyl ist;
jedes R⁶ unabhängig Deutero, Halo oder Alkyl ist;
R⁷ Halo ist;
n 0 oder 1 ist;
p 1 ist; und
r 1-3 ist.

5. Verbindung nach Anspruch 1 mit der Formel (Va) oder (Vb) 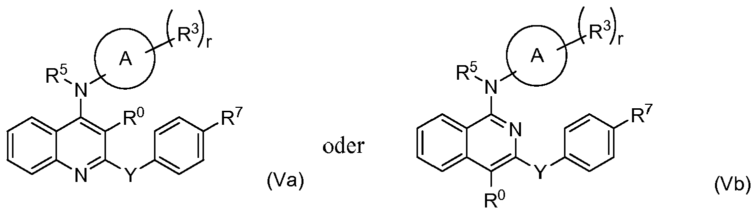 oder ein pharmazeutisch annehmbares Salz, Solvat oder Hydrat davon, wobei
A Pyrazolyl, Imidazolyl, Oxazolyl oder Thiazolyl ist;
Y ein aus -C(R¹)(R²)-, -S(O)- und -S(O)₂- ausgewählter Linker ist;
R⁰ Wasserstoff, Cyano oder Alkyl ist;
R¹ und R² wie folgt ausgewählt sind:
(i) R¹ und R² bilden zusammen =O;
(ii) R¹ ist Wasserstoff oder Halo; und R² ist Halo;
(iii) R¹ ist Alkyl, und R² ist Wasserstoff, Alkyl, Halo, Hydroxy oder Alkoxy;
oder
(iv) R¹ ist Halo, NR¹³R¹⁴, Hydroxy oder Alkoxy; und R² ist Wasserstoff oder Alkyl;
R³ Wasserstoff, Halo, Alkyl, Deuteroalkyl, Hydroxy oder Alkoxy ist;
R⁵ Wasserstoff oder Alkyl ist;
R¹³ Wasserstoff oder Alkyl ist;
R¹⁴ Wasserstoff, Alkyl oder Cycloalkyl ist;
R⁷ Halo ist; und
r 1-3 ist.

6. Verbindung nach einem der Ansprüche 1-4,
wobei A Pyrazolyl, Imidazolyl, Oxazolyl, Thiazolyl, Thiadiazolyl oder Triazolyl ist, und p 1 oder 2 ist; und/oder
wobei Y -S(O)₂- ist, und p 1 oder 2 ist.

7. Verbindung nach einem der Ansprüche 1-6,
wobei R⁷ Halo ist und p 1 oder 2 ist; oder
wobei R⁷ Fluoro ist und p 1 oder 2 ist.

8. Verbindung nach Anspruch 2 mit der Formel ((VIIa) oder (VIIb): 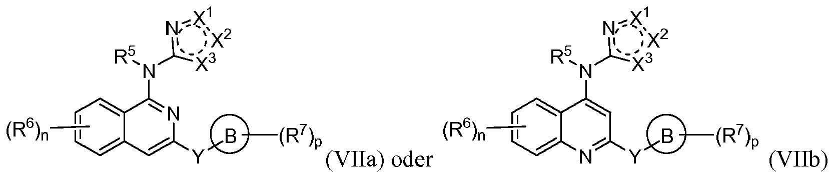 oder ein pharmazeutisch annehmbares Salz, Solvat oder Hydrat davon, wobei
X¹, X² und X³ wie folgt aus (i) und (ii) ausgewählt sind
(i) X¹ ist NR⁴, X² ist CR³ und X³ ist CH; und
(ii) X¹ ist CH, X² ist CR³ und X³ ist S;
R³ Wasserstoff oder Alkyl ist;
R⁴ Wasserstoff oder Alkyl ist;
R⁶ Deutero, Halo oder Alkyl ist;
R⁷ Deutero, Halo oder Alkyl ist; und
p 1 oder 2 ist.

9. Verbindung nach Anspruch 8, wobei X¹ NR⁴ ist, X² CR³ ist und X³ CH ist.

10. Verbindung nach einem der Ansprüche 1-8, wobei jedes R³ unabhängig Wasserstoff, Halo, Alkyl, Deuteroalkyl, Hydroxy oder Alkoxy ist.

11. Verbindung nach einem der Ansprüche 1-7, wobei R⁰ Wasserstoff ist.

12. Verbindung nach Anspruch 1 ausgewählt aus:
3-((4-Fluorophenyl)sulfinyl)-N-(5-methyl-1H-pyrazol-3-yl)isochinolin-1-amin;
(4-Fluorophenyl)(1-((5-methyl-1H-pyrazol-3-yl)amino)isochinolin-3-yl)methanon;
(4-Fluorophenyl)(1-((5-methyl-1H-pyrazol-3-yl)amino)isochinolin-3-yl)methanol;
N-(3-((4-Fluorophenyl)sulfinyl)isochinolin-1-yl)thiazol-2-amin;
N-(3-((4-Fluorophenyl)sulfinyl)isochinolin-1-yl)-5-methylthiazol-2-amin;
N-(3-((4-Fluorophenyl)sulfinyl)isochinolin-1-yl)-4-methyloxazol-2-amin;
3-((4-Fluorophenyl)sulfinyl)-N-(4-methoxypyridin-2-yl)isochinolin-1-amin;
(4-Fluorophenyl)(4-(4-fluorophenyl)-1-((5-methyl-1H-pyrazol-3-yl)amino)isochinolin-3-yl)methanon;
(4-Fluorophenyl)(4-(4-fluorophenyl)-1-((5-methyl-1H-pyrazol-3-yl)amino)isochinolin-3-yl)methanol;
(4-Fluorophenyl)(4-(4-fluorophenyl)-1-((5-methyl-1H-pyrazol-3-yl)amino)isochinolin-3-yl)methylformiat;
(4-Fluorophenyl)(4-(2-methoxyethoxy)-1-((5-methyl-1H-pyrazol-3-yl)amino)isochinolin-3-yl)methanon;
3-((4-Fluorophenyl)sulfinyl)-1-(1H-pyrazol-4-yl)isochinolin;
3-((4-Fluorophenyl)sulfonyl)-N-(5-methyl-1H-pyrazol-3-yl)isochinolin-1-amin;
3-((4-Fluorophenyl)sulfonyl)-N-(5-methyl-1H-pyrazol-3-yl)-4-nitroisochinolin-1-amin;
3 -((4-Fluorophenyl)sulfonyl)-N-(1H-pyrazol-3-yl)isochinolin-1-amin;
3-((4-Fluorophenyl)sulfonyl)-N-(5-methoxy-1H-pyrazol-3-yl)isochinolin-1-amin;
N-(3-((4-Fluorophenyl)sulfonyl)isochinolin-1-yl)-5-methylthiazol-2-amin;
N-(3-((4-Fluorophenyl)sulfonyl)isochinolin-1-yl)thiazol-2-amin;
N-(3-((4-Fluorophenyl)sulfonyl)isochinolin-1-yl)oxazol-2-amin;
3-((3-((4-Fluorophenyl)sulfonyl)isochinolin-1-yl)amino)-1H-pyrazol-5-ol;
N-(3-((4-Fluorophenyl)sulfonyl)isochinolin-1-yl)-1,2,4-thiadiazol-5-amin;
3-((4-Fluorophenyl)sulfonyl)-N-(1-methyl-1H-imidazol-4-yl)isochinolin-1-amin;
3-((4-Fluorophenyl)sulfonyl)-N-(1-methyl-*d₃*-1H-imidazol-4-yl)isochinolin-1-amin;
N-(1-ethyl-1H-imidazol-4-yl)-3-((4-fluorophenyl)sulfonyl)isochinolin-1-amin; und
5-Bromo-3-((4-fluorophenyl)sulfonyl)-N-(5-methyl-1H-pyrazol-3-yl)isochinolin-1-amin;
oder ein pharmazeutisch annehmbares Salz, Solvat oder Hydrat davon.

13. Pharmazeutische Zusammensetzung umfassend eine Verbindung nach einem der Ansprüche 1-12 und ein/einen pharmazeutisch annehmbares/annehmbaren Träger, Verdünnungsmittel oder Exzipienten.

14. Verbindung nach einem der Ansprüche 1-12 für die Verwendung in:
einem Verfahren zur Behandlung einer JAK modulierten Erkrankung; oder
einem Verfahren zur Behandlung einer JAK2 modulierten Erkrankung, wobei optional JAK2 ein Wildtyp- oder mutiertes JAK2 ist;
wobei optional die Erkrankung Krebs, myeloproliferative Erkrankung, Entzündungs- oder Autoimmun-Erkrankung ist.

15. Verbindung für die Verwendung nach Anspruch 14, wobei das Verfahren weiter das Verabreichen eines zweiten pharmazeutischen Mittels ausgewählt aus einem antiproliferativen Mittel, einem anti-Entzündungsmittel, einem immunomodulatorischen Mittel und einem immunosuppressiven Mittel umfasst.

## Revendications

1. Composé présentant la formule (I) 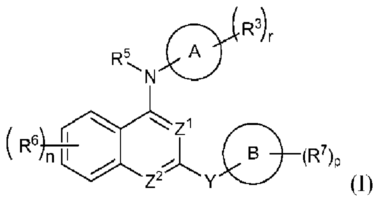 ou un sel, un solvate ou un hydrate pharmaceutiquement acceptable de celui-ci, où :
Z¹ et Z² représentent, chacun indépendamment, N ou CR⁰, de manière telle qu'un parmi Z¹ ou Z² représente N et l'autre représente CR⁰ ;
le cycle A représente azolyle ;
le cycle B représente aryle ou hétéroaryle ;
Y représente un lieur choisi parmi -C(R¹)(R²)-, -S(O)- et -S(O)₂- ;
R⁰ représente hydrogène, cyano ou alkyle ;
R¹ et R² sont choisis parmi (i), (ii), (iii), (iv) et (v) comme suit :
(i) R¹ et R², ensemble, forment =O, =S, =NR⁹ ou =CR¹⁰R¹¹ ;
(ii) R¹ et R² représentent tous deux -OR⁸ ; ou R¹ et R², ensemble avec l'atome de carbone auquel ils sont attachés, forment cycloalkyle ou hétérocyclyle, où cycloalkyle est substitué par un à quatre substituants choisis parmi halogéno, deutéro, alkyle, cycloalkyle, hétérocyclyle, aryle, hétéroaryle, cyano, =O, =N-OR²¹, -R^{x}OR²¹, -R^{x}N(R²²)₂, -R^{x}S(O)_{q}R²³, -C(O)R²¹, -C(O)OR²¹ et -C(O)N(R²²)₂ et où hétérocyclyle contient un ou deux hétéroatomes choisis parmi O, NR²⁴, S, S(O) et S(O)₂ ;
(iii) R¹ représente hydrogène ou halogéno ; et R² représente halogéno ;
(iv) R¹ représente alkyle, alcényle, alcynyle, cycloalkyle ou aryle, où alkyle, alcényle, alcynyle, cycloalkyle et aryle sont, chacun, éventuellement substitués par un à quatre substituants choisis parmi halogéno, cyano, alkyle, -R^{x}OR^{w}, -R^{x}S(O)_{q}R^{v}, -R^{x}NR^{y}R^{z} et -C(O)OR^{w} ; et R² représente hydrogène, halogéno ou -OR⁸ ; et
(v) R¹ représente halogéno, deutéro, -OR¹², -NR¹³R¹⁴ ou -S(O)_{q}R¹⁵ ; et R² représente hydrogène, deutéro, alkyle, alcényle, alcynyle, cycloalkyle ou aryle, où alkyle, alcényle, alcynyle, cycloalkyle et aryle sont, chacun, éventuellement substitués par un à quatre substituants choisis parmi halogéno, cyano, alkyle, -R^{x}OR^{w}, -R^{x}S(O)_{q}R^{v} et -R^{x}NR^{y}R^{z} ;
R³ représente hydrogène, deutéro, halogéno, alkyle, cyano, halogénoalkyle, deutéroalkyle, cycloalkyle, cycloalkylalkyle, hydroxy ou alcoxy ;
R⁵ représente hydrogène ou alkyle ;
chaque R⁶ est choisi, indépendamment, parmi deutéro, halogéno, nitro, cyano, alkyle, alcényle, alcynyle, halogénoalkyle, cycloalkyle, aryle, arylalkyle, hétéroaryle, hétéroarylalkyle, hétérocyclyle, hétérocyclylalkyle, -R^{x}OR¹⁸, -R^{x}NR¹⁹R²⁰, -R^{x}C(O)NR^{y}R^{z}, -R^{x}S(O)_{q}R^{v}, -R^{x}NR¹⁹C(O)R¹⁸, -R^{x}C(O)OR¹⁸ et -R^{x}NR¹⁹S(O)_{q}R^{v}; où les groupes alkyle, alcényle, alcynyle, halogénoalkyle, cycloalkyle, aryle, hétéroaryle et hétérocyclyle sont éventuellement substitués par un, deux ou trois groupes halogéno, oxo, hydroxy, alcoxy, alkyle, alcényle, alcynyle, halogénoalkyle ou cycloalkyle ;
chaque R⁷ représente, indépendamment, halogéno, alkyle, halogénoalkyle ou -R^{x}OR^{w} ;
R⁸ représente alkyle, alcényle ou alcynyle ;
R⁹ représente hydrogène, alkyle, halogénoalkyle, hydroxy, alcoxy ou amino ;
R¹⁰ représente hydrogène ou alkyle ;
R¹¹ représente hydrogène, alkyle, halogénoalkyle ou -C(O)OR⁸ ;
R¹² est choisi parmi hydrogène, alkyle, alcényle, alcynyle, cycloalkyle, cycloalkylalkyle, hétérocyclyle, hétérocyclylalkyle, aryle, aralkyle, hétéroaryle, hétéroaralkyle, -C(O)R^{v}, -C(O)OR^{w} et -C(O)NR^{y}R^{z}, où alkyle, alcényle, alcynyle, cycloalkyle, cycloalkylalkyle, hétérocyclyle, hétérocyclylalkyle, aryle, aralkyle, hétéroaryle et hétéroaralkyle sont, chacun, éventuellement substitués par un à quatre substituants choisis, indépendamment, parmi halogéno, oxo, alkyle, hydroxy, alcoxy, amino et alkylthio ;
R¹³ et R¹⁴ sont choisis comme suit :
(i) R¹³ représente hydrogène ou alkyle ; et R¹⁴ est choisi parmi hydrogène, alkyle, alcényle, alcynyle, cycloalkyle, cycloalkylalkyle, hétérocyclyle, hétérocyclylalkyle, aryle, aralkyle, hétéroaryle, hétéroaralkyle, alcoxy, -C(O)R^{v}, -C(O)OR^{w}, -C(O)NR^{y}R^{z} et -S(O)_{q}R^{v}, où alkyle, alcényle, alcynyle, cycloalkyle, cycloalkylalkyle, hétérocyclyle, hétérocyclylalkyle, aryle, aralkyle, hétéroaryle et hétéroaralkyle sont, chacun, éventuellement substitués par un à quatre substituants choisis, indépendamment, parmi halogéno, oxo, alkyle, hydroxy, alcoxy, amino et alkylthio ; ou
(ii) R¹³ et R¹⁴, ensemble avec l'atome d'azote auquel ils sont attachés, forment hétérocyclyle ou hétéroaryle, où hétérocyclyle et hétéroaryle sont substitués par un à quatre substituants choisis, indépendamment, parmi halogéno, alkyle, hydroxy, alcoxy, amino et alkylthio et où hétérocyclyle est éventuellement substitué par oxo ;
R¹⁵ représente alkyle, alcényle, alcynyle, cycloalkyle, cycloalkylalkyle, hétérocyclyle, hétérocyclylalkyle, aryle, aralkyle, hétéroaryle, hétéroaralkyle, -C(O)NR^{y}R^{z} ou -NR^{y}R^{z}, où alkyle, alcényle, alcynyle, cycloalkyle, cycloalkylalkyle, hétérocyclyle, hétérocyclylalkyle, aryle, aralkyle, hétéroaryle et hétéroaralkyle sont, chacun, éventuellement substitués par un à quatre substituants choisis, indépendamment, parmi halogéno, oxo, alkyle, hydroxy, alcoxy, amino et alkylthio ;
R¹⁸ représente hydrogène, alkyle, halogénoalkyle, hydroxyalkyle, alcényle, alcynyle, cycloalkyle, cycloalkylalkyle, hétérocyclyle, hétérocyclylalkyle, aryle, aralkyle, hétéroaryle ou hétéroarylalkyle ; où R¹⁸ est éventuellement substitué par 1 à 3 groupes Q¹, chaque Q¹ étant choisi, indépendamment, parmi alkyle, hydroxy, halogéno, halogénoalkyle, alcoxy, aryloxy, alcoxyalkyle, alcoxycarbonyle, alcoxysulfonyle, carboxyle, cycloalkyle, hétérocyclyle, aryle, hétéroaryle, halogénoaryle et amino ;
R¹⁹ et R²⁰ sont choisis comme suit :
(i) R¹⁹ et R²⁰ représentent, chacun indépendamment, hydrogène ou alkyle ; ou
(ii) R¹⁹ et R²⁰, ensemble avec l'atome d'azote auquel ils sont attachés, forment hétérocyclyle ou hétéroaryle qui sont, chacun, éventuellement substitués par 1 à 2 groupes chacun choisi indépendamment parmi halogéno, oxo, alkyle, halogénoalkyle, hydroxyle et alcoxy ;
R²¹ représente hydrogène, alkyle, alcényle, alcynyle, halogénoalkyle ou cycloalkyle ;
chaque R²² représente, indépendamment, hydrogène, alkyle, alcényle, alcynyle, halogénoalkyle ou cycloalkyle ; ou les deux R²², ensemble avec l'atome d'azote auquel ils sont attachés, forment hétérocyclyle éventuellement substitué par oxo ;
R²³ représente alkyle, alcényle, alcynyle ou halogénoalkyle ;
R²⁴ représente hydrogène ou alkyle ;
chaque R^{x} représente, indépendamment, alkylène ou une liaison directe ;
R^{v} représente hydrogène, alkyle, alcényle ou alcynyle ;
R^{w} représente, indépendamment, hydrogène, alkyle, alcényle, alcynyle ou halogénoalkyle ;
R^{y} et R^{z} sont choisis comme suit :
(i) R^{y} et R^{z} représentent, chacun indépendamment, hydrogène, alkyle, alcényle, alcynyle, cycloalkyle, halogénoalkyle ou hétérocyclyle ;
(ii) R^{y} et R^{z}, ensemble avec l'atome d'azote auquel ils sont attachés, forment hétérocyclyle ou hétéroaryle qui sont éventuellement substitués par 1 à 2 groupes, chacun choisi, indépendamment, parmi halogéno, alkyle, halogénoalkyle, hydroxyle et alcoxy ;
n vaut 0-4 ;
p vaut 0-5 ;
chaque q vaut, indépendamment, 0, 1 ou 2 ; et
r vaut 1-3.

2. Composé selon la revendication 1, où p vaut 1 ou 2.

3. Composé selon la revendication 1 présentant la formule (II) 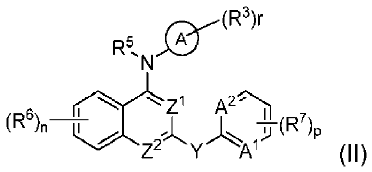 ou un sel, un solvate ou un hydrate pharmaceutiquement acceptable de celui-ci, où A¹ et A² représentent CH et p vaut 1 ou 2.

4. Composé selon la revendication 1 présentant la formule (IIIa) ou (IIIb) 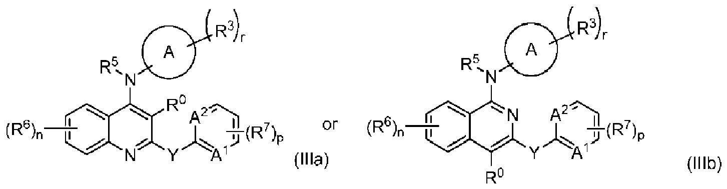 ou un sel, un solvate ou un hydrate pharmaceutiquement acceptable de celui-ci, où :
A¹ et A² sont choisis, chacun indépendamment, parmi N et CH ;
A représente azolyle ;
Y représente un lieur choisi parmi -C(R¹)(R²)-, -S(O)- et -S(O)₂- ;
R⁰ représente hydrogène, cyano ou alkyle ;
R¹ et R² sont choisis comme suit :
(i) R¹ et R² ensemble forment =O ;
(ii) R¹ représente hydrogène ou halogéno ; et R² représente halogéno ;
(iii) R¹ représente alkyle et R² représente hydrogène, alkyle, halogéno, hydroxy ou alcoxy ; ou
(iv) R¹ représente halogéno, NR¹³R¹⁴, hydroxy ou alcoxy ; et R² représente hydrogène ou alkyle ;
R³ représente hydrogène, halogéno, alkyle, deutéroalkyle, hydroxy ou alcoxy ;
R⁵ représente hydrogène ou alkyle ;
R¹³ représente hydrogène ou alkyle ;
R¹⁴ représente hydrogène, alkyle ou cycloalkyle ;
chaque R⁶ représente, indépendamment, deutéro, halogéno ou alkyle ;
R⁷ halogéno ;
n vaut 0 ou 1 ;
p vaut 1 ; et
r vaut 1-3.

5. Composé selon la revendication 1 présentant la formule (Va) ou (Vb) 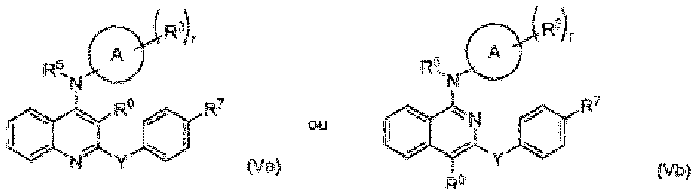 ou un sel, un solvate ou un hydrate pharmaceutiquement acceptable de celui-ci, où :
A représente pyrazolyle, imidazolyle, oxazolyle ou thiazolyle ;
Y représente un lieur choisi parmi -C(R¹)(R²)-, -S(O)- et -S(O)₂- ;
R⁰ représente hydrogène, cyano ou alkyle ;
R¹ et R² sont choisis comme suit :
(i) R¹ et R² ensemble forment =O ;
(ii) R¹ représente hydrogène ou halogéno ; et R² représente halogéno ;
(iii) R¹ représente alkyle et R² représente hydrogène, alkyle, halogéno, hydroxy ou alcoxy ; ou
(iv) R¹ représente halogéno, NR¹³R¹⁴, hydroxy ou alcoxy ; et R² représente hydrogène ou alkyle ;
R³ représente hydrogène, halogéno, alkyle, deutéroalkyle, hydroxy ou alcoxy ;
R⁵ représente hydrogène ou alkyle ;
R¹³ représente hydrogène ou alkyle ;
R¹⁴ représente hydrogène, alkyle ou cycloalkyle ;
R⁷ représente halogéno ; et
r vaut 1-3.

6. Composé selon l'une quelconque des revendications 1-4, où A représente pyrazolyle, imidazolyle, oxazolyle, thiazolyle, thiadiazolyle ou triazolyle et p vaut 1 ou 2 ; et/ou où Y représente -S(O)₂- et p vaut 1 ou 2.

7. Composé selon l'une quelconque des revendications 1-6, où R⁷ représente halogéno et p vaut 1 ou 2 ; ou où R⁷ représente fluoro et p vaut 1 ou 2.

8. Composé selon la revendication 2 présentant la formule (VIIa) ou (VIIb) : 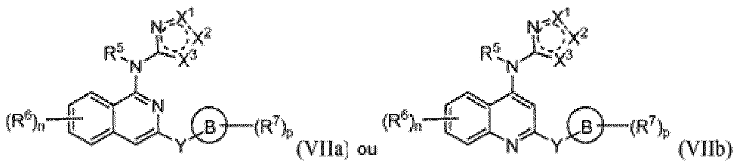 ou un sel, un solvate ou un hydrate pharmaceutiquement acceptable de celui-ci, où :
X¹, X² et X³ sont choisis parmi (i) et (ii) comme suit :
(i) X¹ représente NR⁴, X² représente CR³ et X³ représente CH ; et
(ii) X¹ représente CH, X² représente CR³ et X³ représente S ;
R³ représente hydrogène ou alkyle ;
R⁴ représente hydrogène ou alkyle ;
R⁶ représente deutéro, halogéno ou alkyle ;
R⁷ représente deutéro, halogéno ou alkyle et
p vaut 1 ou 2.

9. Composé selon la revendication 8, où X¹ représente NR⁴, X² représente CR³ et X³ représente CH.

10. Composé selon l'une quelconque des revendications 1-8, où chaque R³ représente, indépendamment, hydrogène, halogéno, alkyle, deutéroalkyle, hydroxy ou alcoxy.

11. Composé selon l'une quelconque des revendications 1 à 7, où R⁰ représente hydrogène.

12. Composé selon la revendication 1 choisi parmi :
3-((4-fluorophényl)sulfinyl)-N-(5-méthyl-1H-pyrazol-3-yl)isoquinoléin-1-amine ;
(4-fluorophényl)(1-((5-méthyl-1H-pyrazol-3-yl)amino)isoquinoléin-3-yl)méthanone ;
(4-fluorophényl)(1-((5-méthyl-1H-pyrazol-3-yl)amino)isoquinoléin-3-yl)méthanol ;
N-(3-((4-fluorophényl)sulfinyl)isoquinoléin-1-yl)thiazol-2-amine ;
N-(3-((4-fluorophényl)sulfinyl)isoquinoléin-1-yl)-5-méthylthiazol-2-amine ;
N-(3-((4-fluorophényl)sulfinyl)isoquinoléin-1-yl)-4-méthyloxazol-2-amine ;
3-((4-fluorophényl)sulfinyl)-N-(4-méthoxypyridin-2-yl)isoquinoléin-1-amine ;
(4-fluorophényl)(4-(4-fluorophényl)-1-((5-méthyl-1H-pyrazol-3-yl)amino)isoquinoléin-3-yl)méthanone ;
(4-fluorophényl)(4-(4-fluorophényl)-1-((5-méthyl-1H-pyrazol-3-yl)amino)isoquinoléin-3-yl)méthanol ;
formiate de (4-fluorophényl)(4-(4-fluorophényl)-1-((5-méthyl-1H-pyrazol-3-yl)amino)isoquinoléin-3-yl)méthyle ;
(4-fluorophényl)(4-(2-méthoxyéthoxy)-1-((5-méthyl-1H-pyrazol-3-yl)amino)isoquinoléin-3-yl)méthanone ;
3-((4-fluorophényl)sulfinyl)-1-(1H-pyrazol-4-yl)isoquinoléine ;
3-((4-fluorophényl)sulfonyl)-N-(5-méthyl-1H-pyrazol-3-yl)isoquinoléin-1-amine ;
3-((4-fluorophényl)sulfonyl)-N-(5-méthyl-1H-pyrazol-3-yl)-4-nitroisoquinoléin-1-amine ;
3-((4-fluorophényl)sulfonyl)-N-(1H-pyrazol-3-yl)isoquinoléin-1-amine ;
3-((4-fluorophényl)sulfonyl)-N-(5-méthoxy-1H-pyrazol-3-yl)isoquinoléin-1-amine ;
N-(3-((4-fluorophényl)sulfonyl)isoquinoléin-1-yl)-5-méthylthiazol-2-amine ;
N-(3-((4-fluorophényl)sulfonyl)isoquinoléin-1-yl)thiazol-2-amine ;
N-(3-((4-fluorophényl)sulfonyl)isoquinoléin-1-yl)oxazol-2-amine ;
3-((3-((4-fluorophényl)sulfonyl)isoquinoléin-1-yl)amino)-1H-pyrazol-5-ol ;
N-(3-((4-fluorophényl)sulfonyl)isoquinoléin-1-yl)-1,2,4-thiadiazol-5-amine ;
3-((4-fluorophényl)sulfonyl)-N-(1-méthyl-1H-imidazol-4-yl)isoquinoléin-1-amine ;
3-((4-fluorophényl)sulfonyl)-N-(1-méthyl-d₃-1H-imidazol-4-yl)isoquinoléin-1-amine ;
N-(1-éthyl-1H-imidazol-4-yl)-3-((4-fluorophényl)sulfonyl)isoquinoléin-1-amine ; et
5-bromo-3-((4-fluorophényl)sulfonyl)-N-(5-méthyl-1H-pyrazol-3-yl)isoquinoléin-1-amine ;
ou un sel, un solvate ou un hydrate pharmaceutiquement acceptable de celui-ci.

13. Composition pharmaceutique comprenant un composé selon l'une quelconque des revendications 1 à 12 et un support, un diluant ou un excipient pharmaceutiquement acceptable.

14. Composé selon l'une quelconque des revendications 1 à 12, pour une utilisation dans :
un procédé pour le traitement d'une maladie modulée par JAK ; ou
un procédé pour le traitement d'une maladie modulée par JAK2, éventuellement où JAK2 est JAK2 de type sauvage ou JAK2 mutant ;
éventuellement où la maladie est un cancer, un trouble de myéloprolifération, une inflammation ou une maladie auto-immune.

15. Composé pour une utilisation selon la revendication 14, où le procédé comprend en outre l'administration d'un deuxième agent pharmaceutique choisi parmi un agent d'antiprolifération, un agent anti-inflammatoire, un agent d'immunomodulation et un agent immunosuppresseur.
